# EUROPEAN PATENT APPLICATION

(11) **EP 4 342 983 A1**
(43) Date of publication of application: **27.03.2024**
(21) Application number: 22803964.0
(22) Date of filing: 17.05.2022
(51) Int. Cl.: C12N 5/10, C12N 15/64, C12N 15/85, C12N 15/90, A61P 35/00

(54) **METHOD FOR MODIFYING CELL**

(30) Priority: 18.05.2021 CN 202110542584; 18.05.2021 CN 202110542564; 18.05.2021 CN 202110541397
(71) Applicant: Cells & Genes Biotech (Shanghai) Co., Ltd, Shanghai 201203 (CN)
(72) Inventor: LI, Linhong, Shanghai 201203 (CN); ZHU, Xiangyang, Shanghai 201203 (CN); LIU, Wuqing, Shanghai 201203 (CN); YU, Shijun, Shanghai 201203 (CN); XIN, Pengcheng, Shanghai 201203 (CN); WANG, Li, Shanghai 201203 (CN); ZHANG, Shuangshuang, Shanghai 201203 (CN)
(74) Representative: Santoro, Sofia
(86) International application number: PCT/CN2022/093334
(87) International publication number: WO 2022/242644

(57) **Abstract**

A method for modifying a cell. The method comprises: transfecting, using a transfection composition, a cell to be modified so that the cell contains and/or expresses an exogenous gene, the transfection composition containing an exogenous nucleie acid molecule comprising the exogenous gene, at least a part of the exogenous nucleic acid molecule being obtained from a host cell, and in the part of the exogenous nucleic acid molecule obtained from the host cell, the content of the genomic DNA of the host cell being about 10% (w/w) or less.

## Description

### Technical Field

The present application relates to the field of biomedicine, in particular to a method for modifying cell.

### Technical Background

Improving cell function through genetic modification is an important means to increase the efficacy of cell therapy.At present, the field of cell therapy mainly uses viral vectors to transfect cells to achieve the stable expression of various molecules , so as to achieve the purpose of cell therapy .However, the design of viral vector transfection is time consuming, the preparation process is complicated, the test period is long, and the cost is high. Meanwhile, the introduction of viral sequences into human hosts possibly triggers host immunogenicity, insertional mutagenesis, and causes unpredictable safety hazards.

It has been reported that there have been attempts to use non-viral systems for cell transformation or cell transfection, to achieve the purpose of cell therapy .However, the transfection efficiency mediated by the non-viral system is low, and it is highly toxic to the transfected cells , which seriously affects the viability of the transfected cells. In particular, so far, non-viral transfection of immune cells, stem cells, fibroblasts, etc. (especially primary cells) has produced even more cytotoxicity, which results in high cell death rates and low expansion rates. Thus, the efficiency of transfection and the recovery rate of living cells cannot reach the level required by cell therapy. Under the limitations of the existing non-viral methods, in order to achieve the amount of cells required for cell therapy, it is necessary to carry out long-term directed expansion of cells *in vitro* .However, long-term directed expansion *in vitro* will inevitably change the phenotype and function of the cells and will significantly limit the cell therapy effect of the transfected cells.

Therefore, there is an urgent need of developing a non-viral cell modification method that is safe, has high transfection efficiency and good cell viability, and can produce enough cells for clinical treatment in a short period of time, so as to overcome the aforementioned problems.

### Summary of invention

The present application provides a non-viral cell transfection method. The method innovatively and greatly improves the problems of the current non-viral plasmid electroporation technology, which comprise low transfection efficiency of transfecting cells, low expression of exogenous nucleic acid molecules, poor cell viability and poor amplification rate after transfection, inability to produce enough cells for clinical treatment in a short period of time, and thus, the difficulty of being directly used for cell therapy.The inventors of the present application have surprisingly discovered that the residual host genomic DNA in the plasmid extracted from the host is one of the main reasons for the current difficulties in electroporating cells with non-viral plasmids When the inventors tried to reduce the content of host genomic DNA in these plasmids , they were surprised to find that when transfected with the optimized plasmids, the cell expansion ability was significantly enhanced, and the number of cells required for clinical treatment may be produced within a short period of several days, and the expression rate of exogenous nucleic acid molecules is also greatly improved . Moreover, the transfected cells can significantly and specifically enhance the inhibitory effect on the growth of target tumor cells *in vivo.*

The stability and reproducibility of transfecting different donors or different batches of cells are important factors to be considered in the production of cell therapy products.The inventors of the present application have found that, when the genomic DNA of the host (such as microorganisms) in the plasmid used for transfection is reduced to a certain threshold, the negative impact on cell viability and expression rate during transfection will be reduced to a certain extent that makes the transfection of cells from different batches and different donor sources (for example, T cells) not only become highly efficient, but also have good reproducibility and stability, and the transfected cells also repeatitively inhibit the growth of target tumor cells in vivo with efficiency and specificity, thus making it possible to manufacture the number of cells required for clinical treatment of tumors in a short period of time for effective cell therapy.The present invention endows the use of non-viral DNA plasmid to transfect cells with great application potential in the field of cell therapy.The inventors have also discovered that, reducing the host genomic DNA in the transfection composition to a certain threshold, can also significantly reduce its cytotoxicity. The method described in this application, has one or more of the following effects: 1) Improve the efficiency of transfection; 2) Improve the editing efficiency of gene editing; 3) Improve the efficiency of DNA homologous recombination; 4) Improve cell viability of transfected cells (e.g. improving cell viability, cell expansion ability, cell killing ability, cell secretion ability, cell preservation (especially cryopreservation) and/or cell recovery ability), and *in vivo* specific target cell killing ability necessary for cell therapy; 5) Quickly prepare the number of cells required for clinical treatment; 6) Can be used (during the preparation process) to control and/or determine the quality of the transfection composition (such as containing DNA plasmids); 7) Easy to operate; 8) Low cost; 9) Good repeatability; 10) High safety level.

In one aspect, the present application provides a method of modifying a cell. The method may comprise: transfecting the cells to be modified with a transfection composition, so that the cells contain and/or express an exogenous gene, and the transfection composition contains an exogenous nucleic acid molecule comprising the exogenous gene, at least part of the exogenous nucleic acid molecule is obtained from a host cell; and in the portion of the exogenous nucleic acid molecule obtained from the host cell, the content of genomic DNA of the host cell is less than about 10% (w/w).

In some embodiments, the cell to be modified is eukaryotic cell. In some embodiments, the cell to be modified is mammalian cell. In some embodiments, the cell to be modified is human cell. In some embodiments, the cell to be modified comprises stem cell, immune cell, fibroblast, fibrocyte, and/or muscle cell. In some embodiments, the cell to be modified comprises pluripotent stem cell (or multipotent stem cell), hematopoietic stem cell and/or mesenchymal stem cell. In some embodiments, the cell to be modified comprises immune effector cell. In some embodiments, the cell to be modified comprises T lymphocytes, B lymphocyte, NK cell, macrophage, dendritic cell, monocyte, granulocyte and/or mast cell. In some embodiments, the cell to be modified comprises peripheral blood lymphocyte. In some embodiments, the cell to be modified is primary cell. In some embodiments, the cell to be modified is autologous cell derived from a subject.

In some embodiments, the transfected cell to be modified is activated cell. In some embodiments, the activation comprises contacting the cell to be modified with an activation composition. In some embodiments, the cell to be modified comprises immune cell, and the activation composition comprises anti-CD3 and/or anti-CD28 antibodies. In some embodiments, the activation comprises contacting the cell to be modified with the activation composition for no more than about 3 days. In some embodiments, the method comprises performing the transfection in the period of contacting the cell to be transfected with the activating composition for about 2 days or less.

In some embodiments, the transfection comprises electroporating the cells to be modified.

In some embodiments, the exogenous nucleic acid molecule obtained from the host cell is a plasmid.

In some embodiments, the exogenous nucleic acid molecule comprises circular nucleic acid molecule, supercoiled nucleic acid molecule and/or linear nucleic acid molecule.

In some embodiments, the exogenous nucleic acid molecule comprise DNA molecule and/or RNA molecule.

In some embodiments, the exogenous nucleic acid molecule comprises single-stranded nucleic acid molecule and/or double-stranded nucleic acid molecule.

In some embodiments, the concentration of the exogenous nucleic acid molecule in the transfection composition is from about 5 µg/mL to about 3000 µg/mL.In some embodiments, the concentration of the exogenous nucleic acid molecule in the transfection composition is from about 200 µg/mL to about 800 µg/mL.

In some embodiments, the exogenous nucleic acid molecule is extracted from the host cell.

In some embodiments, the size of the exogenous nucleic acid molecule is at least about 3 kb.In some embodiments, the size of the exogenous nucleic acid molecule is at least about 4 kb.

In some embodiments, the host is a microbial host.In some embodiments, the host is selected from one or more of the group consisting of, bacteria, fungi, actinomycetes, mycoplasma, chlamydia, rickettsia and spirochetes. In some embodiments, the host comprises Gram-negative bacteria. In some embodiments, the host comprises E. coli.

In some embodiments, the size of the genomic DNA of the host cell is at least about 10 kb.

In some embodiments, the content of the genomic DNA of the host cell accounts for less than about 10% (w/w) of the exogenous nucleic acid molecule obtained from the host cell, such as less than about 1% (w/w). In some embodiments, the content of the genomic DNA of the host cell accounts for less than about 9‰ (w/w) of the exogenous nucleic acid molecule obtained from the host cell, for example, less than about 5‰ (w/w ), less than about 2‰ (w/w), less than and 1‰ (w/w).

In some embodiments of the methods of the present application, the content of the genomic DNA of the host cell is determined by qPCR method.

In some embodiments, the genomic DNA of the host cell is not comprised in the portion of exogenous nucleic acid molecule.

In some embodiments, the portion of the exogenous nucleic acid molecule obtained from the host cell is treated so that the amount of genomic DNA of the host cell therein is reduced.

In some embodiments, the method further comprises measuring the content of the genomic DNA of the host cell in the portion of the exogenous nucleic acid molecule obtained from the host cell, so as to determine whether the portion of the exogenous nucleic acid molecular portion should be treated according to the content so that the amount of genomic DNA of the host cell therein is reduced.

For example, when the genomic DNA content of the host cell accounts for more than about 10% (w/w) (for example, about 9% (w/w), about 8% (w/w), about 7% (w/w), about 6% (w/w), about 5% (w/w), about 4% (w/w), about 3% (w/w) , about 2.5% (w/w), about 2% (w/w), about 1.5% (w/w), about 1% (w/w), about 9 ‰ (w/w), about 8 ‰ (w/w), about 7 ‰ (w/w), about 6 ‰ (w/w), about 5 ‰ (w/w), about 4.5 ‰ (w/w), about 4 ‰ (w/w), about 3.5 ‰ (w/w), about 3 ‰ (w/w), about 2.5 ‰ (w/w), about 2 ‰ (w/w), about 1.5 ‰ (w/w), about 1 ‰ (w /w), about 0.5 ‰ (w/w), about 0. 1 ‰ (w/w), about 0.01 ‰ (w/w), about 0.001 ‰ (w/w)) of the exogenous nucleic acid molecule obtained from the host cell, the treatment is carried out.

In some embodiments, the method further comprises measuring the content of the genomic DNA of the host cell in a portion of the exogenous nucleic acid molecules obtained from the host cell, wherein when the content of the genomic DNA of the host cell is more than about 10% (w/w) (e.g., about 9%(w/w), about 8%(w/w), about 7%(w/w), about 6%(w/w), about 5%(w/w), about 4%(w/w), about 3%(w/w), about 2.5%(w/w), about 2%(w/w), about 1.5%(w/w), about 1%(w/w), about 9‰(w/w), about 8‰(w/w), about 7‰(w/w), about 6‰(w/w), about 5‰(w/w), about 4.5‰(w/w), about 4‰(w/w), about 3.5‰(w/w), about 3‰(w/w), about 2.5‰(w/w), about 2‰(w/w), about 1.5‰(w/w), about 1‰(w/w), about 0.5‰(w/w), about 0.1‰(w/w), about 0.01‰(w/w), about 0.001‰(w/w)), the portion of exogenous nucleic acid molecule is treated to reduce the content of the genomic DNA of the host cell therein.

In some embodiments, the treatment comprises contacting the portion of exogenous nucleic acid molecule portion with one or more reagents selected from the group consisting of: deoxyribonuclease (DNase), SDS, TX-100, CTAB, and cesium chloride-ethidium bromide. In some embodiments, the DNase is capable of non-specifically cleaving linear DNA. In some embodiments, the DNase comprises an exonuclease. In some embodiments, the treatment comprises contacting the portion of the exogenous nucleic acid molecule with the reagent in the presence of Mg²⁺ and Ca²⁺.

In some embodiments, the exogenous gene encodes one or more exogenous proteins.

In some embodiments, the exogenous protein comprises one or more of the following: an antibody or antigen-binding fragment, a chimeric antigen receptor (CAR), a cytokine, and a chemokine.

In some embodiments, the CAR comprises a target binding domain targeting tumor-associated antigens. In some embodiments, the tumor-associated antigen is selected from: GPC3, CD19, BCMA, Claudin18.2, and Mesothelin. In some embodiments, the antibody or antigen-binding fragment comprises a multispecific antibody or antigen-binding fragment thereof. In some embodiments, the multispecific antibody or antigen-binding fragment thereof comprises a bispecific T-cell engager (BiTE). In some embodiments, the BiTE comprises a CD3 binding domain. In some embodiments, the BiTE further comprises a tumor-associated antigen binding domain.

In some embodiments, the cytokine comprises an interleukin. In some embodiments, the interleukin comprises IL15, IL7, or functional active fragments thereof.

In some embodiments, the chemokine comprises CCL19 or functional active fragment thereof.

In some embodiments, the exogenous protein comprises a polyprotein. In some embodiments, there is a cleavable part between two or more proteins in the polyprotein. In some embodiments, the cleavable part comprises a 2A peptide. In some embodiments, the 2A peptide comprises P2A, T2A, F2A, or E2A.

In some embodiments, the exogenous nucleic acid molecule further comprises one or more homologous regions. In some embodiments, each of the homologous regions comprises at least 10 nucleotides. In some embodiments, in the exogenous nucleic acid molecule, the homologous regions are located at the 3' end and/or 5' end of the exogenous gene.

In some embodiments, the exogenous gene is integrated into the genome of the cell.

In some embodiments, after the exogenous gene is integrated into the genome of the cell, its expression is regulated by an endogenous promoter or an exogenous promoter.

In some embodiments, the homology regions are homologous to the target region of the cell's genomic DNA. In some embodiments, the target region is located in the TCR-α constant (TRAC) gene or at the AAVS-I site.

In some embodiments, the transfection composition does not comprise a viral vector.

In some embodiments, the transfection composition further comprises a gene editing system capable of integrating the exogenous gene into a specific location in the cell genome. In some embodiments, the gene editing system comprises a site-specific enzyme or a nucleic acid molecule encoding the enzyme. In some embodiments, the site-specific enzyme is selected from: transcription activator-like effector nucleases (TALEN), zinc finger nucleases (ZFN), transposases, integrases, and Cas proteins. In some embodiments, the Cas protein is Cas9 protein. In some embodiments, the transposase comprises PiggyBac (PB) transposase, and/or Sleeping Beauty (SB) transposase.

In some embodiments, the gene editing further comprises knockout of one or more genes in the cell. The knocked-out gene may comprise PD-1 and/or CD95.

In some embodiments, the gene editing system further comprises one or more guide RNAs.

For example, the gene editing system may comprise one or more guide RNAs targeting the gene(s) to be knocked out. For example, the gene editing system may comprise a guide RNA targeting PD-1. For example, the gene editing system may comprise a guide RNA targeting CD95.

In some embodiments, the guide RNA is complementary to the nucleic acid sequence in the target region of the cell genome. In some embodiments, the gene editing system comprises a ribonucleoprotein complex (RNP), and the RNP comprises the Cas protein and the guide RNA.

In another aspect, the present application provides a method for improving the cell transfection efficiency of exogenous nucleic acid molecules derived from host cells, wherein the method comprises: reducing the content of host cell genomic DNA in the part of the exogenous nucleic acid molecules derived from the host cells.

In some embodiments, the cell to be transfected is eukaryotic cell. In some embodiments, the cell to be transfected is mammalian cell. In some embodiments, the cell to be transfected is a human cell. In some embodiments, the cell to be transfected comprises stem cells, immune cells, fibroblasts, fibrocytes, and/or muscle cells. In some embodiments, the cell to be transfected comprises multipotent stem cells, hematopoietic stem cells, and/or mesenchymal stem cells. In some embodiments, the cell to be transfected comprises immune effector cells. In some embodiments, the cell to be transfected comprises T lymphocytes, B lymphocytes, NK cells, macrophages, dendritic cells, monocytes, granulocytes, and/or mast cells. In some embodiments, the cell to be transfected comprises peripheral blood lymphocytes. In some embodiments, the cell to be transfected is a primary cell. In some embodiments, the cell to be transfected is an autologous cell derived from the subject.

In some embodiments, the transfected cell to be modified is an activated cell. In some embodiments, the activation comprises contacting the cell to be modified with an activation composition. In some embodiments, the cell comprises immune cell, and the activation composition comprises anti-CD3 and/or anti-CD28 antibodies. In some embodiments, the activation comprises contacting the cell to be modified with the activation composition for no more than about 4 days.

In some embodiments, the method comprises transfecting the cell to be transfected when contacting with the activation composition for a period of less than about 2 days.

In some embodiments, the transfection comprises electroporating the cell to be modified.

In some embodiments, the exogenous nucleic acid molecule is a plasmid. In some embodiments, the exogenous nucleic acid molecule comprises circular nucleic acid molecules, supercoiled nucleic acid molecules, and/or linear nucleic acid molecules. In some embodiments, the exogenous nucleic acid molecule comprises DNA molecule and/or RNA molecule. In some embodiments, the exogenous nucleic acid molecule comprises single-stranded nucleic acid molecule and/or double-stranded nucleic acid molecule. In some embodiments, the concentration of the exogenous nucleic acid molecule in the transfection composition is about 5µg/mL to about 3000µg/mL. In some embodiments, the concentration of the exogenous nucleic acid molecule in the transfection composition is about 200µg/mL to about 800µg/mL. In some embodiments, the exogenous nucleic acid molecule is obtained from the host cell. In some embodiments, the size of the exogenous nucleic acid molecule is at least about 3kb.

In some embodiments, the host is a microbial host. In some embodiments, the host is selected from one or more of the following group: bacteria, fungi, actinomycetes, mycoplasmas, chlamydias, rickettsias, and spirochetes. In some embodiments, the host comprises gram-negative bacteria. In some embodiments, the host comprises Escherichia coli.

In some embodiments, the size of the genomic DNA of the host cell is at least about 10kb.

In some embodiments, after the reduction, the content of the genomic DNA of the host cell is less than about 10% (w/w). In some embodiments, after the reduction, the content of the genomic DNA of the host cell is less than about 1% (w/w). In some embodiments, after the reduction, the content of the genomic DNA of the host cell is less than about 9‰ (w/w).

In some embodiments, the content of the genomic DNA of the host cell is determined by qPCR.

In some embodiments, reducing the content of the genomic DNA of the host cell comprises contacting the exogenous nucleic acid molecule obtained from the host cell with one or more reagents selected from the following group: deoxyribonuclease (DNase), SDS, TX-100, CTAB, and cesium chloride-ethidium bromide. In some embodiments, the DNase can non-specifically cleave linear DNA. In some embodiments, the DNase comprises an exonuclease.

In some embodiments, reducing the content of the genomic DNA of the host cell comprises contacting the exogenous nucleic acid molecule obtained from the host cell with the reagent in the presence of Mg²⁺ and Ca²⁺.

In some embodiments, the exogenous nucleic acid molecule comprises an exogenous gene described in any aspect of the present application.

In some embodiments, the exogenous nucleic acid molecule further comprises one or more homologous regions described in any aspect of the present application.

In some embodiments, the exogenous nucleic acid molecule does not comprise a viral vector.

In some embodiments, the transfection composition also comprises a gene editing system capable of integrating the exogenous gene into a specific location in the cell genome. In some embodiments, the gene editing system is as described in any aspect of the present application.

In another aspect, the present application provides a method for improving the cell transfection efficiency of exogenous nucleic acid molecules obtained from host cells, wherein the method comprises: treating the exogenous nucleic acid molecules with DNase. In some embodiments, the DNase can non-specifically cleave linear DNA. In some embodiments, the DNase comprises a nuclease. In some embodiments, the treatment comprises contacting the exogenous nucleic acid molecules with the DNase in the presence of Mg²⁺ and Ca²⁺.

In some embodiments, the transfection comprises electroporating the cell.

In some embodiments, the exogenous nucleic acid molecule is as described in any aspect of the present application.

In some embodiments, the cell is as described in any aspect of the present application.

In another aspect, the present application provides a method for optimizing a transfection composition comprising exogenous nucleic acid molecules obtained from host cells, wherein the method comprises: reducing the content of host cell genomic DNA in the exogenous nucleic acid molecules obtained from the host cells. In some embodiments, the transfection composition is as described in any aspect of the present application. In some embodiments, the host is as described in any aspect of the present application.

In some embodiments, after the optimization, the content of the host cell genomic DNA is less than about 10% (w/w). In some embodiments, after the optimization, the content of the host cell genomic DNA is less than about 1% (w/w). In some embodiments, after the optimization, the content of the host cell genomic DNA is less than about 0.9‰ (w/w). In some embodiments, the content of the host cell genomic DNA is determined by qPCR.

In some embodiments, the optimization comprises contacting the exogenous nucleic acid molecules obtained from the host cells with one or more reagents selected from the group consisting of: deoxyribonuclease (DNase), SDS, TX-100, CTAB, and cesium chloride-ethidium bromide. In some embodiments, the DNase can non-specifically cleave linear DNA. In some embodiments, the DNase comprises a nuclease. In some embodiments, the optimization comprises contacting the exogenous nucleic acid molecules obtained from the host cells with the reagents in the presence of Mg²⁺ and Ca²⁺.

In another aspect, the present application provides a method for optimizing a transfection composition comprising a part of exogenous nucleic acid molecules obtained from host cells, wherein the method comprises: treating the part of exogenous nucleic acid molecules obtained from host cells with DNase. In some embodiments, the DNase can non-specifically cleave linear DNA. In some embodiments, the DNase comprises a nuclease. In some embodiments, the treatment comprises contacting the part of exogenous nucleic acid molecules obtained from host cells with the DNase in the presence of Mg²⁺ and Ca²⁺. In some embodiments, the transfection composition is as described in any aspect of the present application.

In another aspect, the present application provides a method for determining the quality of transfection composition, wherein the transfection composition comprises a part of exogenous nucleic acid molecules obtained from host cells, wherein the method comprises: measuring the content of host cell genomic DNA in the part of exogenous nucleic acid molecules obtained from host cells and determining the quality of the transfection composition based on the content. In some embodiments, the transfection composition is as described in any aspect of the present application. In some embodiments, the host is as described in any aspect of the present application.

In some embodiments, when the content of the host cell genomic DNA is less than about 10% (w/w), it is determined that the quality of the transfection composition meets the standard.

In some embodiments, when the content of the host cell genomic DNA is less than about 1% (w/w), it is determined that the quality of the transfection composition meets the standard.

In some embodiments, when the content of the host cell genomic DNA is less than about 0.9‰ (w/w), it is determined that the quality of the transfection composition meets the standard.

In some embodiments, the content of the host cell genomic DNA is measured by qPCR.

In some embodiments, the method of this application is *in vitro* or ex *vivo* method.

In another aspect, this application provides the use of the aforementioned reagents (e.g., deoxyribonuclease (DNase), SDS, TX-100, CTAB and/or cesium chloride-ethidium bromide) for optimizing transfection composition.

In another aspect, the present application provides a reagent kit for cell transfection (e.g., electroporation), which comprises: 1) exogenous nucleic acid molecule derived from host cell as described in any aspect of the present application; and 2) a reagent capable of reducing or degrading the genomic DNA of the host cells. In some embodiments, the reagent of 2) comprises one or more selected from the group consisting of deoxyribonuclease (DNase), SDS, TX-100, CTAB, and cesium chloride-ethidium bromide. In some embodiments, the DNase is capable of non-specifically cleaving linear DNA. In some embodiments, the DNase comprises a nuclease. In some embodiments, the reagent kit further comprises a reagent containing Mg²⁺ and Ca²⁺.

In another aspect, the present application provides a reagent kit, wherein the reagent kit comprises: 1) exogenous nucleic acid molecules derived from host cells as described in any aspect of the present application; and 2) an instruction, which describes the processing of the exogenous nucleic acid molecule derived from host cell in 1) by the methods of the present application and/or determining the quality of the transfection composition containing the exogenous nucleic acid molecule derived from host cell by the method of the present application.

In another aspect, the present application provides a transfection composition comprising exogenous nucleic acid molecules treated by the methods of the present application.

In another aspect, the present application provides a cell transfected with the transfection composition described in the present application. In some embodiments, the transfection comprises electroporating the cell.

In another aspect, the present application provides a cell modified by the method of the present application.

In another aspect, the present application provides a cell population comprising the cell and/or progeny thereof described in the present application.

In another aspect, the present application provides a pharmaceutical composition comprising the transfection composition described in the present application, the cell described in the present application, and/or the cell population described in the present application. In some embodiments, the pharmaceutical composition further comprises a pharmaceutically acceptable adjuvant.

In another aspect, the present application provides the use of the transfection composition described in the present application, the cell described in the present application, the cell population described in the present application, and/or the pharmaceutical composition described in the present application for the preparation of a medicament. In some embodiments, the medicament is for the prevention, treatment, and/or alleviation of cancer. In some embodiments, the medicament is for regulating immune responses.

In another aspect, the present application provides a method for preventing, treating, and/or alleviating a disease or disorder of a subject, which comprises administering an effective amount of the transfection composition described in the present application, the cell described in the present application, the cell population described in the present application, and/or the pharmaceutical composition described in the present application to the subject. In some embodiments, the disease or disorder is cancer. In some embodiments, the disease or disorder is related to the immune response of the subject.

Those skilled in the art can easily appreciate other aspects and advantages of the present application from the detailed description below. The detailed description below only shows and describes exemplary embodiments of the present application. As those skilled in the art will recognize, the disclosure of the present application enables those skilled in the art to modify the disclosed specific embodiments without departing from the spirit and scope of the inventions involved in the present application. Accordingly, the descriptions in the drawings and specification of the present application are merely exemplary and nonrestrictive.

### Brief Description of the Drawings

The specific features of the invention involved in the present application are as shown in the appended claims. The features and advantages of the invention involved in the present application may be better understood by referring to the detailed description of the exemplary embodiments and the drawings. Brief descriptions of the drawings are as follows:
Figure 1 shows the detection results of multiple parameters for plasmids from different sources.
Figure 2 shows the measurement of host genomic DNA content in plasmids from different sources.
Figures 3A-3C show the detection of cell viability, cell proliferation ability, and protein expression ability of cells transfected with plasmids containing different amounts of host genomic DNA.
Figure 4 shows the detection of cell viability and protein expression ability (i.e., integrated expression) of cells transfected with plasmids containing different amounts of host genomic DNA.
Figure 5A-5C show the results of detecting cell viability, cell proliferation ability, and protein expression ability of cells transfected with plasmids containing different amounts of host genomic DNA.
Figure 6 shows the results of detecting the *in vitro* tumor cell killing ability of CART prepared with plasmids containing different amounts of host genomic DNA.
Figure 7 shows the results of detecting the in vivo non-solid tumor cell killing ability of CART prepared with plasmids containing different amounts ofhost genomic DNA.
Figures 8A-8C show the results of cell survival rate, cell proliferation ability after transfection with plasmids containing less host genomic DNA; and the results of CART cell specific killing of tumor cells prepared with such plasmids.
Figure 9A-9C show the results of cell survival rate, cell proliferation ability, and protein expression ability after transfection with plasmids containing less host genomic DNA.
Figure 10 shows the results of specific killing of tumor cells by cells expressing CAR and BiTE prepared with plasmids containing less host genomic DNA.
Figure 11 shows the results of specific killing of tumor cells by culture supernatants of cells expressing CAR and BiTE prepared with plasmids containing less host genomic DNA.
Figure 12 shows the results of specific killing of tumor cells in vivo by cells expressing CAR and BiTE prepared with plasmids containing less host genomic DNA.
Figures 13A-13D show that reducing the content of host genomic DNA in the plasmid can significantly improve the cell survival rate, cell proliferation ability, and protein expression ability after transfection with the plasmid.
Figure 14 shows the *in vivo* tumor suppression effect of cells expressing CAR and IL-15 prepared with plasmids containing less host genomic DNA.
Figure 15 shows the *in vivo* tumor suppression effect of cells expressing CAR and IL-15 prepared with plasmids containing less host genomic DNA when the exogenous gene is integrated into the AAVS-I site.
Figure 16 shows the *in vivo* tumor suppression effect of cells expressing CAR, IL-7, and CCL-19 prepared with plasmids containing less host genomic DNA.
Figures 17A-17D show the transfection efficiency of non-activated T cells transfected with plasmids containing less host genomic DNA.
Figures 18A-18D show the transfection efficiency of activated T cells transfected with plasmids containing less host genomic DNA.
Figures 19A-19B show that T cells expressing CAR and with CD95 and PD-1 knocked out after transfection with plasmids containing less host genomic DNA can specifically kill tumor cells.

### Detailed description

The following specific examples illustrate the embodiments of the present invention, and those skilled in the art can easily understand other advantages and effects of the present invention from the content disclosed in this specification.

### Terms

In the present application, the term "transfection efficiency" generally refers to the relative amount of material introduced into the transfected cells and/or expressed by the transfected cells. In the present application, the transfection may refer to introducing one or more materials (e.g., polynucleotides) into cells. The introduced material may be stably or transitorily maintained in the transfected cells. In the present application, the transfection efficiency may be measured by the amount of the introduced material.

In the present application, the term "deoxyribonuclease (DNase)" generally refers to an enzyme capable of cleaving phosphodiester bonds on the DNA backbone. The DNase may be a type of nuclease. The DNase may digest double-stranded DNA into deoxynucleotides (e.g., under alkalescent conditions). For example, the DNase may have essentially no activity (e.g., unable to cleave) to circular or supercoiled nucleic acid molecules or fragment thereof (e.g., closed circular double-stranded DNA and/or supercoiled DNA). For example, the DNase can cleave linear double-stranded DNA. For example, the DNase may be a DNA exonuclease. For example, the DNase may be exonuclease V. For example, the DNase may be ATP-Dependent DNase, such as Plasmid-Safe^{™} ATP-Dependent DNase.

In the present application, the term "nucleic acid molecule or fragment thereof" generally refers to a nucleotide (e.g., ribonucleotide, deoxyribonucleotide, and/or modified forms thereof) or fragment thereof. The nucleic acid molecule or fragment thereof may comprise the polymer form or fragment of nucleotides. The nucleic acid molecule or its fragment may sometimes be interchangeably used with "polynucleotide". The nucleic acid molecule may comprise DNA, RNA, cDNA, sense strands and antisense strands of genomic DNA, and the synthetic forms, mixtures, and/or polymers thereof. The nucleic acid molecule or its fragment can comprise any topological conformations, such as single-stranded, double-stranded, partially double-stranded, triple-stranded, hairpin structures, circular, and/or padlock conformations. The nucleotides in the nucleic acid molecule or fragment thereof may be natural or modified. The nucleotides in the nucleic acid molecule or fragment thereof may be connected by naturally occurring and/or non-naturally occurring nucleic acid bonds.

In the present application, the term "exogenous nucleic acid molecule" typically refers to a nucleic acid molecule that is not directly produced within an organism, tissue, or cell. For example, the nucleic acid molecule is introduced into an organism, tissue, or cell in certain form from the outside. The structure, composition, or function of the nucleic acid molecule may be the same or different from the corresponding nucleic acid molecule endogenously expressed. For example, in some cases, the exogenous nucleic acid molecule may have the same nucleotide sequence as the corresponding nucleic acid molecule endogenously expressed. In some cases, the exogenous nucleic acid molecule is different from any endogenously expressed nucleic acid molecules.

In the present application, the terms "transfection composition" and "transfection mixture" can be used interchangeably, and typically refer to a mixture required for transfection. In the present application, the transfection mixture may comprise one or more materials to be introduced, such as polynucleotides or exogenous nucleic acid molecules. For example, the transfection mixture may contain an exogenous nucleic acid molecule encoding the exogenous gene to be transfected. For example, the transfection mixture may comprise a vector containing the nucleic acid molecule. For example, the transfection mixture may also comprise impurities (in some cases, the impurities may comprise nucleic acid molecules or fragments thereof). In the present application, the impurities may comprise nucleic acid molecules or fragments thereof from microbial sources. The impurities associated with the vector can comprise impurities associated with the vector (e.g., nucleic acid molecules or fragments thereof homologous or heterologous to the vector backbone).

In this application, the terms "total nucleic acid molecule content" and "total DNA content" can be used interchangeably, and generally refer to the total mass of all substances containing nucleotides in the transfection mixture. For example, the substances containing nucleotides can comprise the nucleic acid molecules or fragment thereof and the materials.

In this application, the term "gene editing efficiency" typically refers to the proportion of nucleic acid molecules with cuts at the target location produced by gene editing means among all nucleic acid molecules treated by the gene editing means. The gene editing efficiency may reflect the ability of the gene editing to affect on the target location.

In the present application, the term "DNA homologous recombination efficiency" typically refers to the proportion of individuals (e.g., the number of cells) undergoing DNA homologous recombination among the total individuals used for DNA homologous recombination. The DNA homologous recombination efficiency can be verified by means such as gene sequencing and detection of corresponding protein expression.

In the present application, the term "cell viability" typically refers to the ability of a cell to survive and/or perform biological functions (e.g., division, proliferation, secretion, killing, preservation, and/or recovery) under certain conditions. In some cases, cell viability may be measured by the proportion of live cells among the total number of live and dead cells at a specific time and condition. In the present application, cell viability can also be measured by the proportion of cells with a certain biological function and/or activity among the total number of cells at a specific time and condition.

In the present application, the term "host" typically refers to an organism used to carry, amplify, or produce the exogenous nucleic acid molecule to be transfected, which may comprise host cells, such as microbial or mammalian cells.

In the present application, the term "microorganism" typically refers to eukaryotic and prokaryotic microbial species from the domains *Archaea, Bacteria,* and/or *Eucarya.* For example, the microorganism may comprise bacteria, viruses, fungi, actinomycetes, rickettsiae, mycoplasmas, chlamydiae, and/or spirochetes. In the present application, the term bacteria typically refers to any type of prokaryotic organism, comprising prokaryotic organisms from all phyla within the kingdom Prokaryotae. The bacteria may comprise cocci, bacilli, spirilla, protoplasts, and mycoplasmas. The bacteria may comprise Gram-positive and Gram-negative bacteria. "Gram-negative" and "Gram-positive" refer to staining patterns using the Gram staining method, which is well known in the art (see, for example, Finegold and Martin, Diagnostic Microbiology, 6th edition, CV Mosby St. Louis, pp. 13-15 (1982)).

In this application, the term "host cell genome DNA" typically refers to the DNA molecule or fragment thereof from host cell genome.

In the present application, the term "nucleic acid molecules or fragments thereof derived from the genome of a host (e.g., microorganism)" and "host genomic DNA" may be used interchangeably, generally referring to nucleic acid molecules or fragments thereof, which are derived from the genome of the microorganism. Information on the genomes of hosts (e.g., microorganisms) may be found in A genomic catalog of Earth's microbiomes, Nature Biotechnology (2020).

In the present application, the term "Gram-negative bacteria" generally refers to bacteria that do not retain the primary dye used in Gram staining but are stained by the counterstain. Thus, Gram-negative bacteria typically appear red in Gram staining. The cell walls of said Gram-negative bacteria have a lower content of peptidoglycan and a higher content of lipids. For example, the cell wall of said Gram-negative bacteria may have a lipopolysaccharide layer. For example, the Gram-negative bacteria may comprise *Escherichia coli, Pseudomonas aeruginosa, Proteus species, Shigella species, Klebsiella pneumoniae, Brucella species, Haemophilus influenzae, Haemophilus parainfluenzae, Campylobacter species, Acinetobacter species, Yersinia species, Legionella pneumophila, Bordetella pertussis, Bordetella parapertussis, Shigella species, Pasteurella species, Vibrio cholerae, Aeromonas hydrophila,* and/or *Citrobacter freundii.*

In the present application, the term " *E*. *coli"* generally refers to *Escherichia coli,* which belongs to the family *Enterobacteriaceae* and the genus *Escherichia.*

In the present application, the term "transient transfection" generally refers to a mode of transfection in which the exogenous gene introduced into the cell by transfection is not integrated into the cell's own genome. The transient transfection can achieve rapid expression of the exogenous gene for a short period (e.g., at least about 1 day, at least about 2 days). The exogenous gene transfected into the cell may be gradually lost with the growth and/or division of the cell. The transient transfection may have advantages selected from the following group: simple operation, short experimental cycle, high expression efficiency, safety, and no need for gene screening. The operation steps of the transient transfection may be known to those skilled in the art. For example, the transient transfection may be performed by liposome-mediated transfection. For example, the transient transfection may comprise electroporation. The transient transfection may use transfection reagents, such as FuGENE6.

In the present application, the term "stable transfection" generally refers to the introduction of exogenous nucleic acid molecules into a cell and their integration into the genome of the transfected cell. For example, the integration of the exogenous gene into the genome of the transfected cell.

In the present application, the term "stem cell" generally refers to a class of undifferentiated cells with the ability for self-renewal and retaining various degrees of potential to form differentiated cells and tissues. The stem cells may be unipotent stem cells, multipotent stem cells, or pluripotent stem cells. The unipotent stem cells therein are derived stem cells that have lost their differentiation ability. The pluripotent stem cells can form all cells and tissues found in a complete organism. For example, the pluripotent stem cells can form a complete organism.

In the present application, the terms "multipotent stem cell" and "multi-function stem cell" may be used interchangeably, generally referring to stem cells that have the ability to form cells and tissues ultimately found in a complete organism but cannot form a complete organism. For example, the multipotent stem cells can proliferate extensively or virtually indefinitely *in vitro* while maintaining their undifferentiated state and exhibiting a normal karyotype (chromosomes). The multipotent stem cells may have the ability to differentiate into all three germ layers (ectoderm, mesoderm, and endoderm) under appropriate conditions. For example, the multipotent stem cells may comprise embryonic stem (ES) cells isolated from early embryos and/or embryonic germ (EG) cells isolated from fetal germ cells.

In the present application, the term "mesenchymal stem cell" generally refers to cells capable of producing mesenchymal lineages. The mesenchymal stem cells may be considered as a type of multipotent stem cell. The mesenchymal stem cells may produce one or more mesenchymal lineage cells. The mesenchymal lineage cells may be derived from different tissues, for example, from bone marrow tissue, adipose tissue, muscle tissue, reproductive tissue (e.g., amniotic membrane, amniotic fluid, or umbilical cord tissue), skin tissue, and/or dental tissue.

In the present application, the term "immune cell" generally refers to cells that play a role in immune responses. The immune cells may comprise lymphocytes, monocytes, and/or granulocytes, as well as their precursors and/or mature derivatives. The immune cells may comprise T cells, B cells, Th cells, natural killer cells, monocytes, macrophages, eosinophils, basophils, mast cells, dendritic cells, and/or granulocytes. The immune cells may comprise immune effector cells. The immune effector cells may participate in immune responses, such as promoting immune effector responses. The immune effector cells may comprise T cells, for example, α/βT cells and γ/δT cells, B cells, natural killer (NK) cells, natural killer T (NKT) cells, mast cells, and bone marrow-derived phagocytes.

In the present application, the term "fibrocyte" generally refers to functionally inactive fibroblasts. The fibrocytes may transform into fibroblasts (e.g., they may participate in the repair process when tissue is damaged). The fibroblasts (also called fibroblast cells) may secrete structural proteins that constitute the extracellular matrix. The fibroblasts may be closely related to physiological or pathological processes such as wound healing and fibrosis.

In the present application, the term "muscle cell" generally refers to a cell or group of cells originating from muscle tissue. The muscle cells may originate from skeletal muscle, smooth muscle (e.g., from the gastrointestinal tract, bladder, and blood vessels), and cardiac muscle cells and tissues. The muscle cells may comprise *in vitro* and *in vivo* myocytes. The muscle cells may also comprise differentiated and undifferentiated muscle cells such as myocytes (e.g., myotubes), divided and differentiated myoblasts, cardiomyocytes, and cardiomyogenic cells-derived myocytes and muscle tissues.

In the present application, the term "plasmid" generally refers to a construct containing genetic material. The plasmid may be designed to deliver genetic material (e.g., one or more nucleic acid sequences) into cells. The plasmid may comprise autonomously replicating sequences of single-stranded or double-stranded nucleic acids (e.g., DNA or RNA) from any source. The term "plasmid" may be used interchangeably with the term "vector" in the present application. The plasmid may have different conformations, such as linear plasmids, circular plasmids, or supercoiled plasmids. The linear plasmids may be linear DNA molecules. The supercoiled plasmids may comprise two nucleic acid strands maintaining an intact structure (e.g., they may comprise covalently closed circular DNA, cccDNA) and exhibit a supercoiled conformation. The circular plasmids may have at least one nucleic acid strand maintaining a circular structure. The plasmid may not be integrated into the cell's genome.

In the present application, the term "multiple cloning site" or "MCS" generally refers to a nucleic acid sequence containing at least one restriction site. The multiple cloning site may link nucleic acid molecules to the vector described in the present application, e.g., insertion of nucleic acid molecules at specified sites may be achieved through the restriction site. The restriction site may be a recognition site for a restriction endonuclease. For example, the restriction endonuclease may be AcIU, Hindlll, Sspl, MLuCI, Tsp5091, Pcil, Agel, BspMI, BfuAI, SexAI, MLuI, BceAI, HpyCH4IV, HpyCH4III, Bael, BsaXI, Spel, Bsrl, Bmrl, BgIII, Afel, AluI, Stul, Scal, Clal, BspDI, PI-SceI, Nsil, Asel, Swal, CspCI, Mfel, BssSI, BmgBI, Pmll, Dralll, AleI, EcoP151, Pvull, AlwNI, or BtsCI.

In the present application, the term "exogenous promoter" generally refers to a promoter not originating from the host in which it is placed. The exogenous promoter may be transfected and/or inserted into the host cell (e.g., the cell's genome) in which it is placed. The promoter may be a recognition site for RNA polymerase binding to a polynucleotide (DNA or RNA). The RNA polymerase may effectively catalyze the assembly of messenger RNA complementary to the appropriate DNA strand of the coding region. The number of promoters may be one or more.

In the present application, the term "gene editing" generally refers to the operation of nucleic acid insertion, deletion, and/or replacement in the genome. The gene editing may be achieved through homology-directed repair (HDR), non-homologous end joining (NHEJ), or single base editing. The gene editing may employ gene editing tools familiar to those skilled in the art, such as using zinc finger nuclease systems (ZFN), TALEN systems, and/or CRISPR technology.

In the present application, the term "gene editing knock-in" generally refers to a genetic engineering process (which may be called knock-in), involving the one-to-one replacement or insertion of DNA sequence information at a genetic locus with sequence information not found in the endogenous locus. The gene editing knock-in can utilize homologous recombination. For example, in some cases, functional exogenous genes (not originally present in the genome ordeactivated genes) may be introduced into cells and undergo homologous recombination with homologous sequences in the genome to be inserted and expressed in cells. For example, the gene editing knock-in may partially displace the cellular genome with an exogenous gene. The gene editing knock-in may use CRISPR technology to achieve "targeted knock-in." Additionally, the gene editing knock-in can use transposon and transposase systems, such as PiggyBac (PB) transposase and/or Sleeping Beauty (SB) transposase.

In the present application, the term "donor plasmid" generally refers to a plasmid that can transcribe and/or translate the encoded exogenous gene in the introduced cell. For example, the donor plasmid may be suitable for gene editing knock-in. For example, the donor plasmid may comprise a nucleic acid molecule encoding the exogenous gene. The donor plasmid may also comprise elements such as promoters to regulate the expression of the exogenous gene (for example, occurring at the transcriptional and/or translational levels of production and/or accumulation). The donor plasmid may be suitable for eukaryotic expression systems.

In the present application, the term "exogenous gene to be knocked-in" generally refers to a heterologous gene that may be introduced by gene editing knock-in. The exogenous gene to be knocked-in may be integrated into the introduced object (e.g., cells, such as within a subject). The exogenous gene to be knocked-in may comprise genes integrated into the genome of the introduced object. The exogenous gene to be knocked-in may be a natural gene from different species or an engineered gene (for example, a chimeric gene).

In the present application, the term "antibody" generally refers to an immunoglobulin reactive to a specific protein or peptide or fragment thereof. Antibodies may be from any class, comprising but not limited to IgG, IgA, IgM, IgD, and IgE, and from any subclass (e.g., IgG1, IgG2, IgG3, and IgG4). Antibodies may have a constant region of the heavy chain selected from, for example, IgG1, IgG2, IgG3, or IgG4. Antibodies may also have a light chain selected from, for example, kappa (κ) or lambda (λ). Antibodies in the present application may be derived from any species.

In the present application, the term "antigen-binding fragment" generally refers to a portion of an antibody molecule that contains amino acid residues, which interact with an antigen and provide the antibody with specificity and affinity for the antigen. Examples of antigen-binding fragments may comprise, but are not limited to, Fab, Fab', F(ab)2, Fv fragments, F(ab')2, scFv, di-scFv, and/or dAb. In the present application, the term "Fab" generally refers to a fragment containing a heavy chain variable domain and a light chain variable domain, as well as the constant domain of the light chain and the first constant domain (CH1) of the heavy chain; the term "Fab'" generally refers to a fragment different from Fab by the addition of a small number of residues (comprising one or more cysteines from the antibody hinge region) at the carboxyl end of the heavy chain CH1 domain; the term "F(ab')₂" generally refers to a dimer of Fab', an antibody fragment containing two Fab fragments connected by disulfide bonds in the hinge region. The term "Fv" generally refers to the smallest antibody fragment that contains the complete antigen recognition and binding site. In some cases, this fragment may be composed of a dimer of a heavy chain variable region and a light chain variable region in tight non-covalent binding; the term "dsFv" generally refers to disulfide-stabilized Fv fragments, in which the bond between a single light chain variable region and a single heavy chain variable region is a disulfide bond. The term "dAb fragment" generally refers to an antibody fragment composed of a VH domain. In the present application, the term "scFv" generally refers to a monovalent molecule formed by covalently linking one heavy chain variable domain and one light chain variable domain of an antibody via a flexible peptide linker; such scFv molecules may have a general structure: NH₂-VL-linker-VH-COOH or NH₂-VH-linker-VL-COOH.

In the present application, the term "bispecific antibody" generally refers to an antibody with a variable region capable of recognizing one or more epitopes on one or more antigens. Bispecific antibodies comprise, but are not limited to, full-length antibodies, antibodies with two or more VL and VH domains, antibody fragments such as Fab, Fv, dsFv, scFv, bi-antibodies, as well as covalently or non-covalently linked antibody fragments. In some cases, the bispecific antibody may recognize two different epitopes on the same or different antigens. In some cases, the bispecific antibody may recognize two different antigens. In the present application, multispecific antibodies comprise bispecific or trispecific antibodies, or their antigen-binding fragments.

In the present application, the term "antigen-binding domain" generally refers to a domain capable of binding to a target antigen. Antigen-binding domains may comprise currency and antigen receptors and fragment thereof, antibodies or their antigen-binding fragments. Antigen-binding domains may be domains capable of binding to tumor-associated antigens, and in the present application, the tumor-associated antigens may comprise but are not limited to: CD19, CD20, CD22, CD123, CD33/IL3Ra, CD138, CD33, BCMA, CS1, C-Met, EGFRvIII, CEA, Her2, GD2, MAG3, GPC3, Claudin18.2, Mesothelin, and NY-ESO-1.

In the present application, the term "chimeric antigen receptor" generally refers to a fusion protein comprising an extracellular domain capable of binding to an antigen and at least one intracellular domain. CAR is the core component of chimeric antigen receptor T cells (CAR-T), which may comprise antigen (e.g., tumor-specific antigen and/or tumor-associated antigen) binding domains, transmembrane domains, costimulatory domains, and intracellular signaling domains. In the present application, the CAR can combine with the T cell receptor activation intracellular domain based on the antigen (e.g., CD19) specificity of an antibody. Genetically modified T cells expressing CAR can specifically recognize and eliminate malignant cells expressing the target antigen. Descriptions of CAR and CAR-T cells can see, for example, Sadelain M, Brentjens R, Rivière I. The basic principles of chimeric antigen receptor design. Cancer Discov. 2013;3(4):388-398; Turtle CJ, Hudecek M, Jensen MC, Riddell SR. Engineered T cells for anti-cancer therapy. Curr Opin Immunol. 2012;24(5):633-639; Dotti G, Gottschalk S, Savoldo B, Brenner MK. Design and development of therapies using chimeric antigen receptor-expressing T cells. Immunol Rev. 2014;257(1):107-126.

In the present application, the term "BiTE" generally refers to bispecific T cell engagers. The BiTE may be a single polypeptide chain molecule with two antigen-binding structural domains, one of which binds to a T cell antigen and the other binds to an antigen present on the surface of a target cell (see WO05/061547; Baeuerle, P et al. (2008) "BiTE®: A New Class Of Antibodies That Recruit T Cells Drugs of the Future 33:137-147; or Bargou et al. (2008) "Tumor Regression in Cancer Patients by Very Low Doses of a T Cell-Engaging Antibody/"Science 321:974-977).

In the present application, the term "polyprotein" generally refers to a polypeptide chain that comprises multiple protein molecules, which may exist in tandem in the polypeptide chain. In the polypeptide chain, any two protein molecules may be optionally separated by a cleavable part (e.g., 2A peptide).

In the present application, the term "homology arm" generally refers to a polynucleotide suitable for targeting an exogenous gene to be knocked-in in a donor plasmid to the genome via homologous recombination. The homologous recombination may refer to recombination occurring between sister chromatins or between or within DNA molecules with homologous sequences on the same chromosome. The homology arm may have two parts (e.g., a 5' homology arm and/or a 3' homology arm). The homology arms may be located upstream and downstream of the exogenous gene to be knocked into the donor plasmid. In some cases, the target site of the exogenous gene to be knocked into the genome may be cleaved due to the action of a nucleic acid nuclease. The homology arm may be completely identical to or have at least 80% identity with the DNA sequence at the 5' and/or 3' ends of the cleavage site at the target location. For example, the homology arm may have at least about 80%, at least about 85%, at least about 90%, at least about 91%, at least about 92%, at least about 93%, at least about 94%, at least about 95%, at least about 96%, at least about 97%, at least about 98%, at least about 99%, or 100% identity with the DNA sequence at the ends of the cleavage site at the target location. In the present application, the donor plasmid may comprise a 5' homology arm and/or a 3' homology arm.

In the present application, the term "cell survival rate" generally refers to the ability of cells to maintain their survival under certain conditions. The cell survival rate may be measured by the proportion of living cells to the total number of cells present at that time under those conditions within a certain period of time. The cell can reflect the effects of certain conditions on cells. For example, the higher the cell survival rate, the more favorable the condition for cell survival.

In the present application, the term "cell proliferation capacity" generally refers to the ability of cells to proliferate and/or self-replicate. The cell proliferation capacity may be measured by the total number of cells produced by cell proliferation within a certain period of time. For example, the more cells increase in number within a certain period of time, the higher the cell proliferation capacity. The cell proliferation capacity may also be reflected in the improvement of cell functions (e.g., proliferation).

In the present application, the term "cell killing capacity" generally refers to the killing capacity of cells against their target cells. For example, immune effector cells (e.g., T cells, NK cells) may mediate the killing of target cells (e.g., tumor cells) and kill target cells. For example, the mediation may comprise the localization of immune effector cells to target cells (e.g., through the mutual recognition of molecules and/or epitopes expressed by immune effector cells and target cells). The cell killing capacity may be measured by the number of target cells killed by cell-mediated killing within a certain period of time and under certain conditions.

In the present application, the term "cell secretion capacity" generally refers to the ability of cells to secrete and produce secretory factors. For example, the secretory factors may be molecules that leave the cell due to secretion. The secretory factors may comprise proteins encoded by exogenous genes. The cell secretion capacity may be measured by the quantity of secretory factors produced by cell secretion within a certain period of time and under certain conditions.

In the present application, the term "cell preservation capability" generally refers to the ability of cells to survive and/or retain their original function under preservation conditions. For example, the preservation conditions may comprise long-term storage at ambient temperature or low temperature (e.g., non-refrigerated temperature or refrigerated temperature, such as under liquid nitrogen conditions). Preservation may reduce the metabolic level of the cells and may temporarily suspend their growth state. The cells may be revived after preservation. The cell preservation capability may be measured by the ratio of the cell number after preservation for a certain time and under certain conditions to the cell number before preservation.

In the present application, the term "cell revival capability" generally refers to the ability of cells to recover growth after re-cultivation. The re-cultivation may refer to re-culturing cells after thawing them from liquid nitrogen or a -80°C freezer. The cell revival capability may be measured by the ratio of the number of cells that recover their growth ability after re-cultivation to the total number of cells subjected to re-cultivation under certain time and conditions.

In the present application, the term "cell line" generally refers to a clonal population of cells capable of continuous division. For example, the cell line may have acquired the ability to proliferate indefinitely *in vitro.*

In the present application, the term "complementary" generally refers to Watson-Crick base pairing between nucleotides, and specifically refers to nucleotides that hydrogen bond with each other, in which thymine or uracil residues are connected to adenine residues by two hydrogen bonds, and cytosine and guanine residues are connected by three hydrogen bonds. Typically, nucleic acids comprise nucleotide sequences described as having a "percentage of complementarity" with a specified second nucleotide sequence. For example, a nucleotide sequence may have 80%, 90%, or 100% complementarity with a specified second nucleotide sequence, indicating that 8, 9, or all 10 nucleotides out of a sequence of 10 nucleotides are complementary to the specified second nucleotide sequence. For example, the nucleotide sequence 3'-TCGA-5' is 100% complementary to the nucleotide sequence 5'-AGCT-3'. Additionally, a region of the nucleotide sequence 3'-TCGA- is 100% complementary to a region of the nucleotide sequence 5'-TTAGCTGG-3'. Those skilled in the art will recognize that two complementary nucleotide sequences comprise a sense strand and an antisense strand.

In the present application, the terms "homology", "identity", or "similarity" generally refer to sequence similarity between two peptides or between two nucleic acid molecules. The term "homologous region" generally refers to a region on a donor molecule that has a certain degree of homology with the target sequence. Homology may be determined by comparing the positions in each sequence. For example, homology between sequences may be determined by performing sequence alignment. When the positions in the compared sequences are occupied by the same base or amino acid, the molecules are homologous at that position. The degree of homology between sequences is a function of the number of matching or homologous positions shared by the sequences. "Unrelated" or "non-homologous" sequences have less than 40% identity with one of the sequences of the present application, although preferably less than 25% identity. A polynucleotide or polynucleotide region (or polypeptide or polypeptide region) has a certain percentage (e.g., 60%, 65%, 70%, 75%, 80%, 85%, 90%, 95%, 98%, or 99%) of "sequence identity" or "homology" with another sequence, meaning that in alignment, the percentage of bases (or amino acids) in the two sequences being compared are the same. This alignment and percentage homology or sequence identity may be determined using software programs known in the art, such as those described in Ausubel et al. (2007), Current Protocols in Molecular Biology.

In the present application, the term "transfection" generally refers to methods for introducing biologically active substances (e.g., nucleic acids, proteins, enzymes, or small molecules) into cells. Nucleic acids may be DNA (as plasmid or oligomer delivery) and/or RNA or combinations thereof.

In the present application, the term "electroporation" generally refers to a transfection method that involves applying an external electric field to cells. In some embodiments, the electroporation method used is electrostatic poration.

Unless otherwise specified in the context, in the present application, the terms "comprise," "have," and "comprise" may be used interchangeably, generally referring to the possibility of comprising other components, elements, values, steps, etc. In some cases, "comprise" covers the cases of "for" and "composed of," for example, based on a record of a composition "comprising" component "A," those skilled in the art can infer that, in some cases, the composition may be composed solely of component A.

In the present application, the term "about" generally refers to within 30%, 25%, 20%, 15%, 10%, 9%, 8%, 7%, 6%, 5%, 4%, 3%, 2%, 1%, 0.5%, or 0.05% of the specified value or numerical range involved in the present application. In the present application, when the term "about" is placed before the first value of two or more values, it applies to each value in the series.

### Detailed Description of the Invention

In one aspect, the present application provides a method for improving transfection (e.g., electroporation) efficiency, comprising the following steps: making the content of nucleic acid molecules or fragment thereof with a size of at least about 10kb (e.g., at least about 15kb, at least about 20kb, at least about 30kb, at least about 35kb, at least about 40kb, at least about 48kb, at least about 50kb, at least about 100kb, at least about 150kb, at least about 200kb, at least about 250kb, at least about 300kb, at least about 350kb, at least about 400kb, at least about 450kb, at least about 500kb, at least about 600kb, at least about 700kb, at least about 800kb, at least about 900kb, at least about 1Mb, at least about 2Mb, at least about 3Mb, at least about 4Mb, at least about 5Mb, at least about 6Mb, at least about 7Mb, at least about 8Mb, at least about 9Mb, at least about 10Mb, at least about 20Mb, at least about 50Mb, at least about 100Mb, at least about 200Mb or larger) account for less than 10% of the total nucleic acid content of the transfection mixture (e.g., about 10% or less, about 9% or less, about 8% or less, about 7% or less, about 6% or less, about 5% or less, about 4% or less, about 3% or less, about 2.5% or less, about 2% or less, about 1.5% or less, about 1% or less, about 9‰ or less, about 8‰ or less, about 7‰ or less, about 6‰ or less, about 5.5‰ or less, about 5‰ or less, about 4.5‰ or less, about 4‰ or less, about 3.5‰ or less, about 3‰ or less, about 2.5‰ or less, about 2‰ or less, about 1.5‰ or less, about 1‰ or less, about 0.5‰ or less, about 0.1‰ or less, about 0.01‰ or less, about 0.001‰ or less or lower). The percentage may be weight percentage w/w%.

In another aspect, the present application provides a method for improving gene editing efficiency (e.g., *ex vivo* gene editing of cells), comprising the following steps: making the content of nucleic acid molecules or fragments thereof with a size of at least about 10kb (e.g., at least about 15kb, at least about 20kb, at least about 30kb, at least about 35kb, at least about 40kb, at least about 48kb, at least about 50kb, at least about 100kb, at least about 150kb, at least about 200kb, at least about 250kb, at least about 300kb, at least about 350kb, at least about 400kb, at least about 450kb, at least about 500kb, at least about 600kb, at least about 700kb, at least about 800kb, at least about 900kb, at least about 1Mb, at least about 2Mb, at least about 3Mb, at least about 4Mb, at least about 5Mb, at least about6Mb, at least about 7Mb, at least about 8Mb, at least about 9Mb, at least about 10Mb, at least about 20Mb, at least about 50Mb, at least about 100Mb, at least about 200Mb or larger) account for less than 10% of the total nucleic acid content of the transfection mixture (e.g., about 10% or less, about 9% or less, about 8% or less, about 7% or less, about 6% or less, about 5% or less, about 4% or less, about 3% or less, about 2.5% or less, about 2% or less, about 1.5% or less, about 1% or less, about 9‰ or less, about 8‰ or less, about 7‰ or less, about 6‰ or less, about 5.5‰ or less, about 5‰ or less, about 4.5‰ or less, about 4‰ or less, about 3.5‰ or less, about 3‰ or less, about 2.5‰ or less, about 2‰ or less, about 1.5‰ or less, about 1‰ or less, about 0.5‰ or less, about 0.1‰ or less, about 0.01‰ or less, about 0.001‰ or less or lower). The percentage may be weight percentage w/w%.

In another aspect, the present application provides a method for improving DNA homologous recombination efficiency (for example, *in vitro* homologous recombination in cells), comprising the following steps: allowing the content of nucleic acid molecules or fragments thereof having a size of at least about 10kb (for example, may be at least about 15kb, at least about 20kb, at least about 30kb, at least about 35kb, at least about 40kb, at least about 48kb, at least about 50kb, at least about 100kb, at least about 150kb, at least about 200kb, at least about 250kb, at least about 300kb, at least about 350kb, at least about 400kb, at least about 450kb, at least about 500kb, at least about 600kb, at least about 700kb, at least about 800kb, at least about 900kb, at least about 1Mb, at least about 2Mb, at least about 3Mb, at least about 4Mb, at least about 5Mb, at least about 6Mb, at least about 7Mb, at least about 8Mb, at least about 9Mb, at least about 10Mb, at least about 20Mb, at least about 50Mb, at least about 100Mb, at least about 200Mb or larger) to account for less than 10% of the total content of nucleic acid molecules in the transfection mixture (for example, may be about 10% or less, about 9% or less, about 8% or less, about 7% or less, about 6% or less, about 5% or less, about 4% or less, about 3% or less, about 2.5% or less, about 2% or less, about 1.5% or less, about 1% or less, about 9‰ or less, about 8‰ or less, about 7‰ or less, about 6‰ or less, about 5.5‰ or less, about 5‰ or less, about 4.5‰ or less, about 4‰ or less, about 3.5‰ or less, about 3‰ or less, about 2.5‰ or less, about 2‰ or less, about 1.5‰ or less, about 1‰ or less, about 0.5‰ or less, about 0.1‰ or less, about 0.01‰ or less, about 0.001‰ or less or lower). The percentage may be weight percentage w/w%.

In another aspect, the present application provides a method for avoiding a decrease in cell viability after transfection, comprising the following steps: making the content of nucleic acid molecules or fragments thereof having a size of at least about 10kb (for example, may be at least about 15kb, at least about 20kb, at least about 30kb, at least about 35kb, at least about 40kb, at least about 48kb, at least about 50kb, at least about 100kb, at least about 150kb, at least about 200kb, at least about 250kb, at least about 300kb, at least about 350kb, at least about 400kb, at least about 450kb, at least about 500kb, at least about 600kb, at least about 700kb, at least about 800kb, at least about 900kb, at least about 1Mb, at least about 2Mb, at least about 3Mb, at least about 4Mb, at least about 5Mb, at least about 6Mb, at least about 7Mb, at least about 8Mb, at least about 9Mb, at least about 10Mb, at least about 20Mb, at least about 50Mb, at least about 100Mb, at least about 200Mb or larger) to account for less than 10% of the total content of nucleic acid molecules in the transfection mixture (for example, may be about 10% or less, about 9% or less, about 8% or less, about 7% or less, about 6% or less, about 5% or less, about 4% or less, about 3% or less, about 2.5% or less, about 2% or less, about 1.5% or less, about 1% or less, about 9‰ or less, about 8‰ or less, about 7‰ or less, about 6‰ or less, about 5.5‰ or less, about 5‰ or less, about 4.5‰ or less, about 4‰ or less, about 3.5‰ or less, about 3‰ or less, about 2.5‰ or less, about 2‰ or less, about 1.5‰ or less, about 1‰ or less, about 0.5‰ or less, about 0.1‰ or less, about 0.01‰ or less, about 0.001‰ or less or lower). The percentage may be weight percentage w/w%.

In another aspect, the present application provides a method for preparing a transfection mixture, comprising the following steps: making the content of nucleic acid molecules with a size of at least about 10kb (for example, may be at least about 15kb, at least about 20kb, at least about 30kb, at least about 35kb, at least about 40kb, at least about 48kb, at least about 50kb, at least about 100kb, at least about 150kb, at least about 200kb, at least about 250kb, at least about 300kb, at least about 350kb, at least about 400kb, at least about 450kb, at least about 500kb, at least about 600kb, at least about 700kb, at least about 800kb, at least about 900kb, at least about 1Mb, at least about 2Mb, at least about 3Mb, at least about 4Mb, at least about 5Mb, at least about 6Mb, at least about 7Mb, at least about 8Mb, at least about 9Mb, at least about 10Mb, at least about 20Mb, at least about 50Mb, at least about 100Mb, at least about 200Mb or larger) to account for about 10% or less of the total nucleic acid molecule content of the transfection mixture (for example, may be about 10% or less, about 9% or less, about 8% or less, about 7% or less, about 6% or less, about 5% or less, about 4% or less, about 3% or less, about 2.5% or less, about 2% or less, about 1.5% or less, about 1% or less, about 9‰ or less, about 8‰ or less, about 7‰ or less, about 6‰ or less, about 5.5‰ or less, about 5‰ or less, about 4.5‰ or less, about 4‰ or less, about 3.5‰ or less, about 3‰ or less, about 2.5‰ or less, about 2‰ or less, about 1.5‰ or less, about 1‰ or less, about 0.5‰ or less, about 0.1‰ or less, about 0.01‰ or less, about 0.001‰ or less or lower). The percentage may be a weight percentage w/w%.

In another aspect, the present application provides a method for determining the quality of a transfection mixture, comprising the following steps: determining whether the content of nucleic acid molecules or fragments thereof with a size of at least about 10kb (for example, may be at least about 15kb, at least about 20kb, at least about 30kb, at least about 35kb, at least about 40kb, at least about 48kb, at least about 50kb, at least about 100kb, at least about 150kb, at least about 200kb, at least about 250kb, at least about 300kb, at least about 350kb, at least about 400kb, at least about 450kb, at least about 500kb, at least about 600kb, at least about 700kb, at least about 800kb, at least about 900kb, at least about 1Mb, at least about 2Mb, at least about 3Mb, at least about 4Mb, at least about 5Mb, at least about 6Mb, at least about 7Mb, at least about 8Mb, at least about 9Mb, at least about 10Mb, at least about 20Mb, at least about 50Mb, at least about 100Mb, at least about 200Mb or larger) accounts for about 10% or less of the total nucleic acid molecule content of the transfection mixture (for example, may be about 10% or less, about 9% or less, about 8% or less, about 7% or less, about 6% or less, about 5% or less, about 4% or less, about 3% or less, about 2.5% or less, about 2% or less, about 1.5% or less, about 1% or less, about 9‰ or less, about 8‰ or less, about 7‰ or less, about 6‰ or less, about 5.5‰ or less, about 5‰ or less, about 4.5‰ or less, about 4‰ or less, about 3.5‰ or less, about 3‰ or less, about 2.5‰ or less, about 2‰ or less, about 1.5‰ or less, about 1‰ or less, about 0.5‰ or less, about 0.1‰ or less, about 0.01‰ or less, about 0.001‰ or less or lower). The percentage may be a weight percentage w/w%.

In another aspect, the present application provides a method for increasing transfection efficiency (e.g., an *in vitro* method), comprising the following steps: making the content of nucleic acid molecules or fragments thereof originating from a host (e.g., a microorganism) genome account for about 10% or less of the total nucleic acid molecules content in the transfection mixture (for example, it may be about 10% or less, about 9% or less, about 8% or less, about 7% or less, about 6% or less, about 5% or less, about 4% or less, about 3% or less, about 2.5% or less, about 2% or less, about 1.5% or less, about 1% or less, about 0.9‰ or less, about 0.8‰ or less, about 0.7‰ or less, about 0.6‰ or less, about 0.55‰ or less, about 0.5‰ or less, about 0.45‰ or less, about 0.4‰ or less, about 0.35‰ or less, about 0.3‰ or less, about 0.25‰ or less, about 0.2‰ or less, about 0.15‰ or less, about 0.1‰ or less, about 0.05‰ or less, about 0.01‰ or less, about 0.001‰ or less or lower). The percentage may be a weight percentage (w/w%).

In another aspect, the present application provides a method for increasing gene editing efficiency (e.g., an *in vitro* method), comprising the following steps: making the content of nucleic acid molecules or fragments thereof originating from a host (e.g., a microorganism) genome account for about 10% or less of the total nucleic acid molecules content in the transfection mixture (for example, it may be about 10% or less, about 9% or less, about 8% or less, about 7% or less, about 6% or less, about 5% or less, about 4% or less, about 3% or less, about 2.5% or less, about 2% or less, about 1.5% or less, about 1% or less, about 0.9‰ or less, about 0.8‰ or less, about 0.7‰ or less, about 0.6‰ or less, about 0.55‰ or less, about 0.5‰ or less, about 0.45‰ or less, about 0.4‰ or less, about 0.35‰ or less, about 0.3‰ or less, about 0.25‰ or less, about 0.2‰ or less, about 0.15‰ or less, about 0.1‰ or less, about 0.05‰ or less, about 0.01‰ or less, about 0.001‰ or less or lower). The percentage may be a weight percentage (w/w%).

In another aspect, the present application provides a method for increasing DNA homologous recombination efficiency (e.g., an *in vitro* method), comprising the following steps: making the content of nucleic acid molecules or fragments thereof originating from a host (e.g., a microorganism) genome account for about 10% or less of the total nucleic acid molecules content in the transfection mixture (for example, it may be about 10% or less, about 9% or less, about 8% or less, about 7% or less, about 6% or less, about 5% or less, about 4% or less, about 3% or less, about 2.5% or less, about 2% or less, about 1.5% or less, about 1% or less, about 0.9‰ or less, about 0.8‰ or less, about 0.7‰ or less, about 0.6‰ or less, about 0.55‰ or less, about 0.5‰ or less, about 0.45‰ or less, about 0.4‰ or less, about 0.35‰ or less, about 0.3‰ or less, about 0.25‰ or less, about 0.2‰ or less, about 0.15‰ or less, about 0.1‰ or less, about 0.05‰ or less, about 0.01‰ or less, about 0.001‰ or less or lower). The percentage may be a weight percentage (w/w%).

In another aspect, the present application provides a method for improving the cell viability after transfection (e.g., a method for avoiding a decrease in cell viability after transfection), comprising the following steps: making the content of nucleic acid molecules or fragments thereof derived from the host genome (e.g., microorganisms) account for less than about 10% of the total nucleic acid content of the transfection mixture (e.g., may be about 10% or less, about 9% or less, about 8% or less, about 7% or less, about 6% or less, about 5% or less, about 4% or less, about 3% or less, about 2.5% or less, about 2% or less, about 1.5% or less, about 1% or less, about 9‰ or less, about 8‰ or less, about 7‰ or less, about 6‰ or less, about 5.5‰ or less, about 5‰ or less, about 4.5‰ or less, about 4‰ or less, about 3.5‰ or less, about 3‰ or less, about 2.5‰ or less, about 2‰ or less, about 1.5‰ or less, about 1‰ or less, about 0.5‰ or less, about 0.1‰ or less, about 0.01‰ or less, about 0.001‰ or less, or lower). The percentage may be a weight percentage w/w%.

In another aspect, the present application provides a method for preparing a transfection mixture, comprising the following steps: making the content of nucleic acid molecules or fragments thereof derived from the host genome (e.g., microorganisms) account for less than about 10% of the total nucleic acid content of the transfection mixture (e.g., may be about 10% or less, about 9% or less, about 8% or less, about 7% or less, about 6% or less, about 5% or less, about 4% or less, about 3% or less, about 2.5% or less, about 2% or less, about 1.5% or less, about 1% or less, about 9‰ or less, about 8‰ or less, about 7‰ or less, about 6‰ or less, about 5.5‰ or less, about 5‰ or less, about 4.5‰ or less, about 4‰ or less, about 3.5‰ or less, about 3‰ or less, about 2.5‰ or less, about 2‰ or less, about 1.5‰ or less, about 1‰ or less, about 0.5‰ or less, about 0.1‰ or less, about 0.01‰ or less, about 0.001‰ or less, or lower). The percentage may be a weight percentage w/w%.

In another aspect, the present application provides a method for determining the quality of a transfection mixture, comprising the following steps: determining whether the content of nucleic acid molecules or fragments thereof derived from the host genome (e.g., microorganisms) accounts for less than about 10% of the total nucleic acid content of the transfection mixture (e.g., may be about 10% or less, about 9% or less, about 8% or less, about 7% or less, about 6% or less, about 5% or less, about 4% or less, about 3% or less, about 2.5% or less, about 2% or less, about 1.5% or less, about 1% or less, about 9‰ or less, about 8‰ or less, about 7‰ or less, about 6‰ or less, about 5.5‰ or less, about 5‰ or less, about 4.5‰ or less, about 4‰ or less, about 3.5‰ or less, about 3‰ or less, about 2.5‰ or less, about 2‰ or less, about 1.5‰ or less, about 1‰ or less, about 0.5‰ or less, about 0.1‰ or less, about 0.01‰ or less, about 0.001‰ or less, or lower). The percentage may be a weight percentage w/w%.

In one aspect, the present application provides a method for modifying cells. The method may be an *in vitro* method or an ex *vivo* method. The method may comprise: transfecting the cell to be modified with a transfection composition to cause the cell to comprise and/or express an exogenous gene, wherein the transfection composition contains an exogenous nucleic acid molecule containing the exogenous gene, and at least a portion (e.g., at least 1w/w%, at least 5w/w%, at least 10w/w%, at least 15w/w%, at least 20w/w%, at least 25w/w%, at least 30w/w%, at least 35w/w%, at least 40w/w%, at least 45w/w%, at least 50w/w%, at least 55w/w%, at least 60w/w%, at least 65w/w%, at least 70w/w%, at least 75w/w%, at least 80w/w%, at least 85w/w%, at least 90w/w%, at least 95w/w%, at least 99w/w%, at least 100w/w%) of the exogenous nucleic acid molecules are derived from host cells, and the genomic DNA of the host cells accounts for about 10% (w/w) or less (e.g., about 9% (w/w) or less, about 8% (w/w) or less, about 7% (w/w) or less, about 6% (w/w) or less, about 5% (w/w) or less, about 4% (w/w) or less, about 3% (w/w) or less, about 2% (w/w) or less, about 1.5% (w/w) or less, about 1% (w/w) or less, about 9‰ (w/w) or less, about 8‰ (w/w) or less, about 7‰ (w/w) or less, about 6‰ (w/w) or less, about 5‰ (w/w) or less, about 4‰ (w/w) or less, about 3‰ (w/w) or less, about 2‰ (w/w) or less, about 1.5‰ (w/w) or less, about 1‰ (w/w) or less, about 0.5‰ (w/w) or less, about 0. 1‰ (w/w) or less, about 0.01‰ (w/w) or less, about 0.001‰ (w/w) or less, or lower) of the amount of the exogenous nucleic acid molecules derived from the host cells.

In another aspect, the present application provides a method for improving the cell transfection efficiency of exogenous nucleic acid molecules derived from host cells, the method comprising: reducing the content of the host cell genomic DNA in the portion of the exogenous nucleic acid molecules derived from the host cells.

In some embodiments, the composition used for transfection comprises exogenous nucleic acid molecules to be transfected into the cell, and at least a portion (e.g., at least 1w/w%, at least 5w/w%, at least 10w/w%, at least 15w/w%, at least 20w/w%, at least 25w/w%, at least 30w/w%, at least 35w/w%, at least 40w/w%, at least 45w/w%, at least 50w/w%, at least 55w/w%, at least 60w/w%, at least 65w/w%, at least 70w/w%, at least 75w/w%, at least 80w/w%, at least 85w/w%, at least 90w/w%, at least 95w/w%, at least 99w/w%, at least 100w/w%) of the exogenous nucleic acid molecules are the exogenous nucleic acid molecules derived from the host cells (e.g., obtained by extraction from the host cells).

In some embodiments, after the reduction, the content of the host cell genomic DNA is about 10% (w/w) or less (e.g., about 9% (w/w) or less, about 8% (w/w) or less, about 7% (w/w) or less, about 6% (w/w) or less, about 5% (w/w) or less, about 4% (w/w) or less, about 3% (w/w) or less, about 2% (w/w) or less, about 1.5% (w/w) or less, about 1% (w/w) or less, about 9‰ (w/w) or less, about 8‰ (w/w) or less, about 7‰ (w/w) or less, about 6‰ (w/w) or less, about 5‰ (w/w) or less, about 4‰ (w/w) or less, about 3‰ (w/w) or less, about 2‰ (w/w) or less, about 1.5‰ (w/w) or less, about 1‰ (w/w) or less, about 0.5‰ (w/w) or less, about 0.1‰ (w/w) or less, about 0.01‰ (w/w) or less, about 0.001‰ (w/w) or less, or lower).

In another aspect, he present application provides a method for improving the cell transfection efficiency of exogenous nucleic acid molecules obtained from host cells, comprising: treating the exogenous nucleic acid molecules with DNase. In some embodiments, the DNase can non-specifically cleave linear DNA. In some embodiments, the DNase can comprise a nucleic acid exonuclease. In some embodiments, the treatment can comprise contacting the exogenous nucleic acid molecules with the DNase in the presence of Mg²⁺ and Ca²⁺.

In another aspect, the present application provides a method for optimizing a transfection composition, which comprises exogenous nucleic acid molecules obtained from host cells, the method comprising: reducing the content of host cell genomic DNA in the exogenous nucleic acid molecules obtained from the host cells.

In some embodiments, after the optimization, the content of genomic DNA of the host cell is about 10% (w/w) or less (for example, about 10% or less, about 9% or less, about 8% or less, about 7% or less, about 6% or less, about 5% or less, about 4% or less, about 3% or less, about 2.5% or less, about 2% or less, about 1.5% or less, about 1% or less, about 0.9‰ or less, about 0.8‰ or less, about 0.7‰ or less, about 0.6‰ or less, about 0.55‰ or less, about 0.5‰ or less, about 0.45‰ or less, about 0.4‰ or less, about 0.35‰ or less, about 0.3‰ or less, about 0.25‰ or less, about 0.2‰ or less, about 0.15‰ or less, about 0.1‰ or less, about 0.05‰ or less, about 0.01‰ or less, about 0.001‰ or less or lower; all are mass percentages).

In another aspect, the present application provides a method for optimizing a transfection composition, which comprises a portion of exogenous nucleic acid molecule derived from a host cell, the method comprising: treating the portion of exogenous nucleic acid molecule derived from the host cell with DNase. In some embodiments, the DNase can non-specifically cleave linear DNA. In some embodiments, the DNase comprises a nucleic acid exonuclease. In some embodiments, the treatment comprises contacting the portion of exogenous nucleic acid molecule derived from the host cell with the DNase in the presence of Mg²⁺ and Ca²⁺.

In the present application, compared with exogenous nucleic acid molecules not treated with deoxyribonuclease (DNase), the transfection efficiency of exogenous nucleic acid molecules treated with deoxyribonuclease (DNase) may be increased by at least about 10%, at least about 20%, at least about 30%, at least about 40%, at least about 50%, at least about 60%, at least about 70%, at least about 80%, at least about 90%, at least about 100%, at least about 110%, at least about 120%, at least about 130%, at least about 140%, at least about 150%, at least about 200%, at least about 300%, at least about 400%, at least about 500%, at least about 1000% or more.

The methods of the present application may be *in vitro* or ex *vivo* methods.

In the present application, the transfection efficiency of the transfection composition (exogenous nucleic acid molecules or plasmids) to cells may be determined by the following methods: 1) measuring the proportion of cells expressing exogenous genes in the transfected cells, wherein the transfection composition (or plasmid) contains exogenous nucleic acid molecules comprising the exogenous gene; and/or 2) measuring the proportion of cells containing exogenous genes in the transfected cells, wherein the transfection composition contains exogenous nucleic acid molecules comprising the exogenous gene.

In another aspect, the present application provides the use of the aforementioned reagents (e.g., deoxyribonuclease (DNase), SDS, TX-100, CTAB, and/or cesium chloride-ethidium bromide) for optimizing transfection compositions.

In another aspect, the present application provides a transfection (e.g., electroporation) reagent kit. The reagent kit comprises: 1) the portion of exogenous nucleic acid molecule derived from the host cell as described in any aspect of the present application; and 2) a reagent capable of reducing or degrading the genomic DNA of the host cell. In some embodiments, the reagent of 2) comprises one or more of the following: deoxyribonuclease (DNase), SDS, TX-100, CTAB, and cesium chloride-ethidium bromide. In some embodiments, the DNase can non-specifically cleave linear DNA. In some embodiments, the DNase comprises a nucleic acid exonuclease. In some embodiments, the reagent kit also comprises a reagent containing Mg²⁺ and Ca²⁺.

In another aspect, the present application provides a reagent kit for improving plasmid transfection efficiency, comprising deoxyribonuclease (DNase) and reagents and/or instruments required for transient transfection. In the present application, the reagents required for transient transfection may comprise reagents required for electroporation. For example, the reagents required for electroporation may comprise ddH₂O and/or glycerol. For example, the instruments required for electroporation may comprise electroporator, electroporation cuvette, and/or centrifuge.

In the present application, the reagent kit may also comprise reagents and/or instruments required for obtaining the transformed state of cells required for the electroporation. For example, the transformed state of cells may be stored at ultra-low temperature (e.g., -70°C). In the present application, during the electroporation, the voltage of the electroporator may be about 1500-2500V. In the present application, the electroporation cuvette may be suitable for ultra-low temperature conditions.

In the present application, the DNase may cleave single-stranded DNA and/or double-stranded DNA. For example, the DNase may non-specifically cleave linear DNA. For example, the DNase may comprise a nucleic acid exonuclease. For example, the DNase may not have any RNase. In some embodiments, the DNase may not cleave circular DNA. In some embodiments, the DNase may not cleave single-stranded DNA.

In the present application, the reagent kit may also comprise a buffer solution, which may comprise Mg²⁺ and Ca²⁺. For example, the buffer solution may be the Reaction Buffer corresponding to the DNase. In the present application, the reagent kit may also comprise deionized water (e.g., DEPC-treated) required for DNase digestion of DNA.

In another aspect, the present application provides a reagent kit, comprising: 1) the portion of exogenous nucleic acid molecule derived from host cells as described in any aspect of the present application; and 2) an instructions, which describe the processing of the portion of exogenous nucleic acid molecule derived from host cells by the method of the present application and/or determining the quality of the transfection compositions containing the portion of exogenous nucleic acid molecule derived from host cells by the method of the present application.

In another aspect, the present application provides a transfection composition containing exogenous nucleic acid molecules treated by the method of the present application.

In another aspect, the present application provides cells transfected with the transfection composition of the present application.

In another aspect, the present application provides cells modified by the method of the present application.

In another aspect, the present application provides a cell population comprising the cells of the present application and/or their progeny. For example, the cell population may comprise at least 10³ cells (e.g., at least 10⁴ cells, at least 10⁵ cells, at least 10⁶ cells, at least 10⁷ cells, at least 10⁸ cells, at least 10⁹ cells, at least 10¹⁰ cells, or more).

In another aspect, the present application provides a pharmaceutical composition comprising the transfection composition of the present application, the cells of the present application, and/or the cell population of the present application. In some embodiments, the pharmaceutical composition may also comprise pharmaceutically acceptable adjuvants. The pharmaceutically acceptable adjuvants may comprise, for example, materials that do not cause significant irritation to the organism and do not significantly negatively affect the biological activity and properties of the active ingredient(s) (e.g., the modified cells or cell population). For example, the pharmaceutically acceptable adjuvants may comprise but are not limited to: diluents, buffers, binders, surfactants, wetting agents, adsorbents, lubricants, fillers, and/or disintegrants.

In another aspect, the present application provides the use of the transfection composition of the present application, the cells of the present application, the cell population of the present application, and/or the pharmaceutical composition of the present application for the preparation of a drug. In some embodiments, the drug may be used for the prevention, treatment, and/or alleviation of cancer. In some embodiments, the drug may be used for regulating immune responses.

In another aspect, the present application provides a method for preventing, treating, and/or alleviating a disease or disorder in a subject, comprising administering an effective amount of the transfection composition of the present application, the cells of the present application, the cell population of the present application, and/or the pharmaceutical composition of the present application to the subject. In some embodiments, the disease or disorder may be cancer. In some embodiments, the disease or disorder may be related to the immune response of the subject.

In another aspect, the present application provides an electroporation method (e.g., plasmid electroporation method), the method comprising: electroporating cells with a transfection composition comprising a plasmid encoding one or more exogenous genes, so as to enable the cell to comprise and/or express the one or more exogenous genes, wherein the plasmid is extracted from a host cell, and the content of the host cell genomic DNA comprised in the transfection composition accounts for about 10% (w/w) or less of the plasmid DNA content in the transfection composition (e.g., about 9% (w/w) or less, about 8% (w/w) or less, about 7% (w/w) or less, about 6% (w/w) or less, about 5% (w/w) or less, about 4% (w/w) or less, about 3% (w/w) or less, about 2% (w/w) or less, about 1.5% (w/w) or less, about 1% (w/w) or less, about 9‰ (w/w) or less, about 8‰ (w/w) or less, about 7‰ (w/w) or less, about 6‰ (w/w) or less, about 5‰ (w/w) or less, about 4‰ (w/w) or less, about 3‰ (w/w) or less, about 2‰ (w/w) or less, about 1.5‰ (w/w) or less, about 1‰ (w/w) or less, about 0.5‰ (w/w) or less, about 0.1‰ (w/w) or less, about 0.01‰ (w/w) or less, about 0.001‰ (w/w) or less, or lower).

In another aspect, the present application provides an electroporation method, the method comprising: electroporating cells with a transfection composition comprising a plasmid encoding an exogenous gene, so as to enable the cell to comprise and/or express the exogenous gene, wherein the size of the exogenous gene is at least about 3kb (e.g., at least about 3.5kb, at least about 4kb, at least about 4.5kb, at least about 5kb, at least about 5.5kb, at least about 6kb, at least about 6.5kb, at least about 7kb, at least about 7.5kb, at least about 8kb, at least about 8.5kb, at least about 9kb or larger), wherein the plasmid is extracted from a host cell, and the content of the host cell genomic DNA comprised in the transfection composition accounts for about 10% (w/w) or less of the plasmid DNA content in the transfection composition (e.g., about 9% (w/w) or less, about 8% (w/w) or less, about 7% (w/w) or less, about 6% (w/w) or less, about 5% (w/w) or less, about 4% (w/w) or less, about 3% (w/w) or less, about 2% (w/w) or less, about 1.5% (w/w) or less, about 1% (w/w) or less, about 9‰ (w/w) or less, about 8‰ (w/w) or less, about 7‰ (w/w) or less, about 6‰ (w/w) or less, about 5‰ (w/w) or less, about 4‰ (w/w) or less, about 3‰ (w/w) or less, about 2‰ (w/w) or less, about 1.5‰ (w/w) or less, about 1‰ (w/w) or less, about 0.5‰ (w/w) or less, about 0.1‰ (w/w) or less, about 0.01‰ (w/w) or less, about 0.001‰ (w/w) or less, or lower).

In another aspect, the present application provides an electroporation method (e.g., plasmid electroporation method), the method comprising: electroporating cells (e.g., immune cells, such as T cells) encoding a chimeric antigen receptor (CAR) with a transfection composition comprising a plasmid, so as to enable the cell (e.g., immune cells, such as T cells) to comprise and/or express the CAR, wherein the plasmid is extracted from a host cell, and the content of the host cell genomic DNA comprised in the transfection composition accounts for about 10% (w/w) or less of the plasmid DNA content in the transfection composition (e.g., about 9% (w/w) or less, about 8% (w/w) or less, about 7% (w/w) or less, about 6% (w/w) or less, about 5% (w/w) or less, about 4% (w/w) or less, about 3% (w/w) or less, about 2% (w/w) or less, about 1.5% (w/w) or less, about 1% (w/w) or less, about 9‰ (w/w) or less, about 8‰ (w/w) or less, about 7‰ (w/w) or less, about 6‰ (w/w) or less, about 5‰ (w/w) or less, about 4‰ (w/w) or less, about 3‰ (w/w) or less, about 2‰ (w/w) or less, about 1.5‰ (w/w) or less, about 1‰ (w/w) or less, about 0.5‰ (w/w) or less, about 0.1‰ (w/w) or less, about 0.01‰ (w/w) or less, about 0.001‰ (w/w) or less, or lower).

In another aspect, the present application provides an electroporation method (e.g., plasmid electroporation method), the method comprising: electroporating a cell (e.g., immune cell, such as T cell) with a transfection composition comprising plasmid encoding chimeric antigen receptor (CAR) and one or more second proteins (e.g., cytokines, antibodies, and/or chemokines), wherein the cell (e.g., immune cell, such as T cell) comprises and/or expresses the CAR and said one or more second proteins, the plasmid is extracted from a host cell, and the content of the host cell genomic DNA in the transfection composition is about 10% (w/w) or less of the plasmid DNA content in the transfection composition (e.g., about 9% (w/w) or less, about 8% (w/w) or less, about 7% (w/w) or less, about 6% (w/w) or less, about 5% (w/w) or less, about 4% (w/w) or less, about 3% (w/w) or less, about 2% (w/w) or less, about 1.5% (w/w) or less, about 1% (w/w) or less, about 9‰ (w/w) or less, about 8‰ (w/w) or less, about 7‰ (w/w) or less, about 6‰ (w/w) or less, about 5‰ (w/w) or less, about 4‰ (w/w) or less, about 3‰ (w/w) or less, about 2‰ (w/w) or less, about 1.5‰ (w/w) or less, about 1‰ (w/w) or less, about 0.5‰ (w/w) or less, about 0. 1‰ (w/w) or less, about 0.01‰ (w/w) or less, about 0.001‰ (w/w) or less or lower).

In another aspect, the present application provides an electroporation method (e.g., plasmid electroporation method), the method comprising: electroporating a cell (e.g., immune cell, such as T cell) with a transfection composition comprising plasmid encoding chimeric antigen receptor (CAR) and multispecific antibody or its antigen-binding fragments (e.g., BiTE), wherein the cell (e.g., immune cell, such as T cell) comprises and/or expresses said CAR and said multispecific antibody or its antigen-binding fragments, the plasmid is extracted from a host cell, and the content of the host cell genomic DNA in the transfection composition is about 10% (w/w) or less of the plasmid DNA content in the transfection composition (e.g., about 9% (w/w) or less, about 8% (w/w) or less, about 7% (w/w) or less, about 6% (w/w) or less, about 5% (w/w) or less, about 4% (w/w) or less, about 3% (w/w) or less, about 2% (w/w) or less, about 1.5% (w/w) or less, about 1% (w/w) or less, about 9‰ (w/w) or less, about 8‰ (w/w) or less, about 7‰ (w/w) or less, about 6‰ (w/w) or less, about 5‰ (w/w) or less, about 4‰ (w/w) or less, about 3‰ (w/w) or less, about 2‰ (w/w) or less, about 1.5‰ (w/w) or less, about 1‰ (w/w) or less, about 0.5‰ (w/w) or less, about 0. 1‰ (w/w) or less, about 0.01‰ (w/w) or less, about 0.001‰ (w/w) or less or lower).

In another aspect, the present application provides an electroporation method (e.g., plasmid electroporation method), the method comprising: electroporating a cell (e.g., immune cell, such as T cell) with a transfection composition comprising plasmid encoding multispecific antibody or its antigen-binding fragments (e.g., BiTE), wherein the cell (e.g., immune cell, such as T cell) comprises and/or expresses said multispecific antibody or its antigen-binding fragments, the plasmid is extracted from a host cell, and the content of the host cell genomic DNA in the transfection composition is about 10% (w/w) or less of the plasmid DNA content in the transfection composition (e.g., about 9% (w/w) or less, about 8% (w/w) or less, about 7% (w/w) or less, about 6% (w/w) or less, about 5% (w/w) or less, about 4% (w/w) or less, about 3% (w/w) or less, about 2% (w/w) or less, about 1.5% (w/w) or less, about 1% (w/w) or less, about 9‰ (w/w) or less, about 8‰ (w/w) or less, about 7‰ (w/w) or less, about 6‰ (w/w) or less, about 5‰ (w/w) or less, about 4‰ (w/w) or less, about 3‰ (w/w) or less, about 2‰ (w/w) or less, about 1.5‰ (w/w) or less, about 1‰ (w/w) or less, about 0.5‰ (w/w) or less, about 0.1‰ (w/w) or less, about 0.01‰ (w/w) or less, about 0.001‰ (w/w) or less or lower).

In another aspect, the present application provides an electroporation method (e.g., plasmid electroporation method), the method comprising: electroporating cells (e.g., immune cells, such as T cells) with a transfection composition comprising plasmids encoding a chimeric antigen receptor (CAR), one or more cytokines and/or chemokines, and multispecific antibodies or their antigen-binding fragments (e.g., BiTE), wherein the plasmids are extracted from host cells, and the content of genomic DNA of the host cells in the transfection composition is about 10% (w/w) or less of the plasmid DNA content in the transfection composition (e.g., about 9% (w/w) or less, about 8% (w/w) or less, about 7% (w/w) or less, about 6% (w/w) or less, about 5% (w/w) or less, about 4% (w/w) or less, about 3% (w/w) or less, about 2% (w/w) or less, about 1.5% (w/w) or less, about 1% (w/w) or less, about 9‰ (w/w) or less, about 8‰ (w/w) or less, about 7‰ (w/w) or less, about 6‰ (w/w) or less, about 5‰ (w/w) or less, about 4‰ (w/w) or less, about 3‰ (w/w) or less, about 2‰ (w/w) or less, about 1.5‰ (w/w) or less, about 1‰ (w/w) or less, about 0.5‰ (w/w) or less, about 0.1‰ (w/w) or less, about 0.01‰ (w/w) or less, about 0.001‰ (w/w) or less or lower).

In another aspect, the present application provides an electroporation method (e.g., plasmid electroporation method), the method comprising: electroporating cells (e.g., immune cells, such as T cells) with a transfection composition comprising plasmids encoding one or more cytokines and/or chemokines, and multispecific antibodies or their antigen-binding fragments (e.g., BiTE), wherein the plasmids are extracted from host cells, and the content of genomic DNA of the host cells in the transfection composition is about 10% (w/w) or less of the plasmid DNA content in the transfection composition (e.g., about 9% (w/w) or less, about 8% (w/w) or less, about 7% (w/w) or less, about 6% (w/w) or less, about 5% (w/w) or less, about 4% (w/w) or less, about 3% (w/w) or less, about 2% (w/w) or less, about 1.5% (w/w) or less, about 1% (w/w) or less, about 9‰ (w/w) or less, about 8‰ (w/w) or less, about 7‰ (w/w) or less, about 6‰ (w/w) or less, about 5‰ (w/w) or less, about 4‰ (w/w) or less, about 3‰ (w/w) or less, about 2‰ (w/w) or less, about 1.5‰ (w/w) or less, about 1‰ (w/w) or less, about 0.5‰ (w/w) or less, about 0.1‰ (w/w) or less, about 0.01‰ (w/w) or less, about 0.001‰ (w/w) or less or lower).

In another aspect, the present application provides a one-step electroporation method, the method comprising: electroporating cells with a transfection composition comprising a plasmid encoding an exogenous gene, so that the cell comprises and/or expresses the exogenous gene (for example, the exogenous gene is knocked into the cell's genome) and one or more endogenous genes in the cell's genome are knocked out, wherein the size of the exogenous gene is at least about 3kb (for example, at least about 3.5kb, at least about 4kb, at least about 4.5kb, at least about 5kb, at least about 5.5kb, at least about 6kb, at least about 6.5kb, at least about 7kb, at least about 7.5kb, at least about 8kb, at least about 8.5kb, at least about 9kb or larger). In some embodiments, the plasmid is extracted from a host cell. In some embodiments, the content of the host cell's genomic DNA contained in the transfection composition accounts for about 10% (w/w) or less of the plasmid DNA content in the transfection composition (for example, about 9% (w/w) or less, about 8% (w/w) or less, about 7% (w/w) or less, about 6% (w/w) or less, about 5% (w/w) or less, about 4% (w/w) or less, about 3% (w/w) or less, about 2% (w/w) or less, about 1.5% (w/w) or less, about 1% (w/w) or less, about 9‰ (w/w) or less, about 8‰ (w/w) or less, about 7‰ (w/w) or less, about 6‰ (w/w) or less, about 5‰ (w/w) or less, about 4‰ (w/w) or less, about 3‰ (w/w) or less, about 2‰ (w/w) or less, about 1.5‰ (w/w) or less, about 1‰ (w/w) or less, about 0.5‰ (w/w) or less, about 0.1‰ (w/w) or less, about 0.01‰ (w/w) or less, about 0.001‰ (w/w) or less, or lower).

In another aspect, the present application provides a one-step electroporation method, the method comprising: electroporating cells with a transfection composition comprising a plasmid encoding an exogenous gene, so that the cell comprises and/or expresses the exogenous gene (for example, the exogenous gene is knocked into the cell's genome) and one or more endogenous genes in the cell's genome are knocked out, wherein the exogenous gene encodes CAR. In some embodiments, the plasmid is extracted from a host cell. In some embodiments, the content of the host cell's genomic DNA contained in the transfection composition accounts for about 10% (w/w) or less of the plasmid DNA content in the transfection composition (for example, about 9% (w/w) or less, about 8% (w/w) or less, about 7% (w/w) or less, about 6% (w/w) or less, about 5% (w/w) or less, about 4% (w/w) or less, about 3% (w/w) or less, about 2% (w/w) or less, about 1.5% (w/w) or less, about 1% (w/w) or less, about 9‰ (w/w) or less, about 8‰ (w/w) or less, about 7‰ (w/w) or less, about 6‰ (w/w) or less, about 5‰ (w/w) or less, about 4‰ (w/w) or less, about 3‰ (w/w) or less, about 2‰ (w/w) or less, about 1.5‰ (w/w) or less, about 1‰ (w/w) or less, about 0.5‰ (w/w) or less, about 0.1‰ (w/w) or less, about 0.01‰ (w/w) or less, about 0.001‰ (w/w) or less, or lower).

In another aspect, the present application provides a one-step electroporation method, the method comprising: electroporating cells with a transfection composition comprising a plasmid encoding an exogenous gene, so that the cell comprises and/or expressesthe exogenous gene (for example, the exogenous gene is knocked into the cell's genome) and one or more endogenous genes in the cell's genome are knocked out, wherein the exogenous gene encodes a transcription factor. In some embodiments, the plasmid is extracted from a host cell. In some embodiments, the content of the host cell's genomic DNA contained in the transfection composition accounts for about 10% (w/w) or less of the plasmid DNA content in the transfection composition (for example, about 9% (w/w) or less, about 8% (w/w) or less, about 7% (w/w) or less, about 6% (w/w) or less, about 5% (w/w) or less, about 4% (w/w) or less, about 3% (w/w) or less, about 2% (w/w) or less, about 1.5% (w/w) or less, about 1% (w/w) or less, about 9‰ (w/w) or less, about 8‰ (w/w) or less, about 7‰ (w/w) or less, about 6‰ (w/w) or less, about 5‰ (w/w) or less, about 4‰ (w/w) or less, about 3‰ (w/w) or less, about 2‰ (w/w) or less, about 1.5‰ (w/w) or less, about 1‰ (w/w) or less, about 0.5‰ (w/w) or less, about 0.1‰ (w/w) or less, about 0.01‰ (w/w) or less, about 0.001‰ (w/w) or less, or lower).

In another aspect, the present application provides a one-step electroporation method, the method comprising: electroporating a cell with a transfection composition containing a plasmid encoding an exogenous gene, so that the cell comprises and/or expresses the exogenous gene (for example, the exogenous gene is knocked into the genome of the cell) and one or more endogenous genes in the cell genome are knocked out, the exogenous gene encodes a CAR and one or more cytokines and/or chemokines. In some embodiments, the plasmid is extracted from a host cell. In some embodiments, the content of the host cell genomic DNA contained in the transfection composition accounts for about 10% (w/w) or less of the plasmid DNA content in the transfection composition (for example, about 9% (w/w) or less, about 8% (w/w) or less, about 7% (w/w) or less, about 6% (w/w) or less, about 5% (w/w) or less, about 4% (w/w) or less, about 3% (w/w) or less, about 2% (w/w) or less, about 1.5% (w/w) or less, about 1% (w/w) or less, about 9‰(w/w) or less, about 8‰(w/w) or less, about 7‰(w/w) or less, about 6‰(w/w) or less, about 5‰(w/w) or less, about 4‰(w/w) or less, about 3‰(w/w) or less, about 2‰(w/w) or less, about 1.5‰(w/w) or less, about 1‰(w/w) or less, about 0.5‰(w/w) or less, about 0.1‰(w/w) or less, about 0.01‰(w/w) or less, about 0.001‰(w/w) or less or lower).

In another aspect, the present application provides a one-step electroporation method, the method comprising: electroporating a cell with a transfection composition containing a plasmid encoding an exogenous gene, so that the cell comprises and/or expresses the exogenous gene (for example, the exogenous gene is knocked into the genome of the cell) and one or more endogenous genes in the cell genome are knocked out, the exogenous gene encodes a CAR and multispecific antibodies or their antigen-binding fragments (for example, BiTE). In some embodiments, the plasmid is extracted from a host cell. In some embodiments, the content of the host cell genomic DNA contained in the transfection composition accounts for about 10% (w/w) or less of the plasmid DNA content in the transfection composition (for example, about 9% (w/w) or less, about 8% (w/w) or less, about 7% (w/w) or less, about 6% (w/w) or less, about 5% (w/w) or less, about 4% (w/w) or less, about 3% (w/w) or less, about 2% (w/w) or less, about 1.5% (w/w) or less, about 1% (w/w) or less, about 9‰(w/w) or less, about 8‰(w/w) or less, about 7‰(w/w) or less, about 6‰(w/w) or less, about 5‰(w/w) or less, about 4‰(w/w) or less, about 3‰(w/w) or less, about 2‰(w/w) or less, about 1.5‰(w/w) or less, about 1‰(w/w) or less, about 0.5‰(w/w) or less, about 0.1‰(w/w) or less, about 0.01‰(w/w) or less, about 0.001‰(w/w) or less or lower).

In another aspect, the present application provides a one-step electroporation method, the method comprising: electroporating a cell with a transfection composition containing a plasmid encoding an exogenous gene, so that the cell comprises and/or expresses the exogenous gene (for example, the exogenous gene is knocked into the genome of the cell) and one or more endogenous genes in the cell genome are knocked out, the exogenous gene encodes a CAR, one or more cytokines and/or chemokines, and multispecific antibodies or their antigen-binding fragments (for example, BiTE). In some embodiments, the plasmid is extracted from a host cell. In some embodiments, the content of the host cell genomic DNA contained in the transfection composition accounts for about 10% (w/w) or less of the plasmid DNA content in the transfection composition (for example, about 9% (w/w) or less, about 8% (w/w) or less, about 7% (w/w) or less, about 6% (w/w) or less, about 5% (w/w) or less, about 4% (w/w) or less, about 3% (w/w) or less, about 2% (w/w) or less, about 1.5% (w/w) or less, about 1% (w/w) or less, about 9‰(w/w) or less, about 8‰(w/w) or less, about 7‰(w/w) or less, about 6‰(w/w) or less, about 5‰(w/w) or less, about 4‰(w/w) or less, about 3‰(w/w) or less, about 2‰(w/w) or less, about 1.5‰(w/w) or less, about 1‰(w/w) or less, about 0.5‰(w/w) or less, about 0.1‰(w/w) or less, about 0.01‰(w/w) or less, about 0.001‰(w/w) or less or lower).

In another aspect, the present application provides a one-step electroporation method, the method comprising: electroporating cells with a transfection composition comprising a plasmid encoding an exogenous gene, so that the cell comprises and/or expresses the exogenous gene (for example, the exogenous gene is knocked into the genome of the cell) and one or more endogenous genes in the cell genome are knocked out, the exogenous gene encodes one or more cytokines and/or chemokines, as well as multispecific antibodies or their antigen-binding fragments (for example, BiTE). In some embodiments, the plasmid is extracted from a host cell. In some embodiments, the content of the host cell genomic DNA in the transfection composition comprises approximately 10% (w/w) or less of the plasmid DNA content in the transfection composition (for example, approximately 9% (w/w) or less, approximately 8% (w/w) or less, approximately 7% (w/w) or less, approximately 6% (w/w) or less, approximately 5% (w/w) or less, approximately 4% (w/w) or less, approximately 3% (w/w) or less, approximately 2% (w/w) or less, approximately 1.5% (w/w) or less, approximately 1% (w/w) or less, approximately 9‰ (w/w) or less, approximately 8‰ (w/w) or less, approximately 7‰ (w/w) or less, approximately 6‰ (w/w) or less, approximately 5‰ (w/w) or less, approximately 4‰ (w/w) or less, approximately 3‰ (w/w) or less, approximately 2‰ (w/w) or less, approximately 1.5‰ (w/w) or less, approximately 1‰ (w/w) or less, approximately 0.5‰ (w/w) or less, approximately 0.1‰ (w/w) or less, approximately 0.01‰ (w/w) or less, approximately 0.001‰ (w/w) or less, or lower).

In some embodiments, the electroporation method of the present application is a one-step electroporation method, of which the knock-in and/or knockout of the exogenous gene is completed in a single electroporation step.

### Host genomic DNA

The host cell of the present application may be a prokaryotic cell or a eukaryotic cell, which comprises any transformable organism capable of replicating or expressing a heterologous gene encoded by a vector or expression vector. The host cell may and have been used as a recipient of exogenous nucleic acid molecules.

For example, the host may be a microbial host. For example, the host may be selected from one or more of the following group: bacteria, fungi, actinomycetes, mycoplasma, chlamydia, rickettsia, and spirochetes. For example, the host may comprise gram-negative bacteria. For example, the host may comprise Escherichia coli (for example, competent E. coli cells).

In the present application, the genomic DNA of the host cell may not be comprised in the exogenous nucleic acid molecule.

In the present application, the content of the host cell genomic DNA may be determined by qPCR method.

In the present application, the size of the genomic DNA of the host cell (for example, the nucleic acid molecule or fragments thereof of the present application) may be at least about 50kb, at least about 100kb, at least about 150kb, at least about 200kb, at least about 250kb, at least about 300kb, at least about 350kb, at least about 400kb, at least about 450kb, at least about 500kb, at least about 600kb, at least about 700kb, at least about 800kb, at least about 900kb, at least about 1Mb, at least about 2Mb, at least about 3Mb, at least about 4Mb, at least about 5Mb, at least about 6Mb, at least about 7Mb, at least about 8Mb, at least about 9Mb, at least about 10Mb, at least about 20Mb, at least about 50Mb, at least about 100Mb, at least about 200Mb or larger. For example, the size of the nucleic acid molecule or fragments thereof (for example, the genomic DNA of the host cell of the present application) may be at least about 10kb. In the present application, the size of the fragments of nucleic acid molecules derived from the host (for example, microbial) genome (for example, the genomic DNA of the host cell of the present application) may have a size of at least about 10kb (for example, may be at least about 15kb, at least about 20kb, at least about 30kb, at least about 35kb, at least about 40kb, at least about 48kb, at least about 50kb, at least about 100kb, at least about 150kb, at least about 200kb, at least about 250kb, at least about 300kb, at least about 350kb, at least about 400kb, at least about 450kb, at least about 500kb, at least about 600kb, at least about 700kb, at least about 800kb, at least about 900kb, at least about 1Mb, at least about 2Mb, at least about 3Mb, at least about 4Mb, at least about 5Mb, at least about 6Mb, at least about 7Mb, at least about 8Mb, at least about 9Mb, at least about 10Mb, at least about 20Mb, at least about 50Mb, at least about 100Mb, at least about 200Mb or larger).

In the present application, the nucleic acid molecule or fragments thereof may be derived from a microorganism (for example, the genomic DNA of the host cell of the present application). For example, the microorganism may be selected from one or more of the following group: bacteria, fungi, actinomycetes, mycoplasma, chlamydia, rickettsia, and spirochetes.

In the present application, the microorganism may comprise Gram-negative bacteria. For example, the microorganism may be suitable for preparing a vector backbone of vectors. For example, the microorganism may comprise *Escherichia coli.*

In the present application, the content of the nucleic acid molecule or fragments thereof of at least about 10kb (e.g., at least about 15kb, at least about 20kb, at least about 30kb, at least about 35kb, at least about 40kb, at least about 48kb, or larger) (e.g., genomic DNA of the host cell of the present application) may account for about 10 (w/w)% or less, about 9 (w/w)% or less, about 8 (w/w)% or less, about 7 (w/w)% or less, about 6 (w/w)% or less, about 5 (w/w)% or less, about 4 (w/w)% or less, about 3 (w/w)% or less, about 2.5 (w/w)% or less, about 2 (w/w)% or less, about 1.5 (w/w)% or less, about 1 (w/w)% or less, about 9 (w/w)‰ or less, about 8 (w/w)‰ or less, about 7 (w/w)‰ or less, about 6 (w/w)‰ or less, about 5 (w/w)‰ or less, about 4 (w/w)‰ or less, about 3 (w/w)‰ or less, about 2 (w/w)‰ or less, about 1 (w/w)‰ or less, about 0.1 (w/w)‰ or less, about 0.01 (w/w)‰ or less, about 0.001 (w/w)‰ or less, or lower, in the transfection mixture derived from exogenous nucleic acid molecules of the host cell (e.g., plasmids). For example, the content of the nucleic acid molecule or fragments thereof of at least about 48kb (e.g., at least about 15kb, at least about 20kb, at least about 30kb, at least about 35kb, at least about 40kb, at least about 48kb, or larger) (e.g., genomic DNA of the host cell of the present application) may account for about 2% or less of the plasmid content in the transfection mixture; may account for about 5‰ or less of the plasmid content in the transfection mixture; may account for about 1‰ or less of the plasmid content in the transfection mixture.

### Transfection

Transfection is a method of intentionally introducing nucleic acids into cells. In some embodiments, transfection is non-viral, meaning that the sequences used in the plasmid context are non-viral, and the exogenous nucleic acid molecules do not enter cells via viral mechanisms. Transfection of animal cells typically involves the opening of transient pores or "holes" in the cell membrane to allow uptake of the material. Transfection may be carried out using methods known in the art and those described below.

In the present application, the transfection may comprise transient transfection. For example, the transfection may comprise electroporation. For example, the transfection may comprise electroporation of the cell to be modified. The electroporation may refer to both cell electroporation and cell electrotransfection. Electroporation can utilize the action of a powerful instantaneous battery to introduce charged substances into cells through a cell membrane with a certain permeability. Electroporation can produce minimal cytotoxicity. Compared to chemical transfection methods and/or viral transfection methods, the cytotoxicity produced by electroporation is significantly reduced. Electroporation may be applied to almost all types of eukaryotic cells. Electroporation may be used for transient or stable expression of exogenous proteins.

In the present application, the transfection may comprise transfection of transposon systems (e.g., may comprise Sleeping beauty transposon system, or PiggyBac (PB) transposon system).

In the present application, the transfection may comprise transfection of cells (e.g., the cell to be transfected or to be modified as described in the present application). In the present application, the cell may comprise eukaryotic cells. The eukaryotic cells may comprise plant cells, fungal cells, and/or animal cells. Among them, the animal cells may comprise mammalian cells (e.g., human cells, such as immune cells, such as T cells, such as human PBMCs).

In the present application, other transfection methods known in the art can also be comprised, such as chemically-based transfection methods and non-chemically-based transfection methods. Chemically-based transfection methods may comprise, for example, calcium phosphate, dendrimers, lipofection, and cationic polymers (e.g., DEAE-dextran or polyethyleneimine) methods. Non-chemical methods may comprise cell squeezing, sonoporation, optoporation, impalefection, and hydrodynamic delivery, etc. Particle-based methods, such as gene gun transfection, magnetofection (i.e., magnetically-assisted transfection), and particle bombardment, are also comprised.

In some embodiments, electroporation is used to assist one or more nucleic acid molecules in entering host cells. In the present application, "electroporation" or "electroloading" generally refers to applying current or electric field to cells to facilitate the entry of nucleic acid molecules into cells. For example, flow electroporation may be performed using a flow electroporation instrument. In some embodiments, static electroporation methods may be employed.

In the methods of the present application, transfection efficiencies of greater than about 35%, greater than about 40%, greater than about 50%, greater than about 60%, greater than about 70%, greater than about 80%, or greater than about 90% (or any range derivable therefrom) may be achieved after electroporating cells. Transfection efficiency may be measured by the percentage of cells expressing gene products or by the secretion levels of products expressed by the gene. During and after the electroporation process, cells maintain high viability. The viability is generally greater than about 40% or higher. The viability of electroporated cells may be at least about 20%, at least about 25%, at least about 30%, at least about 35%, at least about 40%, at least about 45%, at least about 50%, at least about 55%, at least about 60%, at least about 65%, at least about 70%, at least about 75%, at least about 80%, at least about 85%, at least about 90%, at least about 95%, or higher compared to the initial non-electroporated cell population or electroporated cell population transfected with a control construct.

Cell squeezing is a transfection method that delivers molecules to cells by gently squeezing the cell membrane. Cell squeezing is a high-throughput, carrier-free microfluidic platform for intracellular delivery. Cell squeezing does not rely on exogenous materials or electric fields.

Sonoporation uses high-intensity ultrasound to induce pore formation in the cell membrane. This pore formation is mainly attributed to bubble cavitation interacting with the nearby cell membrane, enhanced by the addition of ultrasound contrast agents (a source of cavitation nuclei).

Optoporation is a method of using highly focused lasers to instantaneously create tiny (approximately 1µm in diameter) pores in the cell's plasma membrane. In this technique, cells are processed one at a time, making it particularly suitable for single-cell analysis.

Hydrodynamic delivery is performed in mice and rats, but to a lesser extent in larger animals, using hydrodynamic injection to deliver DNA (comprising transposons) typically in plasmids to the liver, which involves infusing a relatively large volume of blood in less than 10 seconds; almost all of the DNA is expressed in the liver through this process.

Chemically-based transfection may be divided into several categories: cyclodextrins, polymers, liposomes, or nanoparticles (with or without chemical or viral processes acting). For example, in the calcium phosphate method, a HEPES-buffered saline solution (HeBS) containing phosphate ions is combined with a calcium chloride solution containing the DNA to be transfected. When the two are combined, a fine precipitate of positively charged calcium and negatively charged phosphate forms, with the DNA to be transfected binding to its surface. The suspension of the precipitate is then added to the cells to be transfected (usually monolayer cell cultures). Cells take up some of the precipitate along with the associated DNA. Other methods use highly branched organic compounds (so-called dendrimers) to bind DNA and facilitate its entry into cells. A very effective method is to encapsulate the DNA to be transfected in liposomes, which are small membrane-coated vesicles that, in some ways, resemble cell structures and can actually fuse with the cell membrane, releasing DNA into the cell. For eukaryotic cells, the use of cationic liposomes (or mixtures thereof) achieves better transfection because cells are more sensitive. Another method is to use cationic polymers, such as DEAE-dextran or polyethyleneimine. Negatively charged DNA binds to polycations, and the complex is accepted by cells through endocytosis.

In some embodiments, a direct transfection method is using a gene gun, in which DNA is coupled to inert solid nanoparticles (typically gold), and then directly "shot" into the nucleus of the target cell.

Magnetofection or magnet-assisted transfection is a transfection method that uses magnetic force to deliver DNA to target cells. First, nucleic acids are associated with magnetic nanoparticles. Then, magnetic force is applied to deliver the nucleic acid-particle complexes to the target cells and into the target cells, where the payload is released.

Puncture transfection is performed by piercing cells with elongated nanostructures and arrays of such nanostructures, such as carbon nanofibers or silicon nanowires functionalized with plasmid DNA.

Another particle-based transfection method is called particle bombardment. Nucleic acids, typically connected to microprojectiles, are delivered at high speed through membrane penetration.

In the present application, the transfection may comprise stable transfection. For example, the transfection may stably express the exogenous protein encoded by the exogenous gene in the transfected cell. For example, the transfection may cause the exogenous gene to be integrated into the genome of the transfected cell. In the present application, the integration may occur at a specific location in the genome.

### Cell to be transfected or modified

In the present application, the terms "cell", "cell line", and "cell culture" may be used interchangeably. All these terms also comprise freshly isolated cells, as well as cells that have been cultured, activated, or expanded ex *vivo.* All these terms also comprise their progeny, i.e., any and all descendants. It should be understood that progeny cells may be not identical due to intentional or unintentional mutations.

In the present application, the cell to be transfected or modified may be a eukaryotic cell. For example, the cell may be a mammalian cell. For example, the cell may be a human cell.

For example, the cell to be transfected comprises stem cells, immune cells, fibroblasts, fibrocytes, and/or muscle cells. For example, the cell may be a multipotent stem cell, a hematopoietic stem cell, and/or a mesenchymal stem cell.

For example, the cell may be an immune effector cell. For example, the cell may be a T lymphocyte, B lymphocyte, NK cell, macrophage, dendritic cell, monocyte, granulocyte, and/or mast cell. For example, the cell may be a peripheral blood lymphocyte.

For example, the cell may be a primary cell. For example, the cell may be an autologous cell derived from the subject.

For example, the cell to be modified may be an activated cell. For example, the activation may comprise contacting the cell to be modified with an activation composition.

For example, the cell may comprise immune cells (e.g., immune effector cells, such as T cells or NK cells, e.g., PBMCs, e.g., primary or autologous T cells or NK cells, e.g., primary or autologous PBMCs), and the activation compositioncompound may comprise anti-CD3 and/or anti-CD28 antibodies (e.g., the antibodies may be provided on magnetic beads).

Reagents or kits for activating T cells are also commercially available. Exemplary kits comprise streptavidin particles (e.g., MACSiBead or Dynabead) and biotinylated antibodies against human CD2, CD3, and CD28. Streptavidin particles loaded with biotinylated antibodies are used to simulate antigen-presenting cells and activate resting T cells from PBMCs as well as purified T cells. T cell expansion is achieved by culturing and reactivating on day 14 of cultivation. For example, T cells may also be activated by mitogens (e.g., ConA, PHA, and PWM).

For example, the activation may comprise contacting the cell to be modified with the activation composition for no more than about 4 days (e.g., within about 96 hours, 90 hours, 85 hours, 80 hours, 75 hours, 72 hours, 70 hours, 65 hours, 60 hours, 55 hours, 50 hours, 48 hours, 45 hours, 40 hours, 36 hours, 30 hours, 24 hours, 20 hours, 15 hours, 12 hours, 8 hours, or shorter times). For example, the activation may comprise contacting the cell to be modified with the activation composition for about 10 hours to about 48 hours (e.g., about 12 hours to about 24 hours). For example, the method may comprise performing the transfection while the cell to be transfected is in contact with the activation composition for about 2 days or shorter periods.

In some embodiments, transfection may be carried out on any prokaryotic or eukaryotic cells. In some aspects, electroporation involves transfecting human cells. In other aspects, electroporation involves transfecting animal cells. In some aspects, transfection involves transfecting cell lines or hybrid cell types. In some aspects, the cell to be transfected is a cancer cell, tumor cell, or immortalized cell. In some cases, tumors, cancers, immortalized cells, or cell lines are induced, while in other cases, tumors, cancers, immortalized cells, or cell lines naturally enter their respective states or conditions. In some aspects, cells or cell lines may comprise A549, B cells, B16, BHK-21, C2C12, C6, CaCo-2, CAP/, CAP-T, CHO, CHO2, CHO-DG44, CHO-K1, COS-1, Cos-7, CV-1, dendritic cells, DLD-1, embryonic stem (ES) cells or derivatives, H1299, HEK, 293, 293T, 293FT, Hep G2, hematopoietic stem cells, HOS, Huh-7, induced pluripotent stem cells (iPSC) or derivatives, Jurkat, K562, L5278Y, LNCaP, MCF7, MDA-MB-231, MDCK, mesenchymal cells, Min-6, monocytes, Neuro2a, NIH 3T3, NIH3T3L1, K562, NK-cells, NS0, Panc-1, PC12, PC-3, peripheral blood cells, plasma cells, primary fibroblasts, RBL, Renca, RLE, SF21, SF9, SH-SY5Y, SK-MES-1, SK-N-SH, SL3, SW403, Stimulus-triggered Acquisition of Pluripotency (STAP) cells or derivatives SW403, T-cells, THP-1, tumor cells, U2OS, U937, peripheral blood lymphocytes, expanded T-cells, hematopoietic stem cells, or Vero cells. In some specific embodiments, the cell is a peripheral blood lymphocyte, expanded T-cell, natural killer cell (NK cell), stem cell, hematopoietic stem cell, or primary cell. In some specific embodiments, the cell is a hematopoietic stem cell. In other specific embodiments, the cell is peripheral blood lymphocytes and/or peripheral blood mononuclear cells (PBMC).

In some embodiments, the cell is a difficult-to-transfect cell known in the art. Such cells are known in the art and comprise, for example, primary cells, insect cells, SF9 cells, Jurkat cells, CHO cells, stem cells, slowly dividing cells, T-cells, and non-dividing cells. In some embodiments, the cell is a T-cell. In some embodiments, the cell is a primary cell. In some embodiments, the cell is a stem cell. In some embodiments, the cell is a hematopoietic stem cell, comprising bone marrow and lymphoid progenitors. In some embodiments, the cell is a mesenchymal stem cell. In some embodiments, the cell is a germ cell, such as an oocyte or sperm cell. In some embodiments, the cell is a fertilized embryo. In some embodiments, the cell is a human fertilized embryo.

In some embodiments, cells may be cultured before or after transfection. For example, cells may be cultured during a post-transfection selection stage, during a maintenance and cloning selection and initial expansion stage, during a screening stage, and/or during a large-scale production stage. Methods for culturing suspension and adherent cells are known to those skilled in the art. In some embodiments, cells may be cultured using commercially available cell culture containers and cell culture media. For example, ADME/TOX plates, cell chamber slides and cover slips, cell counters, cell culture surfaces, Corning HYPER Flask cell culture containers, coated culture dishes, Nalgene Cryoware, culture chambers, culture dishes, glass culture flasks, plastic culture flasks, 3D culture formats, culture multiwell plates, culture plate inserts, glass culture tubes, plastic culture tubes, stackable cell culture containers, hypoxia culture chambers, culture dishes (Petri dish) and bottle carriers, rapid culture containers, large-scale cell culture using roller bottles, rotating flasks, 3D cell culture, or cell culture bags, etc.

In the present application, the cell may comprise stem cells, immune cells, fibroblasts, and/or muscle cells. For example, the stem cells may comprise pluripotent stem cells. For example, the pluripotent stem cells may comprise embryonic stem cells (ESCs). The embryonic stem cells may differentiate into three germ layers (e.g., ectoderm, mesoderm, and endoderm), and these three germ layers may eventually form organs and tissues. The pluripotent stem cells may comprise epiblast stem cells (EpiSCs). The pluripotent stem cells may also comprise induced pluripotent stem cells (e.g., iPS), which may be formed by dedifferentiation of mammalian somatic cells (e.g., skin cells from a mouse tail) after the introduction of transcription factors (e.g., Oct4, SOX2, c-Myc, and Klf4).

In the present application, the stem cells may comprise hematopoietic stem cells and/or mesenchymal stem cells. The hematopoietic stem cells may differentiate into blood cells (e.g., blood cells of the bone marrow lineage and blood cells of the lymphoid lineage). The hematopoietic stem cells may have multipotency and self-renewal properties. The hematopoietic stem cells may differentiate into cells selected from the following group: monocytes, macrophages, neutrophils, basophils, eosinophils, erythrocytes, megakaryocytes, platelets, T cells, B cells, and NK cells.

The mesenchymal stem cells may be adult stem cells with self-renewal, multilineage differentiation potential, and the ability to maintain their biological characteristics after large-scale *in vitro* expansion, derived from the early mesoderm of embryonic development. The mesenchymal stem cells may express HLA-I class antigens. The mesenchymal stem cells may not express or have low expression of HLA-II class antigens. The mesenchymal stem cells may have the ability to differentiate into adipocytes, osteoblasts, and chondrocytes, and may support the differentiation of hematopoietic stem cells into granulocytes, macrophages, and megakaryocytes. The mesenchymal stem cells may secrete cytokines, for example, they may secrete CSF-1, GM-CSF, G-CSF, IL-6, c-kit ligand, and/or IL-3.

In the present application, the immune cells may comprise immune effector cells. For example, the immune effector cells may comprise lymphocytes (e.g., cytotoxic T cells, memory T cells), macrophages, dendritic cells, and NK cells. For example, the immune cells may be selected from the group consisting of: T lymphocytes (e.g., which may be activated T lymphocytes, or may be non-activated T lymphocytes), B lymphocytes, NK cells, macrophages, dendritic cells, monocytes, granulocytes, and mast cells.

### Transfection Mixture

In the present application, the transfection mixture may comprise exogenous nucleic acid molecules (e.g., plasmids). For example, the plasmid may be a circular plasmid, supercoiled plasmid, or linear plasmid. In the present application, the plasmid (e.g., linear plasmid) may be treated (e.g., with a deoxyribonuclease, such as an exonuclease, such as Exonuclease V) to satisfy the conditions of the present application for the content of nucleic acid molecules or fragments thereof (e.g., host genomic DNA, which can be linear nucleic acid molecules or fragments thereof) with a size of at least about 10kb (e.g., at least about 15kb, at least about 20kb, at least about 30kb, at least about 35kb, at least about 40kb, at least about 48kb or larger), and/or nucleic acid molecules or fragments thereof (e.g., which can be linear nucleic acid molecules or fragments thereof) derived from the host (e.g., microorganism) genome. In the present application, the treatment may not affect nucleic acid molecules with a circular structure.

For example, the transfection mixture of the present application may substantially not contain any viral vectors, such as the content of viral vectors being less than about 2 (w/w)%, less than about 1 (w/w)%, less than about 0.9 (w/w)%, less than about 0.8 (w/w)%, less than about 0.7 (w/w)%, less than about 0.6 (w/w)%, less than about 0.5 (w/w)%, less than about 0.4 (w/w)%, less than about 0.3 (w/w)%, less than about 0.2 (w/w)%, or less than about 0.1 (w/w)%.

In the present application, the plasmid may be a DNA plasmid. For example, the DNA plasmid may be a double-stranded, circular DNA molecule. For example, the DNA plasmid may be a double-stranded, linear DNA molecule.

In the present application, the plasmid may comprise multiple cloning sites. The plasmid may encode at least one exogenous gene. The exogenous gene may encode one or more antigen-binding fragments (for example, antibodies), chimeric antigen receptors, cytokines, and/or chemokines. The exogenous gene may also encode any one or more functional proteins. For example, the functional protein may be used to treat diseases related to gene defects. For example, the functional protein may be a protein that is missing and/or mutated in the organism where the transfected cell is located. For example, the exogenous gene expressing the functional protein may be transfected into fibrocytes (for example, primary fibrocytes), muscle cells, and/or stem cells (for example, iPSC cells).

In the present application, the exogenous gene may be expressed with the help of endogenous or exogenous promoters. For example, the plasmid may comprise the exogenous promoter.

In the present application, the host's genomic DNA (for example, nucleic acid molecules or fragments thereof with a size of at least about 10kb (for example, at least about 15kb, at least about 20kb, at least about 30kb, at least about 35kb, at least about 40kb, at least about 48kb, at least about 50kb, at least about 100kb, at least about 150kb, at least about 200kb, at least about 250kb, at least about 300kb, at least about 350kb, at least about 400kb, at least about 450kb, at least about 500kb, at least about 600kb, at least about 700kb, at least about 800kb, at least about 900kb, at least about 1 Mb, at least about 2Mb, at least about 3Mb, at least about 4Mb, at least about 5Mb, at least about 6Mb, at least about 7Mb, at least about 8Mb, at least about 9Mb, at least about 10Mb, at least about 20Mb, at least about 50Mb, at least about 100Mb, at least about 200Mb or larger)) and the plasmid may exist in different nucleic acid molecules. For example, the host's genomic DNA (for example, the nucleic acid molecules or fragments thereof of at least about 10kb (for example, at least about 15kb, at least about 20kb, at least about 30kb, at least about 35kb, at least about 40kb, at least about 48kb or larger)) may be free in the transfection composition. For example, the host's genomic DNA (for example, the nucleic acid molecules or fragments thereof of at least about 10kb (for example, at least about 15kb, at least about 20kb, at least about 30kb, at least about 35kb, at least about 40kb, at least about 48kb or larger)) may be dissociated from the plasmid. The dissociation can be separate and exist in a form that does not attach to the plasmid.

In another aspect, the present application provides a transfection mixture prepared by the method described in the present application.

In the present application, the transfection mixture may significantly improve transfection efficiency. The transfection mixture may significantly reduce cytotoxicity caused by transfection. The transfection mixture may be directly applicable for cell transfection. The method described in the present application may serve as a quality control standard for the preparation and/or quality inspection of the transfection mixture.

### Optimization of transfection composition

For example, the method may also comprise treating the exogenous nucleic acid molecules (for example, plasmid) derived from the host cells before transfection to reduce the content of the host cell's genomic DNA therein.

For example, the treatment may comprise contacting the exogenous nucleic acid molecules (for example, plasmid) derived from the host cells with one or more reagents selected from the following group: deoxyribonuclease (DNase), SDS, TX-100, CTAB, and cesium chloride-ethidium bromide. For example, the DNase may non-specifically cleave linear DNA. For example, the DNase may comprise an exonuclease. For example, the treatment may comprise contacting the exogenous nucleic acid molecules (for example, plasmid) derived from the host cells with the reagent in the presence of Mg²⁺ and Ca²⁺.

The method of the present application may comprise the following steps: reducing the content of host genomic DNA in the transfection mixture. In the present application, the reduction may refer to reducing the content of the host genomic DNA in the transfection mixture to meet the requirements of the method described in the present application.

In the present application, the reduction may comprise purifying the transfection mixture. That is, the content of nucleic acid molecules or fragments thereof in the transfection mixture may be reduced to meet the requirements of the method of the present application by the purification. In the present application, at least about 10kb (e.g., at least about 15kb, at least about 20kb, at least about 30kb, at least about 35kb, at least about 40kb, at least about 48kb or larger) size nucleic acid molecules or fragments thereof may be removed by the purification; and/or, nucleic acid molecules or fragments thereof derived from the host (e.g., microorganism) genome may be removed. For example, the purification may comprise reducing the content of nucleic acid molecules or fragments thereof in the transfection mixture using methods for purifying DNA known to those skilled in the art. In the present application, the purification may not affect the integrity and/or activity of the plasmid DNA. In the present application, the reduction may comprise purifying the transfection mixture using reagents selected from the group consisting of: DNAase, SDS, TX-100, CTAB, and cesium chloride-ethidium bromide. For example, the reduction may use DNAase. For example, DNAase may be used to reduce the content of at least about 10kb (e.g., at least about 15kb, at least about 20kb, at least about 30kb, at least about 35kb, at least about 40kb, at least about 48kb or larger) size nucleic acid molecules or fragments thereof in the transfection mixture to meet the requirements of the method of the present application; and/or, to reduce the content of nucleic acid molecules or fragments thereof derived from the host (e.g., microorganism) genome to meet the requirements of the method of the present application. For example, the DNAase may not affect the integrity and/or activity of the circular plasmid DNA.

In the present application, the DNase may cleave double-stranded DNA. For example, the DNase may non-specifically cleave linear DNA.

The DNase may not affect the integrity and/or activity of the plasmid DNA of the present application. The DNase may not affect the integrity and/or activity of the exogenous gene in the plasmid of the present application (e.g., the circular plasmid).

In the present application, the DNase may (e.g., non-specifically) cleave linear (e.g., double-stranded linear) DNA. For example, the DNase may not affect the structure and/or activity of the plasmid (e.g., circular plasmid). In the present application, the DNase may comprise deoxyribonuclease. In the present application, the DNase may comprise DNA exonuclease. In the present application, the DNase may comprise exonuclease V. In the present application, the DNase may comprise ATP-Dependent DNase, such as Plasmid-Safe^{™} ATP-Dependent DNase.

For example, the method of the present application may comprise the following steps: contacting the DNase with the plasmid (or transfection composition) of the present application. In the present application, the contact may comprise contacting in the presence of a buffer (e.g., the buffer may be from the kit sold with the DNase). In the present application, the buffer may comprise Mg²⁺ and Ca²⁺. For example, the buffer may contain Mg²⁺ and Ca²⁺ and no other additional cations. The buffer may improve the enzymatic efficiency of the DNase.

In the method of the present application, the DNase treatment may comprise the following steps: purifying the plasmid after the contact.

After the DNase treatment, the content of at least about 10kb (e.g., at least about 15kb, at least about 20kb, at least about 30kb, at least about 35kb, at least about 40kb, at least about 48kb or larger) size nucleic acid molecules or fragments thereof in the transfection mixture comprising the plasmid containing the exogenous gene may account for less than about 10% of the total nucleic acid molecule content of the transfection mixture; and/or, after the DNase treatment, the content of nucleic acid molecules or fragments thereof derived from the host (e.g., microorganism) genome in the transfection mixture comprising the plasmid containing the exogenous gene may account for less than about 10% of the total nucleic acid molecule content of the transfection mixture.

In the present application, after treatment with the DNase, the content of large fragment nucleic acid molecules (at least about 10 kb, for example, at least about 15 kb, at least about 20 kb, at least about 30 kb, at least about 35 kb, at least about 40 kb, at least about 48 kb or larger) and/or nucleic acid molecules or fragments thereof originating from the host (e.g., microorganisms) in the transfection mixture comprising the plasmid containing the exogenous gene can be within the content range requirements described in the present application.

In the present application, the contact may also comprise further contact with an RNase.

In the present application, a nuclease or DNase is an enzyme that hydrolyzes nucleic acids. Nucleases may be classified as endonucleases or exonucleases. Endonucleases are any group of enzymes that catalyze the hydrolysis of the bonds between nucleotides within DNA or RNA molecules. Exonucleases are any group of enzymes that catalyze the hydrolysis of individual nucleotides from the ends of DNA or RNA chains. Nucleases may also be classified based on whether they specifically digest DNA or RNA. Nucleases that specifically catalyze the hydrolysis of DNA may be called deoxyribonucleases or DNAases, while nucleases that specifically catalyze the hydrolysis of RNA may be called ribonucleases or RNAases. Some nucleases have specificity for single-stranded or double-stranded nucleic acid sequences. Some enzymes have both exonuclease and endonuclease characteristics. In addition, some enzymes are capable of digesting both DNA and RNA sequences.

In different nucleases, optimal reaction conditions are different. Factors to consider comprise temperature, pH, enzyme cofactors, salt composition, ionic strength, and stabilizers. Suppliers of commercially available nucleases (e.g., Promega Corp.; New England Biolabs, Inc.) provide information on the optimal conditions for various enzymes. As measured at incubation temperature, most nucleases are used between pH 7.2 and pH 8.5. In addition, most nucleases exhibit maximum activity at 37°C; however, a few enzymes require higher or lower temperatures for optimal activity (e.g., Taq I, 65°C; Sma I, 25°C). DNA concentration may also be a factor, as high DNA concentration may reduce enzyme activity, and too diluted DNA concentration may be below the enzyme's Km and also affect enzyme activity. Non-limiting examples of nucleases comprise DNAse I, Benzonase, exonuclease I, exonuclease III, Mung Bean Nuclease, nuclease BAL 31, RNase I, S1 nuclease, Lambda Exonuclease, RecJ, and T7 Exonuclease. DNAse I is an endonuclease that non-specifically cleaves DNA to release dinucleotides, trinucleotides, and oligonucleotides with 5'-phosphorylated and 3'-hydroxylated termini. DNAse I acts on single-stranded and double-stranded DNA, chromatin, and RNA:DNA hybrids. Exonuclease I catalyzes the removal of nucleotides from single-stranded DNA in the 3' to 5' direction. Exonuclease III catalyzes the stepwise removal of mononucleotides from the 3'-hydroxyl termini of double-stranded DNA. Exonuclease III also acts on nicks in double-stranded DNA to produce single-stranded gaps. Single-stranded DNA is resistant to exonuclease III. Mung Bean Nuclease degrades single-stranded extensions from DNA ends. Mung Bean Nuclease is also an RNA endonuclease. Nuclease BAL 31 degrades both 3' and 5' ends of double-stranded DNA. Nuclease BAL 31 is also a highly specific single-stranded endonuclease that cleaves at nicks, gaps, and single-stranded regions in double-stranded DNA and RNA. RNase I is a single-strand-specific RNA endonuclease that cleaves at all RNA dinucleotide sites. S1 nuclease degrades single-stranded DNA and RNA by endonucleolysis to obtain 5'-phosphorylated termini products. Double-stranded nucleic acids (DNA:DNA, DNA:RNA, or RNA:RNA) are resistant to S1 nuclease degradation, except at very high enzyme concentrations. Lambda Exonuclease catalyzes the removal of 5' mononucleotides from double-stranded DNA. Its preferred substrate is 5'-phosphorylated double-stranded DNA, although Lambda Exonuclease also degrades single-stranded and non-phosphorylated substrates at a greatly reduced rate. Lambda Exonuclease cannot initiate DNA digestion at breaks or nicks. RecJ is a single-stranded DNA-specific exonuclease that catalyzes the removal of deoxynucleotide monophosphates from DNA in the 5' to 3' direction. T7 Exonuclease catalyzes the removal of 5' mononucleotides from double-stranded DNA. T7 Exonuclease catalyzes the removal of nucleotides from the 5' end or at nicks and gaps in double-stranded DNA.

In the present application, the reduction may comprise using methods selected from the following group: artificial DNA synthesis of plasmids described in the present application and HPLC detection of transfection mixtures described in the present application. For example, direct DNA artificial synthesis may be performed based on the nucleotide sequence of the plasmid, resulting in a plasmid containing only the nucleotide sequence of that plasmid. For example, HPLC detection of transfection mixtures described in the present application may be performed, and based on the HPLC detection results, the characteristic peak corresponding to the plasmid may be selected, the component corresponding to the characteristic peak may be collected, and the obtained plasmid contains only the nucleotide sequence of that plasmid.

### Gene Editing System

In the present application, the transfection composition also comprises a gene editing system capable of integrating the exogenous gene into a specific location of the cell genome. For example, the gene editing system comprises a site-specific enzyme or a nucleic acid molecule encoding the enzyme. For example, the site-specific enzyme is selected from: transcription activator-like effector nucleases (TALEN), zinc-finger nucleases (ZFN), transposases, integrases, and Cas proteins. For example, the Cas protein is Cas9 protein. For example, the gene editing system further comprises one or more guide RNAs. For example, the guide RNA is complementary to the nucleic acid sequence in the target region of the cell genome.

For example, the gene editing system may also comprise one or more guide RNAs targeting one or more genes to be knocked out.

In some embodiments, the gene editing system may also comprise one or more guide RNAs targeting PD-1.

In some embodiments, the gene editing system may also comprise one or more guide RNAs targeting CD95.

For example, the gene editing system comprises a ribonucleoprotein complex (RNP), and the RNP comprises the Cas protein and the guide RNA.

For example, the gene editing systems and methods may be known to those skilled in the art, as long as the purpose of gene editing may be achieved, and not limited to a specific method. In the present application, the gene editing method may be selected from one or more of the following groups: CRISPR/Cas system, RNA editing system ADAR, RNA-guided endonucleases, zinc-finger proteinases, Mega-TAL nucleases, TALENs, and Meganucleases. For example, the gene editing method may be the CRISPR/Cas system.

In the present application, gene editing may comprise gene editing knockout and/or gene editing knock-in. For example, gene editing may comprise gene editing knock-in. For example, the CRISPR/Cas system may be used for gene editing knock-in.

For example, the CRISPR-CAS system may comprise a class of clustered regularly interspaced short palindromic repeats (CRISPRs) and some functionally related proteins (CRISPR-associated, Cas). The Cas protein-encoding genes may comprise Cas9, Cas1, Cas2, and Csn2. For example, it may be Cas9. The CRISPR-CAS system (e.g., CRISPR/Cas9 system) can have target-specific cutting specificity for DNA molecules.

For example, the gene editing knock-in may be a process where Cas protein targets a specific DNA sequence in the cell for DNA sequence insertion. The gene editing knock-in may be a process mediated by the Cas protein (e.g., Cas9 protein) that allows the exogenous gene to be knocked-in from the donor plasmid to undergo homologous recombination with the specific DNA sequence targeted in the cell.

In the present application, the exogenous nucleic acid molecule (e.g., plasmid) can serve as a donor plasmid in gene editing knock-in. For example, the donor plasmid may be double-stranded DNA or single-stranded DNA. The donor plasmid can serve as a donor template for the HDR repair mechanism.

In the present application, the donor plasmid may comprise nucleic acid molecules or fragments thereof of the exogenous gene to be knocked-in.

The site-specific enzyme of the present application can cleave the bonds between specific nucleotide subunits in the nucleic acid sequence (i.e., phosphodiester bonds). In a specific embodiment, the site-specific enzyme is encoded on RNA. In other embodiments, the site-specific enzyme is an enzyme-active protein, enzyme, or small molecule mimics. In some embodiments, the site-specific enzyme is encoded on DNA. In a specific embodiment, the site-specific enzyme is encoded on plasmid DNA. In some embodiments, the site-specific enzyme and donor DNA are encoded on the same plasmid.

In one embodiment, the site-specific enzyme is a transposase. The transposase may be a Sleeping Beauty transposase. In some cases, the transposase may be a PiggyBac (PB) transposase.

For example, a synthetic DNA transposon can be used, such as the "Sleeping Beauty" transposon system, which is designed for introducing a precisely defined DNA sequence into the chromosome of a vertebrate. The Sleeping Beauty transposon system consists of the Sleeping Beauty (SB) transposase and a transposon designed to insert a specific DNA sequence into the vertebrate genome. DNA transposons translocate from one DNA site to another in a simple cut-and-paste manner. Transposition is a precise process in which a defined DNA fragment is excised from one DNA molecule and moved to another site within the same or different DNA molecule or genome. The SB transposase inserts the transposon into the TA dinucleotide base pairs of the recipient DNA sequence. The insertion site may be another location within the same DNA molecule or within another DNA molecule (or chromosome). In mammalian (comprising human) genomes, there are approximately 200 million TA sites. During the transposon integration process, the TA insertion site is duplicated. The duplication of the TA sequence is a signature of transposition and is used to determine the mechanism in some experiments. The transposase may be encoded within the transposon, or the transposase may be provided by another source, in which case the transposon becomes a non-autonomous element. Non-autonomous transposons are the most useful genetic tools because they cannot independently excise and reinsert after integration. All DNA transposons identified in the human genome and other mammalian genomes are non-autonomous because although they contain transposase genes, these genes are non-functional and cannot produce a transposase capable of moving the transposon.

In the present application, the transposon system may comprise a transposon. The transposon may comprise a nucleic acid molecule capable of being incorporated into nucleic acids by transposase. The transposon may contain two transposon ends (also known as "arms") connected by a sequence long enough to form a loop in the presence of transposase. The transposon may be double-stranded, single-stranded, or mixed, containing single-stranded and double-stranded regions, depending on the transposase used for inserting the transposon. The transposase may comprise Mu, Tn3, Tn5, Tn7, and/or Tn10. The ends of the transposon may be double-stranded. In the present application, the transposon may be inserted into double-stranded DNA through transposition events. In the present application, the transposon may be considered to be the exogenous gene. The plasmid may contain the transposon. The plasmid may also contain the nucleic acid molecules necessary for the transposition event. For example, the transposon system may comprise the Sleeping Beauty transposon system. The Sleeping Beauty transposon system is a member of the Tc1/mariner transposon superfamily. For example, the transposon system may comprise the Piggy Bac transposon system.

In one embodiment, the site-specific enzyme is an integrase. For example, phiC31 integrase is a sequence-specific recombinase encoded within the genome of bacteriophage phiC31. PhiC31 integrase mediates the recombination between two 34 base pair sequences called attachment sites (att), one obtained from the bacteriophage and the other obtained from the bacterial host. It has been shown that this serine integrase works efficiently in many different cell types (comprising mammalian cells). In the presence of phiC31 integrase, donor plasmids containing attB may be unidirectionally integrated into the target genome by recombination at sites with sequences similar to the natural attP site (called pseudo-attP sites). PhiC31 integrase can integrate any size plasmid as a single copy without the need for cofactors. The integrated transgenes are stably expressed and heritable.

In one embodiment, the site-specific nuclease is a Cas nuclease. In a related embodiment, the Cas nuclease is Cas9. In another embodiment, the nuclease is cas9 and the composition further comprises guide RNA. Another example of a sequence-specific nuclease system that may be used in conjunction with the methods and compositions described herein comprises the Cas9/CRISPR system (Wiedenheft, B. et al. Nature 482, 331-338 (2012); Jinek, M. et al. Science 337, 816-821 (2012); Mali, P. et al. Science 339, 823-826 (2013); Cong, L. et al. Science 339, 819-823 (2013)). The Cas9/CRISPR (Clustered Regularly Interspaced Short Palindromic Repeat) system exploits RNA-guided DNA binding and sequence-specific target DNA cleavage. The guide RNA/Cas9 complex confers site specificity to the nuclease. The guide RNA (gRNA) comprises about 20 nucleotides complementary to the target genomic DNA sequence, which is upstream of the genomic PAM (Protospacer Adjacent Motif) site (NNG) and constant RNA scaffold region. The Cas (CRISPR-associated)9 protein binds to gRNA and the target DNA bound to gRNA, and introduces double-strand breaks at a defined position upstream of the PAM site. Cas9 has two independent nuclease domains homologous to HNH and RuvC endonucleases, and by mutating either of the two domains, Cas9 protein may be converted into a nickase that introduces single-strand breaks (Cong, L. et al. Science 339, 819-823 (2013)). In particular, the methods and compositions of the present invention may be used with both single-strand and double-strand induction forms of Cas9, as well as other RNA-guided DNA nucleases (e.g., Cas9-like systems from other bacteria).

The site-specific nucleases of the methods and compositions described herein may be modified, chimeric, or isolated from an organism. Sequence-specific nucleases may be introduced into cells in the form of RNA encoding a sequence-specific nuclease (e.g., mRNA).

In one embodiment, the site-specific enzyme is a site-specific nuclease, such as a zinc finger nuclease. Zinc finger nucleases typically comprise a DNA binding domain (i.e., zinc finger) and a cleavage domain (i.e., nuclease). The zinc finger binding domain may be engineered to recognize and bind any selected nucleic acid sequence. See, for example, Beerli et al. (2002) Nat. Biotechnol. 20: 135-141; Pabo et al. (2001) Ann. Rev. Biochem. 70: 313-340; Isalan et al. (2001) Nat. Biotechnol. 19: 656-660; Segal et al. (2001) Curr. Opin. Biotechnol. 12: 632-637; Choo et al. (2000) Curr. Opin. Struct. Biol. 10: 411-416; Zhang et al. (2000) J. Biol. Chem. 275(43): 33850-33860; Doyon et al. (2008) Nat. Biotechnol. 26: 702-708; and Santiago et al. (2008) Proc. Natl. Acad. Sci. USA 105: 5809-5814. Compared to naturally occurring zinc finger proteins, engineered zinc finger binding domains may have new binding specificities. Engineering methods comprise, but are not limited to, rational design and various types of selection. Rational design comprises, for example, using a database comprising dimer, trimer, and/or tetramer nucleotide sequences and individual zinc finger amino acid sequences, wherein each dimer, trimer, and/or tetramer nucleotide sequence is associated with one or more zinc finger amino acid sequences, the zinc fingers binding to specific trimer or tetramer sequences. See, for example, U.S. Patent No. 6,453,242 and 6,534,261, the disclosures of which are incorporated herein by reference in their entirety. As an example, the algorithm described in U.S. Patent 6,453,242 may be used to design zinc finger binding domains targeting preselected sequences. Alternative methods (e.g., rational design using non-degenerate recognition code tables) can also be used to design zinc finger binding domains targeting specific sequences (Sera et al. (2002) Biochemistry 41: 7074-7081). Web-based tools available to the public for identifying potential target sites in DNA sequences and designing zinc finger binding domains may be found at http://www.zincfingertools.org and http://bindr.gdcb.iastate.edu/ZiFiT/ (Mandell et al. (2006) Nuc. Acid Res. 34: W516-W523; Sander et al. (2007) Nuc. Acid Res. 35: W599-W605). Zinc finger binding domains may be designed to recognize and bind DNA sequences of about 3 nucleotides to about 21 nucleotides in length, or preferably about 9 to about 18 nucleotides in length. Generally, zinc finger binding domains comprise at least three zinc finger recognition regions (i.e., zinc fingers). In one embodiment, the zinc finger binding domain may comprise four zinc finger recognition regions. In another embodiment, the zinc finger binding domain may comprise five zinc finger recognition regions. In another embodiment, the zinc finger binding domain may comprise six zinc finger recognition regions. Zinc finger binding domains may be designed to bind to any suitable target DNA sequence. See, for example, U.S. Patent No. 6,607,882; 6,534,261 and 6,453,242, the disclosures of which are incorporated herein by reference in their entirety. In some embodiments, the zinc finger nuclease may also comprise a nuclear localization signal or sequence (NLS). The NLS is an amino acid sequence that facilitates targeting of the zinc finger nuclease protein to the nucleus to introduce double-strand breaks at the target sequence on the chromosome. Nuclear localization signals are known in the art. See, for example, Makkerh et al. (1996) Current Biology 6: 1025-1027. Zinc finger nucleases also comprise a cleavage domain. The part of cleavage domain of the zinc finger nuclease may be obtained from any endonuclease or exonuclease. Non-limiting examples of cleavage domains that may be derived from endonucleases comprise, but are not limited to, restriction endonucleases and homing endonucleases. See, for example, 2002-2003 Catalog, New England Biolabs, Beverly, Mass.; and Belfort et al. (1997) Nucleic Acids Res. 25: 3379-3388.

In another aspect, the targeted endonuclease may be a meganuclease. Meganucleases are characterized by large recognition sites of endonucleases, that is, recognition sites typically ranging from about 12 base pairs to about 40 base pairs. As a result of this requirement, recognition sites typically appear only once in any given genome. Naturally occurring meganucleases recognize 15 to 40 base pair cleavage sites and are generally divided into four families: LAGLIDADG family, GIY-YIG family, His-Cyst box family, and HNH family. Meganucleases may be targeted to specific chromosomal sequences by modifying their recognition sequences using techniques well-known to those skilled in the art.

In another aspect, the targeted endonuclease may be a transcription activator-like effector (TALE) nuclease. TALEs are transcription factors derived from the plant pathogen Xanthomonas, which may be easily modified to bind new DNA targets. TALEs or their truncated forms may be connected to the catalytic domains of endonucleases (e.g., Fok1) to produce targeted endonucleases called TALE nucleases or TALENs.

In another aspect, the nuclease may be a homing nuclease. Homing endonucleases comprise 1-5'cel, I-CeuI, I-PspI, Vl-Sce, I-SceTV, I-CsmI, I-PanI, I-SceII, I-PpoI, I-SceIII, I-CreI, I-TevI, 1-Tev, and I-7evIII. Their recognition sequences are known. See also U.S. Patent No. 5,420,032; U.S. Patent No. 6,833,252; Belfort et al. (1997) Nucleic Acids Res. 25: 3379-3388; Ou on et al. (1989) Gene 82: 115-118; Perler et al. (1994) Nucleic Acids Res. 22, 1125-1127; Jasin (1996) Trends Genet. 12: 224-228; Gimble et al. (1996) J. Mol. Biol. 263: 163-180; Argast et al. (1998) J. Mol. Biol. 280: 345-353; and New England Biolabs catalog.

In some embodiments, the site-specific enzyme comprises a modified (non-naturally occurring) homing endonuclease (meganuclease). The recognition sequences of homing endonucleases and meganucleases (e.g., I-SceI, I-CeuI, VI-PspI, VI-Sce, I-SceIN, I-CsmI, I-PanI, I-SceII, I-PpoI, I-SceIII, I-CreI, I-TevI, I-TevII, and I-7evIII) are known. See also U.S. Patent No. 5,420,032; U.S. Patent No. 6,833,252; Belfort et al. (1997) Nucleic Acids Res. 25: 3379-3388; Dujon et al. (1989) Gene 82: 115-118; Perler et al. (1994) Nucleic Acids Res. 22, 1125-1127; Jasin (1996) Trends Genet. 12: 224-228; Gimble et al. (1996) J. Mol. Biol. 263: 163-180; Argast et al. (1998) J. Mol. Biol. 280: 345-353; and New England Biolabs catalog. Furthermore, the DNA-binding specificity of homing endonucleases and meganucleases may be modified to bind non-natural target sites. See, for example, Chevalier et al. (2002) Molec. Cell 10: 895-905; Epinat et al. (2003) Nucleic Acids Res. 31: 2952-2962; Ashworth et al. (2006) Nature 441: 656-659; Paques et al. (2007) Current Gene Therapy 7: 49-66; U.S. Patent Application Publication No. 20070117128. The DNA-binding domains of homing endonucleases and meganucleases may be altered as a whole in the context of a nuclease (i.e., the nuclease comprises a homologous cleavage domain) or may be fused to a heterologous cleavage domain.

In one embodiment, the site-specific enzyme is a site-specific nucleic acid enzyme selected from or consisting of a group comprising omega, zinc finger, TALE, and CRISPR/Cas9.

For example, the gene editing system of this application

### Exogenous Nucleic Acid Molecule

For example, the exogenous nucleic acid molecule may be a plasmid. For example, the exogenous nucleic acid molecule may comprise circular nucleic acid molecules, supercoiled nucleic acid molecules, and/or linear nucleic acid molecules. For example, the exogenous nucleic acid molecule may be a DNA molecule. For example, the exogenous nucleic acid molecule may comprise single-stranded nucleic acid molecules and/or double-stranded nucleic acid molecules. For example, the concentration of the exogenous nucleic acid molecule in the transfection composition may be about 5 µg/mL to about 3000 µg/mL (e.g., about 5 µg/mL to about 2500 µg/mL, about 10 µg/mL to about 2000 µg/mL, about 10 µg/mL to about 1500 µg/mL, about 5 µg/mL to about 1000 µg/mL, about 10 µg/mL to about 1200 µg/mL, about 8 µg/mL to about 1000 µg/mL, about 15 µg/mL to about 950 µg/mL, about 20 µg/mL to about 900 µg/mL, about 30 µg/mL to about 900 µg/mL, about 40 µg/mL to about 950 µg/mL, about 50 µg/mL to about 950 µg/mL, about 60 µg/mL to about 950 µg/mL, about 70 µg/mL to about 950 µg/mL, about 80 µg/mL to about 950 µg/mL, about 90 µg/mL to about 950 µg/mL, about 100 µg/mL to about 950 µg/mL, about 110 µg/mL to about 950 µg/mL, about 120 µg/mL to about 950 µg/mL, about 130 µg/mL to about 950 µg/mL, about 140 µg/mL to about 950 µg/mL, about 150 µg/mL to about 950 µg/mL, about 180 µg/mL to about 950 µg/mL, about 200 µg/mL to about 950 µg/mL, about 200 µg/mL to about 850 µg/mL, about 200 µg/mL to about 800 µg/mL, about 250 µg/mL to about 850 µg/mL, about 300 µg/mL to about 750 µg/mL, about 350 µg/mL to about 700 µg/mL, about 400 µg/mL to about 650 µg/mL, about 450 µg/mL to about 600 µg/mL, about 500 µg/mL to about 550 µg/mL, etc.). In some embodiments, the concentration of the exogenous nucleic acid molecule in the transfection composition is about 200 µg/mL to about 800 µg/mL.

In the present application, the plasmid may comprise circular plasmids. For example, the plasmid may be enzymatically degraded without being affected by DNase treatment. For example, the plasmid may be a circular plasmid, supercoiled plasmid, and/or linear plasmid.

For example, the exogenous nucleic acid molecule may be obtained by extraction from the host cell. Common plasmid extraction methods are well known to those skilled in the art.

In the present application, the size of the exogenous nucleic acid molecule may be at least about 1 kb, for example, at least about 1.5 kb, at least about 2 kb, at least about 2.5 kb, at least about 3 kb, at least about 3.5 kb, at least about 4 kb, at least about 4.5 kb, at least about 5 kb, at least about 5.5 kb, at least about 6 kb, at least about 6.5 kb, at least about 7 kb, at least about 7.5 kb, at least about 8 kb, at least about 8.5 kb, at least about 9 kb, or larger.

For example, the exogenous nucleic acid molecule may also comprise one or more homologous regions. For example, each of the homologous regions may comprise at least 10 nucleotides (e.g., at least 20 nucleotides, at least 30 nucleotides, at least 40 nucleotides, at least 50 nucleotides, at least 60 nucleotides, at least 70 nucleotides, at least 80 nucleotides, at least 90 nucleotides, at least 100 nucleotides, at least 110 nucleotides, at least 120 nucleotides, at least 130 nucleotides, at least 140 nucleotides, at least 150 nucleotides, at least 160 nucleotides, at least 170 nucleotides, at least 180 nucleotides, at least 190 nucleotides, at least 200 nucleotides or more). For example, in the exogenous nucleic acid molecule, the homologous regions may be located at the 3' end and/or 5' end of the exogenous gene. In some embodiments, at least one of the homologous regions is located at the 3' end of the exogenous gene, and at least another of the homologous regions is located at the 5' end of the exogenous gene.

For example, the exogenous gene may be integrated into the genome of the cell. For example, the expression of the exogenous gene may be regulated by an endogenous promoter or an exogenous promoter. For example, after the exogenous gene is integrated into the genome of the cell, its expression may be regulated by an endogenous promoter or an exogenous promoter. For example, the homologous regions may be homologous to the target region of the cellular genomic DNA. For example, the target region may be located in the TCR-α constant gene (TRAC). For example, the target region may be located at the AAVS-I site.

For example, the exogenous gene may encode one or more exogenous proteins. For example, the exogenous proteins may comprise one or more of the following group: antibodies or antigen-binding fragments, chimeric antigen receptors (CAR), cytokines, and chemokines.

For example, the CAR may comprise a target-binding domain targeting tumor-associated antigens. For example, the tumor-associated antigens may be selected from: GPC3, CD19, BCMA, Claudin18.2, and Mesothelin. For example, the antibody or antigen-binding fragment may comprise multispecific antibodies or their antigen-binding fragments. For example, the multispecific antibodies or their antigen-binding fragments may simultaneously target immune effector cells (e.g., T cells, NK cells) and tumor cells. For example, the multispecific antibodies or their antigen-binding fragments may comprise bispecific T cell engagers (BiTEs). For example, the BiTE may comprise a CD3-binding domain. For example, the BiTE may also comprise a tumor-associated antigen-binding domain.

For example, the cytokines may comprise interleukins. For example, the interleukins may comprise IL15, IL17, or functional active fragments thereof.

For example, the chemokines may comprise CCL19 or functional active fragments thereof.

For example, the exogenous proteins may comprise the cytokines (e.g., interleukins; e.g., IL15, IL17, or functional active fragments thereof) and the chemokines (e.g., CCL19 or functional active fragments thereof).

For example, the exogenous proteins may comprise CAR (e.g., GPC3 CAR), the cytokines (e.g., interleukins; e.g., IL15, IL17, or functional active fragments thereof), and the chemokines (e.g., CCL19 or functional active fragments thereof).

For example, the exogenous proteins may comprise polyproteins. For example, the cleavable part may be comprised between 2 or more proteins in the polyproteins. For example, the cleavable part may comprise 2A peptides. For example, the 2A peptides may comprise P2A, T2A, F2A, or E2A.

For example, in the present application, the exogenous gene may comprise a gene encoding one or more proteins selected from the following group: antibodies (e.g., monoclonal antibodies, bispecific antibodies, and/or multispecific antibodies; Ig-type full-length antibodies (e.g., IgG antibodies); antibody fragments (e.g., VHH, Fab, Fab', (Fab)₂, scFv), cytokines, chemokines, chimeric antigen receptors, and MHC complexes (e.g., HLA-A, HLA-G).

For example, the exogenous gene may encode CAR. For example, the exogenous gene may simultaneously encode CAR and a cytokine (e.g., IL-15 or IL-7). For example, the exogenous gene may simultaneously encode CAR, a cytokine (e.g., IL-15 or IL-7), and a chemokine (e.g., CCL19). For example, the exogenous gene may encode CAR-BiTE (or CAR-2A-BiTE).

In the present application, the exogenous gene may be natural or engineered.

In the present application, the exogenous gene may comprise a nucleic acid molecule encoding a chimeric antigen receptor (CAR), wherein the exogenous gene may be integrated into the genome of the transfected cell.

In the present application, the size of the exogenous gene may be at least about 1000bp (e.g., at least about 1500bp, at least about 2000bp, at least about 2500bp, at least about 3000bp, at least about 3500bp, at least about 4000bp, at least about 4500bp, at least about 5000bp, at least about 5500bp, at least about 6000bp, at least about 6500bp, at least about 7000bp, at least about 7500bp, at least about 8000bp, at least about 8500bp, at least about 9000bp, at least about 9500bp or more), wherein the exogenous gene is integrated into the genome of the transfected cell. For example, the size of the exogenous gene may be at least about 1000bp, at least about 3000bp, or at least about 3500bp.

In some embodiments, the size of the exogenous gene may be up to about 10kb. For example, it may be up to about 9900bp, up to about 9800bp, up to about 9700bp, up to about 9600bp or more.

In some cases, the size of the exogenous gene may be significantly distinguishable from the size of the at least about 10kb (e.g., at least about 15kb, at least about 20kb, at least about 30kb, at least about 35kb, at least about 40kb, at least about 48kb or larger) nucleic acid molecule or fragments thereof (e.g., host genome DNA) described in the present application. For example, by SDS-PAGE, the band of the exogenous gene may be clearly observed at a different position in the same lane as the band of the at least about 10kb (e.g., at least about 15kb, at least about 20kb, at least about 30kb, at least about 35kb, at least about 40kb, at least about 48kb or larger) nucleic acid molecule or fragments thereof. In some cases, the size of the exogenous gene may be significantly distinguishable from the size of the nucleic acid molecule or fragments thereof derived from the host (e.g., microorganism) genome described in the present application. For example, by SDS-PAGE, the band of the exogenous gene may be clearly observed at a different position in the same lane as the band of the nucleic acid molecule or fragments thereof derived from the host (e.g., microorganism) genome.

In the present application, the exogenous gene may express one or more (e.g., one, two, three or more) proteins. For example, the exogenous gene may encode a chimeric antigen receptor (CAR). For example, the exogenous gene may encode only a chimeric antigen receptor (CAR). Furthermore, the exogenous gene may comprise a nucleic acid molecule encoding a chimeric antigen receptor (CAR) and/or a second protein, wherein the second protein is different from the chimeric antigen receptor. In the present application, the second protein may be any one or more proteins different from the chimeric antigen receptor.

For example, the chimeric antigen receptor may comprise at least one antigen-binding domain targeting an antigen. For example, the chimeric antigen receptor may specifically bind to one antigen. For example, the chimeric antigen receptor may specifically bind to two antigens, and/or, may specifically bind to at least two different epitopes of one antigen. In the present application, the antigen may be a tumor-associated antigen (TAA). The tumor-associated antigen may be a related antigen of the following tumor cells: for example, breast cancer cells, B-cell lymphoma, Hodgkin lymphoma cells, ovarian cancer cells, prostate cancer cells, mesothelioma, lung cancer cells (e.g., small cell lung cancer cells), non-Hodgkin B-cell lymphoma (B-NHL) cells, ovarian cancer cells, prostate cancer cells, mesothelioma cells, lung cancer cells (e.g., small cell lung cancer cells), melanoma cells, chronic lymphocytic leukemia cells, glioma, glioblastoma, neurotube cell tumor, colorectal cancer cells, etc.

Cancer cell-associated antigens can also be expressed by non-cancer cells. For example, the antigen may be CD19. For example, the antigen may be GPC3.

In some cases, the antigen-binding domain may be a single-chain antibody (scFv), or a cAb VHH (camelid antibody variable domain) and its humanized variants, IgNAR VH (shark antibody variable domain) and its humanized variants, sdAb VH (single-domain antibody variable domain) and "camelid-derived" antibody variable domains. The antigen-binding domain may also be a recognition domain based on T-cell receptor (TCR), such as single-chain TCR (scTv, a single-chain two-domain TCR containing VαVβ).

In the present application, the chimeric antigen receptor may comprise a transmembrane domain. The N-terminus of the transmembrane domain may be directly or indirectly connected to the C-terminus of the antigen-binding domain. In the present application, any transmembrane (TM) structure provided for inserting a peptide into the cell membrane of a eukaryotic cell (e.g., mammalian cell) may be applicable to the transmembrane domain.

For example, the transmembrane domain may comprise a transmembrane domain derived from a protein selected from the following: T-cell receptor α, β, or ζ chain, CD28, CD3e, CD45, CD4, CD5, CD8a, CD9, CD16, CD22, CD33, CD37, CD64, CD80, CD86, CD134, CD137, and CD154.

In some embodiments, the chimeric antigen receptor may comprise a hinge region. The hinge region may be located between the antigen-binding domain and the transmembrane domain. The hinge region may be an immunoglobulin heavy chain hinge region, or the hinge region may be a hinge region polypeptide from a receptor (e.g., from the hinge region of CD8).

The length of the hinge region may be about 4 to about 50 amino acids. For example, it may be about 4 to about 10 amino acids, about 10 to about 15 amino acids, about 15 to about 20 amino acids, about 20 to about 25 amino acids, about 25 to about 30 amino acids, about 30 to about 40 amino acids, or about 40 to about 50 amino acids.

The hinge region may comprise at least one cysteine. The amino acid sequence of the hinge region may be known in the art, see for example Tan et al. (1990) Proc. Natl.

Acad. Sci. USA 87:162; and Huck et al. (1986) Nucl. Acids Res.14:1779.

In the present application, the hinge region may comprise the amino acid sequence of a human IgG1, IgG2, IgG3, or IgG4 hinge region. For example, compared to the wild-type (naturally occurring) hinge region, the hinge region may comprise one or more amino acid substitutions and/or insertions and/or deletions.

In the present application, the chimeric antigen receptor may comprise co-stimulatory structural domains. The length of the co-stimulatory structural domain may be about 30 to about 70 amino acids. The co-stimulatory structural domain may be derived from a peptide of the receptor. For example, the co-stimulatory structural domain may be the intracellular part of a transmembrane protein. For example, the co-stimulatory structural domain may comprise co-stimulatory structural domains derived from the following proteins: 4-1BB (CD137), CD28, ICOS, OX-40, BTLA, CD27, CD30, GITR, and HVEM. For example, the co-stimulatory structural domain may comprise co-stimulatory structural domains derived from the following proteins: CD28, 4-1BB, OX-40, and ICOS.

In the present application, the chimeric antigen receptor may also comprise a linker. The linker may be located between the transmembrane structural domain and the co-stimulatory structural domain. The linker may be a linker peptide. For example, the linker peptide may have a length of about 6 to about 40 amino acids. The linker peptide may have any amino acid sequence as long as it can have a flexible structure.

In the present application, the chimeric antigen receptor may comprise an intracellular signal transduction structural domain. For example, the intracellular signal transduction structural domain may comprise intracellular signal transduction structural domains derived from the following proteins: CD8β, CD4, CD3ζ, CD28, CD134, and CD7. For example, the intracellular signal transduction structural domain may comprise signal transduction structural domains derived from CD3ζ.

In the present application, the intracellular signal transduction structural domain may comprise a part having an ITAM motif from a polypeptide containing an ITAM motif. For example, the intracellular signal transduction structural domain may comprise DAP12; FCER1G (Fcε receptor Iγ chain); CD3D (CD3δ); CD3E (CD3ε); CD3G (CD3γ); CD3Z (CD3ζ); and CD79A (antigen receptor complex-associated protein α chain).

In the present application, the second protein may be selected from at least one of the following group: cytokines, chemokines, and antibodies or their antigen-binding fragments. In the present application, the second protein may comprise any one or more functional proteins. For example, the functional protein may be used to treat gene defect-related diseases. For example, the functional protein may be a protein that is missing and/or mutated in the host (host) of the transfected cell. For example, the exogenous gene expressing the functional protein may be transfected into fibroblasts (e.g., primary fibroblasts), muscle cells, and/or stem cells (e.g., iPSC).

In the present application, the cytokine may be any required cytokine as long as it can mediate and/or regulate biological or cellular functions or processes (e.g., immune response). The cytokine may comprise lymphokines, monokines, and/or interleukins. The cytokine may also comprise variants of cytokines that comprise one or more amino acid mutations in the amino acid sequence of the corresponding wild-type cytokine, such as those described in Sauvé et al., Proc Natl Acad Sci USA 88, 4636-40 (1991); Hu et al., Blood 101, 4853-4861 (2003) and U.S. Patent Publication No. 2003/0124678; Shanafelt et al., Nature Biotechnol 18, 1197-1202 (2000); Heaton et al., Cancer Res 53, 2597-602 (1993) and U.S. Patent No. 5,229,109; U.S. Patent Publication No. 2007/0036752; WO 2008/0034473; WO 2009/061853.

For example, the cytokine may be selected from at least one of the following group: GM-CSF, IL-1α, IL-1β, IL-2, IL-3, IL-4, IL-5, IL-6, IL-7, IL-8, IL-10, IL-12, IL-15, IFN-α, IFN-β, IFN-γ, MIP-1α, MIP-1β, TGF-β, TNF-α, and TNF-β. For example, the cytokine may comprise IL-15. For example, the cytokine may comprise IL-7.

In the present application, the chemokine may be selected from at least one of the following group: CCL19, 9E3, AMCF, β-globulin, ENA-78, eotaxin, eotaxin-2, IP-10, KC, LIX, mig, MGSA, mob-I, NAP-2, NAP-3, NAP-4, PBSF, MGSA, mouse KC, MIP-2, MIP-1α, NAP-2, ENA-78, GCP-2, ACT-2, CIO, CCF18, DC-CK1, ELC, Exodus, FIC, GDCF, GDCF-2, HC-21, HCC-1, 1-309, JE, LAG-1, MARC, MCAF, MCP-1, MCP-2, MCP-3, MCP-4, MCP-5, MRP-2, RANTES, SDF, TARC, ATAC, Ltn, SCM-1, and neurotactin.

In the present application, the second protein may comprise multispecific antibodies (e.g., bispecific antibodies). For example, the second protein may comprise bispecific T-cell engagers (BiTE). For example, the bispecific T-cell engager may specifically bind at least one tumor-associated antigen (TAA) and T-cells. For example, the bispecific T-cell engager may specifically bind an antigen expressed on the surface of T-cells, such as the CD3 receptor. For example, the bispecific T-cell engager may specifically target MHC-independent tumor-associated antigens (TAAs).

The bispecific T-cell engager may comprise at least one antigen-binding part, such as at least one single-chain antibody scFv.

In the present application, the bispecific T-cell engager (BiTE) may target CD3 and CD19, CD3 and CEA, CD3 and PSMA, or EpCAM and CD3. For example, the bispecific T-cell engager may target CD3, CD28, and CD38. For example, the bispecific T-cell engager may comprise Blinatumomab (BLINCYTO^{®}), MT111, MT112, BAY2020112, MT110, AMG110, and/or SAR442257.

In the present application, when the exogenous protein comprises CAR and BiTE, the tumor-associated antigen targeted by the BiTE and the tumor-associated antigen targeted by the CAR may be the same or different. In some embodiments, the BiTE and the CAR target the same epitope. In some embodiments, the BiTE and the CAR target the same target but specifically recognize or bind to different epitopes of that target. In some embodiments, the BiTE and the CAR target different targets.

In the present application, the exogenous gene may express the chimeric antigen receptor and the cytokine described in the present application. In the present application, the exogenous gene may express the chimeric antigen receptor and the bispecific T-cell engager (BiTE) described in the present application.

In the present application, the exogenous nucleic acid molecule (e.g., plasmid) may comprise homology arms homologous to the target gene in the cell genome. For example, the homology arms may have nucleic acid sequences with at least about 80%, at least about 85%, at least about 90%, at least about 91%, at least about 92%, at least about 93%, at least about 94%, at least about 95%, at least about 96%, at least about 97%, at least about 98%, at least about 99%, or 100% identity to the 5' and/or 3' ends of the genomic DNA sequences at the intended knock-in locus in the cell to be transfected. For example, the homology arms may be 100-1000 bp in size. For example, the homology arms may be about 150 bp-1000 bp, about 200 bp-1000 bp, about 300 bp-1000 bp, about 400 bp-1000 bp, or about 800 bp-1000 bp.

In some embodiments, the exogenous nucleic acid molecule may be in the form of a nucleic acid vector. The term "vector" is used to refer to a carrier nucleic acid molecule into which a heterologous nucleic acid sequence may be inserted for introduction into a cell where it may be replicated and expressed. The nucleic acid sequence may be "heterologous," meaning that it is foreign to the cell to be transfected or the inserted nucleic acid sequence. Vectors comprise DNA, RNA, plasmids, cosmid, and artificial chromosomes (e.g., YACs). Those skilled in the art can construct vectors using recombinant preparation techniques (e.g., Sambrook et al., 2001; Ausubel et al., 1996). Vectors may be used to transfect cells to produce antibodies or other exogenous proteins.

In the present application, the exogenous nucleic acid molecule may comprise regulatory sequences, such as promoters. Promoters are generally regions in the nucleic acid sequence that control the initiation and rate of transcription. Promoters can contain gene elements that can bind regulatory proteins and molecules (e.g., RNA polymerase and other transcription factors). Promoters may be used in conjunction with or without "enhancers," which are cis-acting regulatory sequences involved in the transcriptional activation of nucleic acid sequences.

Vectors or constructs generally comprise at least one termination signal. The "termination signal" or "terminator" is a DNA sequence that specifies the specific termination of RNA transcripts by RNA polymerase. Therefore, in some embodiments, the termination signal for terminating RNA transcript production is considered. In eukaryotic systems, the terminator region may also contain specific DNA sequences that allow site-specific cleavage to expose polyadenylation sites. This sends a signal to a specific endogenous polymerase to add an extension sequence of about 200 A residues (polyA) to the 3' end of the transcript. RNA molecules modified with this polyA tail appear to be more stable and are translated more efficiently. Thus, in other embodiments involving eukaryotes, the terminator may comprise signals for cleaving RNA, e.g., the terminator signal may promote polyadenylation information.

The exogenous nucleic acid molecules of the present application may also comprise polyadenylation signals that affect the appropriate transcript polyadenylation.

In some embodiments, the exogenous nucleic acid molecule may comprise one or more replication origin sites (commonly called "ori"), which are specific nucleic acid sequences where replication begins. Alternatively, if the host cell is yeast, an autonomously replicating sequence (ARS) may be used.

In some specific embodiments, the transfection composition transfected into cells by electroporation is non-viral (i.e., does not contain any viral components). Non-viral methods are considered to reduce toxicity and/or improve the safety of the method.

### Cell performance evaluation

In the present application, the vitality of the cells may comprise indicators from the following group: cell survival rate, cell proliferation capacity, cell killing ability, cell secretion ability, cell preservation ability, and cell recovery ability.

In the present application, cell proliferation capacity may be reflected by the amount of cell proliferation (e.g., detectable by FACS); cell survival rate may be reflected by the proportion of live cells to total cells (e.g., detectable by FACS); cell secretion ability may be reflected by the expression level of exogenous proteins encoded by exogenous genes secreted by cells (e.g., detectable by flow cytometry, western blot, antigen-antibody specific binding reactions, etc.); cell killing ability may be reflected by the killing rate of cells on tumor cells *in vitro;* may be reflected by the effect of cells on the tumor volume of solid tumors in vivo; may be reflected by the effect of cells on the tumor size of non-solid tumors in vivo. In the present application, cell survival rate and/or cell proliferation capacity may reflect the cell preservation ability and cell recovery ability of the cells. For example, the cell preservation ability and/or cell recovery ability may be reflected by the cell survival rate and/or cell proliferation capacity of the cells under cryopreservation conditions (e.g., liquid nitrogen storage conditions) and after recovery (e.g., recovery from cryopreservation conditions).

### Quality Determination method

In another aspect, the present application provides a method for determining the quality of transfection composition, the method comprising: measuring the content of host cell genomic DNA in the transfection composition, and determining the quality of the transfection composition based on this content. For example, when the content of the host cell genomic DNA accounts for about 10% (w/w) or less of the total DNA amount in the transfection composition (for example, about 9% (w/w) or less, about 8% (w/w) or less, about 7% (w/w) or less, about 6% (w/w) or less, about 5% (w/w) or less, about 4% (w/w) or less, about 3% (w/w) or less, about 2% (w/w) or less, about 1.5% (w/w) or less, about 1% (w/w) or less, about 9‰ (w/w) or less, about 8‰ (w/w) or less, about 7‰ (w/w) or less, about 6‰ (w/w) or less, about 5‰ (w/w) or less, about 4‰ (w/w) or less, about 3‰ (w/w) or less, about 2‰ (w/w) or less, about 1.5‰ (w/w) or less, about 1‰ (w/w) or less, about 0.5‰ (w/w) or less, about 0.1‰ (w/w) or less, about 0.01‰ (w/w) or less, about 0.001‰ (w/w) or lower), the quality of the transfection composition is considered to meet the standard.

Therefore, the method of the present application may comprise the following steps: measuring the content of the host genome DNA (for example, nucleic acid molecules or fragments thereof with a size of at least about 10kb (for example, at least about 15kb, at least about 20kb, at least about 30kb, at least about 35kb, at least about 40kb, at least about 48kb, or larger)). And/or, the method of the present application may comprise the following steps: measuring the content of nucleic acid molecules or fragments thereof derived from the host genome (e.g., microorganism).

In the present application, the standard may be a standard for electroporation used in the present application or a standard for transfecting (e.g., electroporation) cells according to the method of the present application.

In the present application, the method of the present application may comprise the following steps: measuring the total content of nucleic acid molecules in the transfection mixture, or measuring the total amount of exogenous nucleic acid molecules derived from the host cell (e.g., plasmid).

In the present application, by measuring the content of host genome DNA, the content of the host genome DNA in the transfection mixture may be clarified before transfection.

If the calculated ratio meets the ratio threshold in the method of the present application (e.g., about 1 (w/w) % or less of the total plasmid amount in the transfection mixture, for example, about 0.9 (w/w) % or less, about 0.8 (w/w) % or less, about 0.7 (w/w) % or less, about 0.6 (w/w) % or less, about 0.5 (w/w) % or less, about 0.4 (w/w) % or less, about 0.3 (w/w) % or less, about 0.2 (w/w) % or less, about 0.1 (w/w) % or less, about 0.09 (w/w) % or less, about 0.08 (w/w) % or lower), the transfection mixture may be considered to have achieved the technical effect of the method of the present application (e.g., improved transfection efficiency) and may be directly subjected to cell transfection. Conversely, if the calculated ratio does not meet the ratio threshold in the method of the present application, the transfection mixture may be considered to have not achieved the technical effect of the method of the present application. In other words, in order to achieve the technical effect of the method of the present application (e.g., improved transfection efficiency), a step of reducing the content of host genome DNA in the transfection mixture may be performed.

In the present application, the measurement may be carried out using methods for measuring the content of nucleic acid molecules. The methods for the measurement may comprise phosphorus determination (i.e., detecting the phosphorus content in ribonucleic acids and/or deoxyribonucleic acids, thereby calculating the content of nucleic acid molecules, for example, using phosphorus determination reagents, such as ammonium molybdate), sugar determination (i.e., detecting the aldehyde compounds formed by pentose in ribose, producing colored compounds, calculating the content of nucleic acid molecules from the concentration of colored compounds by colorimetry or spectrophotometry), ultraviolet absorption (i.e., detecting the absorption value of nucleotide bases in nucleic acids absorbing ultraviolet light to calculate the content of nucleic acid molecules), fluorescence photometry, and/or qPCR (which can obtain the absolute quantification of nucleic acid molecules).

For example, the measurement may use qPCR. In the present application, the *E*. *coli* DNA Quantitation Assay Kit (Cat# 4458435, Thermal Fisher) may be used, and the content of nucleic acid molecules or fragments thereof, with a size of at least about 10 kb (e.g., at least about 15 kb, at least about 20 kb, at least about 30 kb, at least about 35 kb, at least about 40 kb, at least about 48 kb or larger) in the plasmid to be detected, and/or the content of nucleic acid molecules or fragments thereof derived from the host (e.g., microorganism) genome, may be determined according to the methods described in the instruction manual of the kit.

### Transfected cells or cell populations

In another aspect, the present application provides a cell and/or cell line obtained using the methods described in the present application.

In the present application, the cell and/or cell line may have a significantly increased transfection positive ratio (e.g., may also have a significantly increased gene editing knock-in positive ratio (e.g., target cell activation can increase the positive rate of CAR, antibody, cytokine, and/or chemokine knock-in), significantly increased DNA homologous recombination efficiency, and/or significantly increased cell viability). The cell line of the present application may maintain the ability to have a significantly increased transfection positive ratio during the proliferation process of multiple generations (e.g., passaging at least about 5 generations, at least about 10 generations, at least about 15 generations, at least about 20 generations, or longer).

In some embodiments, the transfection positive ratio is greater than about 2%, 3%, 4%, 5%, 6%, 7%, 8%, 9%, 10%, 15%, 20%, 25%, 30%, 35%, 40%, 45%, or 50%. The exogenous gene positive ratio may be measured by determining the number of cells with exogenous gene modification and dividing by the total number of cells. Integration of the exogenous gene into the cellular genomic DNA may be determined by methods known in the art, such as direct genomic DNA sequencing, differential restriction digestion (if gene editing is adding, removing, or changing restriction enzyme sites), gel electrophoresis, array capillary electrophoresis, MALDI-TOF MS, allele-specific oligonucleotide hybridization, molecular beacons, restriction fragment length polymorphism, primer extension, temperature gradient gel electrophoresis, etc.

In other embodiments, cell viability after electroporation is at least 20%, 25%, 30%, 35%, 40%, 45%, 50%, 55%, 60%, 65%, 70%, 75%, 80%, or 85%. Cell viability may be measured by methods known in the art. For example, cell counting may be performed before and after electroporation using a cell counter device. In other embodiments, apoptosis may be measured. The introduction of large amounts of nucleic acids is considered to induce apoptosis. The methods described herein are believed to result in less apoptosis than other methods in the art. In some embodiments, the amount of cells showing apoptosis after electroporation is less than 50%, 45%, 40%, 35%, 30%, 25%, 20%, 15%, 10%, or 5%. Apoptosis refers to a specific process of programmed cell death and may be measured by methods known in the art. For example, apoptosis may be measured by annexin V assay.

In the present application, the cell and/or cell line may simultaneously express the chimeric antigen receptor CAR as described in the present application and the bispecific T cell engager BiTE as described in the present application.

For example, the cell and/or cell line may express a fusion-type CAR, such as CAR-BiTE.

In the present application, the cells and/or cell lines may simultaneously express the chimeric antigen receptor (CAR) described in the present application and at least one cytokine described in the present application.

For example, the cells and/or cell lines may express a fusion-type CAR, such as CAR-cytokine (e.g., CAR-IL15). For example, CAR-cytokine-chemokine (e.g., CAR-IL7-CCL19) may be expressed.

In the present application, the cells and/or cell lines may have significantly increased transfection-positive ratios (e.g., also significantly increased gene editing knock-in positive ratios (e.g., target cell activation may increase the positive rate of CAR knock-in), significantly increased DNA homologous recombination efficiency, and/or significantly increased cell viability). The cell lines described in the present application can maintain the ability of significantly increased transfection-positive ratios during multiple generations of proliferation (e.g., passaging at least about 5 generations, at least about 10 generations, at least about 15 generations, at least about 20 generations, or longer).

### Therapeutic Uses

In some embodiments, the cells and cell lines produced by the methods described herein are cells and cell lines that provide therapeutic effects after editing the genomic DNA of the cells. Primary cells may be isolated and modified by the methods described herein and used *in vitro* for reintroduction into the subject to be treated. Suitable primary cells comprise peripheral blood mononuclear cells (PBMCs), peripheral blood lymphocytes (PBLs), and other blood cell subpopulations (e.g., but not limited to CD4+ T cells or CD8+ T cells). Other suitable primary cells comprise progenitor cells, such as bone marrow or lymphoid progenitors. Suitable cells also comprise stem cells, such as, for example, embryonic stem cells, induced pluripotent stem cells, hematopoietic stem cells, neuronal stem cells, mesenchymal stem cells, muscle stem cells, and skin stem cells. For example, iPSCs may be obtained *in vitro* from patients with known gene mutations associated, and the mutation may be modified using the methods described herein to a wild-type allele. The modified iPSCs may then be differentiated into dopaminergic neurons and reimplanted into the patient. In another *in vitro* therapeutic application, hematopoietic stem cells may be isolated from patients with known gene mutations and then modified to correct the gene mutation. The HSCs may then be administered back to the patient for therapeutic effects or differentiated into more mature hematopoietic cells in culture before administration to the patient.

In some embodiments, the modified genomic DNA sequences and/or donor DNA contain disease-related genes. In some embodiments, the sequence modification region contains disease-related genes. Disease-related genes are known in the art.

In one embodiment, the method comprises modifying the genomic DNA in hematopoietic stem cells (also known as blood-forming cells) or bone marrow progenitor cells.

Another example of therapeutic use of the methods of the present application is site-specific integration of chimeric antigen receptors (CARs). The term "chimeric antigen receptor" or "CAR" refers to such modified receptors that transplant any specificity onto immune effector cells. These receptors are used to transplant the specificity of monoclonal antibodies onto T cells. The receptors are called chimeras because they are composed of parts from different sources. The most common form of these molecules is a fusion protein consisting of a single-chain variable fragment (scFv) derived from a monoclonal antibody fused with the following: CD3ζ transmembrane and endodomain; CD28 or 41BB intracellular domain; or a combination thereof. Such molecules respond to recognition by their target scFv, leading to signal transduction. An example of such a construct is GPC3-CAR, which recognizes GPC3 (Glypican 3) specifically expressed on the surface of hepatocellular carcinoma cells. When T cells express this molecule, they recognize and kill target cells expressing GPC3 (e.g., hepatoma cells). To target malignant B cells, researchers have redirected T-cell specificity using chimeric immune receptors specific for the B-lineage molecule CD19. The variable parts of the immunoglobulin heavy and light chains are fused to form an scFv by a flexible linker. A signal peptide precedes the scFv to guide the initial protein to the endoplasmic reticulum and subsequent surface expression (which is cleaved). The flexible spacer region allows the scFv to orient in different directions for antigen binding. The transmembrane domain is a typical hydrophobic α-helix, usually derived from the original molecule of the signal transduction intracellular domain that penetrates the cell and transmits the desired signal.

Artificial T cell receptors are being investigated as a treatment for cancer, using a technique called adoptive cell transfer. T cells are removed from the patient and modified to express receptors with specificity for a particular form of cancer. The T cells can then recognize and kill cancer cells when reintroduced into the patient. Modification of T cells derived from donors other than the patient is also under investigation.

These modified CAR T cells may be expanded ex *vivo,* and the expanded CAR T cell population may be infused into the patient. After infusion, the T cells proliferate in the patient's body and, under the guidance of their modified receptors, recognize and kill cancer cells expressing the antigen on their surface. Many existing treatment methods involve the introduction of CAR via viral infection. However, there are always safety concerns when using viral infection in treatment methods. Therefore, the methods described herein are non-viral methods for gene therapy and genome engineering. Previously, it was impossible to transfect immune cells with plasmid DNA, as doing so resulted in severe toxicity to the cells. The inventors of the present application have discovered that by treating the transfection composition prior to cell transfection (for example, reducing the content of host genome DNA), the problems affecting cell viability are overcome, and long DNA fragments (and/or high concentrations) may be transfected into cells while maintaining high levels of viability. Using the methods described herein, CAR may be integrated into specific sites of immune cells. In some embodiments, the cells are autologous immune cells. Long-term expression of CAR in T cells or natural killer (NK) cells may be used for leukemia treatment or treatment of tumors associated with certain antigens.

In some aspects, the methods described herein involve improved methods for ex *vivo* therapy. Cell populations may be separated from the subject, and cells may be activated using methods known in the art and/or methods described herein, and the genomic DNA of the cells may be modified in a manner that corrects defects or integrates target genes in a site-specific manner. The cell population can then be transplanted into the subject for therapeutic purposes. In some cases, the separated cell population may contain cell subpopulations sensitive to certain ex *vivo* manipulations (e.g., conventional transfection and/or electroporation methods) or cell subpopulations resistant to conventional transfection and/or electroporation methods or genomic DNA manipulation. It is contemplated that modifying genomic DNA using the methods described herein results in more efficient sequence modification in such populations.

Furthermore, cells and cell lines produced by the methods described herein may be used for drug development and/or reverse genetics research. Such cells and animals may display phenotypes associated with specific mutations or sequence modifications and may be used to screen drugs that interact specifically with the discussed mutations or mutant proteins, or drugs that may be used to treat diseases in affected animals. These cell lines can also provide tools for studying the effects of specific mutations, as the cell lines and their corresponding "modified" cell lines represent "genetically identical" controls, thus providing a powerful tool for repairing disease-specific mutations, drug screening and discovery, and studying disease mechanisms.

The composition of the present disclosure may be administered *in vivo, ex vivo,* or *in vitro.* For example, the compositions of the present disclosure may be used as cancer vaccines. In the present application, the term "ex *vivo* administration" refers to operations performed on cells removed from the subject or outside the subject, comprising but not limited to cells in culture. The term *"in vitro* administration" refers to cells that have been manipulated *ex vivo* and are subsequently administered to the subject. The term *"in vivo* administration" comprises all operations performed within the subject, comprising administration.

The method of the present application also involves a method for immunizing and/or treating certain cancers in a subject, which method comprises: transfecting cells with a transfection composition of the present application comprising a) exogenous nucleic acid molecule and b) gene editing system (i.e., wherein the host, such as a microorganism, genome DNA content is as defined in the present application, e.g., less than about 2(w/w)% of the total DNA amount in the transfection composition); wherein the exogenous nucleic acid molecule comprises: (i) homologous regions comprising nucleic acid sequences homologous to target genomic DNA regions; and (ii) chimeric antigen receptor (CAR) encoding sequences, as well as optionally one or more antibodies (e.g., BiTE) and/or cytokines (e.g., IL15); and wherein the target genomic DNA region specifically modifies genomic DNA sequences to integrate CAR (antibody and/or cytokine) encoding sequences; and administering the cells to a patient. In some embodiments, the immune cells are autologous. In some embodiments, the immune cells have already contacted the antigen. In some embodiments, the antigen is an antigen expressed by the subject's cancer cells. In some embodiments, the antigen is cell-free. The term "cell-free" refers to a composition lacking any cellular components. In some embodiments, the antigen is an extract from a patient's tumor. In some embodiments, the antigen is a polypeptide. In some embodiments, the antigen comprises one or more of tumor cell lysates, apoptotic tumor cells, tumor-associated antigens, and tumor-derived mRNA.

In some embodiments, the immune cells are antigen-presenting cells. Examples of antigen-presenting cells comprise dendritic cells, macrophages, B cells, and tumor cells (pseudo-antigen-presenting cells), in which T cell stimulatory factors (e.g., B7 or 4-1BBL) are forcibly expressed, e.g., by gene transfer. In some embodiments, the antigen-presenting cell is a dendritic cell.

The route of administration for the immune cells can be , for example, intratumoral, intradermal, subcutaneous, intravenous, intralymphatic, and intraperitoneal administration. In some embodiments, administration is intratumoral or intralymphatic. In some embodiments, the immune cells are administered directly to the cancer tissue or lymph node.

In some embodiments, the immune cells are T cells. T cells may be cells that have already contacted the antigen or antigen-presenting cells. For example, APCs may be cultured with tumor antigens specific to a patient's cancer to differentiate them into, for example, CD8-positive cytotoxic T lymphocytes (CTLs) or CD4-positive helper T cells. The established T cells may be administered to an individual with cancer.

The source of the initial T cells is not particularly limited and may be , for example, from peripheral blood of a vertebrate. The initial T cells used may be CD8-positive cells or CD4-positive cells separated from PBMC fractions. In some embodiments, in terms of inducing CTL efficiency, the initial T cells are CD8-positive cells or CD4-positive cells mixed with other cells and components without being separated from PBMC fractions. For example, when cells from PBMC fractions are cultured in a medium supplemented with serum and tumor antigens, the PBMCs differentiate into dendritic cell precursors. The dendritic cell precursors then bind to the peptide and differentiate into dendritic cells as antigen-presenting cells presenting the peptide/tumor antigen. Antigen-presenting cells stimulate CD8-positive T cells in PBMCs to differentiate into CTLs. Thus, CTLs capable of recognizing the added peptide may be obtained. The obtained CTLs may be isolated and used directly as a cancer vaccine. Alternatively, before being used as a cancer vaccine, they may be further cultured in the presence of leukocyte interleukins (e.g., IL-2), antigen-presenting cells, and tumor antigens. Their route of administration is not particularly limited, and examples comprise intradermal, subcutaneous, intravenous, and intratumoral administration.

In an aspect, the present application provides the following technical solutions:
1. A method for increasing transfection efficiency, comprising the step of: making the content of at least about 48 kb size nucleic acid molecules or fragments thereof account for less than about 10% of the total nucleic acid molecule content of the transfection mixture.
2. A method for increasing gene editing efficiency, comprising the step of: making the content of at least about 48 kb size nucleic acid molecules or fragments thereof account for less than about 10% of the total nucleic acid molecule content of the transfection mixture.
3. A method for increasing DNA homologous recombination efficiency, comprising the step of: making the content of at least about 48 kb size nucleic acid molecules or fragments thereof account for less than about 10% of the total nucleic acid molecule content of the transfection mixture.
4. A method for improving cell viability after transfection, comprising the following steps: making the content of nucleic acid molecules or fragments thereof with a size of at least about 48kb to less than about 10% of the total nucleic acid content in the transfection mixture.
5. A method for preparing a transfection mixture, comprising the following steps: making the content of nucleic acid molecules or fragments thereof with a size of at least about 48kb to less than about 10% of the total nucleic acid content in the transfection mixture.
6. A method for determining the quality of a transfection mixture, comprising the following steps: determining whether the content of nucleic acid molecules or fragments thereof with a size of at least about 48kb is less than about 10% of the total nucleic acid content in the transfection mixture.
7. The method according to any one of technical solutions 1-6 of the present aspect, wherein the size of said nucleic acid molecules or fragments thereof is at least about 500kb.
8. The method according to any one of technical solutions 1-7 of the present aspect, wherein the size of said nucleic acid molecules or fragments thereof is at least about 1Mb.
9. The method according to any one of technical solutions 1-8 of the present aspect, wherein the size of said nucleic acid molecules or fragments thereof is at least about 10Mb.
10. The method according to any one of technical solutions 1-9 of the present aspect, wherein said nucleic acid molecules or fragments thereof are derived from microorganisms.
11. The method according to technical solution 10 of the present aspect, wherein the microorganisms are selected from one or more of the following group: bacteria, fungi, actinomycetes, mycoplasmas, chlamydia, rickettsiae, and spirochetes.
12. The method according to any one of technical solutions 10-11 of the present aspect, wherein said microorganisms comprise gram-negative bacteria.
13. The method according to any one of technical solutions 10-12 of the present aspect, wherein said microorganisms comprise *Escherichia coli.*
14. The method according to any one of technical solutions 1-13 of the present aspect, wherein the content of nucleic acid molecules or fragments thereof with a size of at least about 48kb is less than about 2% of the total nucleic acid content in the transfection mixture.
15. The method according to any one of technical solutions 1-14 of the present aspect, wherein the content of nucleic acid molecules or fragments thereof with a size of at least about 48kbis less than about 5‰ of the total nucleic acid content in the transfection mixture.
16. The method according to any one of technical solutions 1-15 of the present aspect, wherein the content of nucleic acid molecules or fragments thereof with a size of at least about 48kb is less than about 1‰ of the total nucleic acid content in the transfection mixture.
17. The method according to any one of technical solutions 1-16 of the present aspect, wherein said transfection comprises transient transfection and/or stable transfection.
18. The method according to any one of technical solutions 1-17 of the present aspect, wherein the transfection comprises electroporation.
19. The method according to any one of technical solutions 1-18 of the present aspect, wherein the transfection comprises transfection of cells.
20. The method according to technical solution 4, or any one of technical solutions 7-19 of the present aspect, wherein the cells comprise eukaryotic cells.
21. The method according to technical solution 4, or any one of technical solutions 7-20 of the present aspect, wherein the cells comprise stem cells, immune cells, fibroblasts, and/or muscle cells.
22. The method according to technical solution 21 of the present aspect, wherein the stem cells comprise pluripotent stem cells.
23. The method according to any one of technical solutions 21-22 of the present aspect, wherein the stem cells comprise hematopoietic stem cells and/or mesenchymal stem cells.
24. The method according to any one of technical solutions 21-23 of the present aspect, wherein the immune cells are selected from the following group: unactivated or activated T lymphocytes, B lymphocytes, NK cells, macrophages, dendritic cells, monocytes, granulocytes, and mast cells.
25. The method according to any one of technical solutions 1-24 of the present aspect, wherein the transfection mixture comprises a plasmid.
26. The method according to technical solution 25 of the present aspect, wherein the plasmid is a circular plasmid, supercoiled plasmid, or linear plasmid.
27. The method according to any one of technical solutions 25-26 of the present aspect, wherein the plasmid is a DNA plasmid.
28. A method according to any one of technical solutions 25-27 of the present aspect, wherein the plasmid comprises a multiple cloning site.
29. A method according to any one of technical solutions 25-28 of the present aspect, wherein the plasmid comprises an exogenous promoter.
30. A method according to any one of technical solutions 25-29 of the present aspect, wherein the at least 48 kb nucleic acid molecule or fragments thereof consist in different nucleic acid molecules from the plasmid.
31. A method according to any one of technical solutions 1-30 of the present aspect, the method comprising the step of: reducing the content of the at least about 48 kb nucleic acid molecule or fragments thereof in the transfection mixture.
32. A method according to the present aspect technical solution 31, wherein the reduction comprises purifying the transfection mixture.
33. A method according to any one of technical solutions 31-32 of the present aspect, wherein the reduction comprises purifying the transfection mixture using a reagent selected from the following group: DNase, SDS, TX-100, CTAB, and cesium chloride-bromide ethidium.
34. A method according to any one of technical solutions 31-33 of the present aspect, wherein the reduction comprises using a method selected from the following group: DNA artificial synthesis of any one of the plasmids described in the technical solutions 25-33 of the present aspect.
35. A method according to any one of technical solutions 2, 7-34 of the present aspect, wherein the gene editing method is one or more selected from the following group: CRISPR/Cas system, RNA editing system ADAR, RNA-guided nucleases, zinc finger nucleases, Mega-TAL nucleases, TALENs, and Meganucleases.
36. A method according to any one of technical solutions 2, 7-35 of the present aspect, wherein the gene editing comprises gene editing knockout and/or gene editing knock-in.
37. A method according to any one of technical solutions 2, 7-36 of the present aspect, wherein the gene editing comprises gene editing knock-in.
38. A method according to any one of technical solutions 25-37 of the present aspect, wherein the plasmid serves as a donor plasmid in the gene editing knock-in.
39. A method according to technical solution 38 of this aspect, wherein the donor plasmid comprises a nucleic acid molecule or fragments thereof of the exogenous gene to be knocked in.
40. A method according to technical solution 39 of this aspect, wherein the exogenous gene comprises a nucleic acid molecule encoding at least one protein selected from the following group: antibodies or antigen-binding fragments, chimeric antigen receptors (CAR), cytokines, and chemokines.
41. A method according to technical solution 40 of this aspect, wherein the antibody or antigen-binding fragment comprises a bispecific antibody or antigen-binding fragment.
42. A method according to any one of technical solutions 40-41 of this aspect, wherein the antibody or antigen-binding fragment comprises a bispecific T-cell engager (BiTE).
43. A method according to any one of technical solutions 38-42 of this aspect, wherein the donor plasmid comprises homology arms that are homologous to the ends of the nucleic acid molecule or fragments thereof of the exogenous gene to be knocked in.
44. A method according to technical solution 43 of this aspect, wherein the homology arm is 100 - 1000 bp in size.
45. A method according to any one of technical solutions 4, 7-44 of this aspect, wherein the cell viability comprises a selection of the following indicators: cell survival rate, cell proliferation ability, cell killing ability, cell secretion ability, cell preservation ability, and cell recovery ability.
46. A method according to any one of technical solutions 1-45 of this aspect, comprising the step of: measuring the content of the at least about 48 kb nucleic acid molecule or fragments thereof.
47. A method according to any one of technical solutions 1-46 of this aspect, comprising the step of: measuring the total content of nucleic acid molecules in the transfection mixture.
48. A cell and/or cell line obtained by a method according to any one of technical solutions 1-47 of this aspect.
49. A transfection mixture obtained by a method according to any one of technical solutions 1-47 of this aspect.
50. A method for improving transfection efficiency, comprising the step of: reducing the content of nucleic acid molecules or fragments thereof originating from a host (e.g., microorganism) genome to less than about 10% of the total nucleic acid content in the transfection mixture.
51. A method for improving the editing efficiency of gene editing, comprising the following step: making the content of nucleic acid molecules or fragments thereof originating from a host (e.g., microorganism) genome account for about 10% or less of the total nucleic acid molecule content of the transfection mixture.
52. A method for improving the efficiency of DNA homologous recombination, comprising the following step: making the content of nucleic acid molecules or fragments thereof originating from a host (e.g., microorganism) genome account for about 10% or less of the total nucleic acid molecule content of the transfection mixture.
53. A method for improving the cell viability of cells after transfection, comprising the following step: making the content of nucleic acid molecules or fragments thereof originating from a host (e.g., microorganism) genome account for about 10% or less of the total nucleic acid molecule content of the transfection mixture.
54. A method for preparing a transfection mixture, comprising the following step: making the content of nucleic acid molecules or fragments thereof originating from a host (e.g., microorganism) genome account for about 10% or less of the total nucleic acid molecule content of the transfection mixture.
55. A method for determining the quality of a transfection mixture, comprising the following step: determining whether the content of nucleic acid molecules or fragments thereof originating from a host (e.g., microorganism) genome accounts for 10% or less of the total nucleic acid molecule content of the transfection mixture.
56. The method according to any one of technical solutions 50-55 of this aspect, wherein the microorganism is selected from one or more of the following group: bacteria, fungi, actinomycetes, mycoplasmas, chlamydia, rickettsia, and spirochetes.
57. The method according to any one of technical solutions 50-56 of this aspect, wherein the microorganism comprises Gram-negative bacteria.
58. The method according to any one of technical solutions 50-57 of this aspect, wherein the microorganism comprises *Escherichia coli.*
59. The method according to any one of technical solutions 50-58 of this aspect, wherein the size of the nucleic acid molecules or fragments thereof originating from a host (e.g., microorganism) genome is at least about 48kb.
60. The method according to any one of technical solutions 50-59 of this aspect, wherein the size of the nucleic acid molecules or fragments thereof originating from a host (e.g., microorganism) genome is at least about 500kb.
61. The method according to any one of technical solutions 50-60 of this aspect, wherein the size of the nucleic acid molecules or fragments thereof originating from a host (e.g., microorganism) genome is at least about 1Mb.
62. The method according to any one of technical solutions 50-61 of this aspect, wherein the size of the nucleic acid molecules or fragments thereof originating from a host (e.g., microorganism) genome is at least about 10Mb.
63. The method according to any one of technical solutions 50-62 of this aspect, wherein the content of nucleic acid molecules or fragments thereof originating from a host (e.g., microorganism) genome accounts for about 2% or less of the total nucleic acid molecule content of the transfection mixture.
64. The method according to any one of technical solutions 50-63 of this aspect, wherein the content of nucleic acid molecules or fragments thereof originating from a host (e.g., microorganism) genome accounts for about 5‰ or less of the total nucleic acid molecule content of the transfection mixture.
65. The method according to any one of technical solutions 50-64 of this aspect, wherein the content of nucleic acid molecules or fragments thereof originating from a host (e.g., microorganism) genome accounts for about 1‰ or less of the total nucleic acid molecule content of the transfection mixture.
66. The method according to any one of technical solutions 50-65 of this aspect, wherein the transfection comprises transient transfection and/or stable transfection.
67. The method according to any one of technical solutions 50-66 of this aspect, wherein the transfection comprises electroporation.
68. The method according to any one of technical solutions 50-67 of this aspect, wherein the transfection comprises transfecting cells.
69. The method according to any one of technical solutions 53, 56-68 of this aspect, wherein the cell comprises eukaryotic cells.
70. The method according to any one of technical solutions 53, 56-69 of this aspect, wherein the cell comprises stem cells, immune cells, fibroblasts, and/or muscle cells.
71. The method according to technical solutions 70 of this aspect, wherein the stem cells comprise pluripotent stem cells.
72. The method according to any one of technical solutions 70-71 of this aspect, wherein the stem cells comprise hematopoietic stem cells and/or mesenchymal stem cells.
73. The method according to any one of technical solutions 70-72 of this aspect, wherein the immune cells are selected from the group consisting of: unactivated or activated T lymphocytes, B lymphocytes, NK cells, macrophages, dendritic cells, monocytes, granulocytes, and mast cells.
74. The method according to any one of technical solutions 50-73 of this aspect, wherein the transfection mixture comprises a plasmid.
75. The method according to technical solutions 74 of this aspect, wherein the plasmid is a circular plasmid, supercoiled plasmid, or linear plasmid.
76. The method according to any one of technical solutions 74-75 of this aspect, wherein the plasmid is a DNA plasmid.
77. The method according to any one of technical solutions 74-76 of this aspect, wherein the plasmid comprises a multiple cloning site.
78. The method according to any one of technical solutions 74-77 of this aspect, wherein the plasmid comprises an exogenous promoter.
79. The method according to any one of technical solutions 50-78 of this aspect, wherein the nucleic acid molecule or fragments thereof originating from the host (e.g., microorganism) genome and the plasmid are present in different nucleic acid molecules.
80. The method according to any one of technical solutions 50-79 of this aspect, comprising the step of: reducing the content of the nucleic acid molecule or fragments thereof originating from the host (e.g., microorganism) genome in the transfection mixture.
81. The method according to technical solutions 80 of this aspect, wherein the reduction comprises purifying the transfection mixture.
82. The method according to any one of technical solutions 80-81 of this aspect, wherein the reduction comprises purifying the transfection mixture using a reagent selected from the group consisting of: DNase, SDS, TX-100, CTAB, and cesium chloride-ethidium bromide.
83. The method according to any one of technical solutions 80-82 of this aspect, wherein the reduction comprises using a method selected from the group consisting of: DNA synthesis to produce the plasmid of any one of aspects 74-82.
84. The method according to any one of technical solutions 51, 56-83 of this aspect, wherein the gene editing method is selected from one or more of the group consisting of: CRISPR/Cas systems, RNA editing system ADAR, RNA-guided endonucleases, zinc finger nucleases, Mega-TAL nucleases, TALENs, and Meganucleases.
85. The method according to any one of technical solutions 51, 56-84 of this aspect, wherein the gene editing comprises gene editing knockout and/or gene editing knock-in.
86. The method according to any one of technical solutions 51, 56-85 of this aspect, wherein the gene editing comprises gene editing knock-in.
87. The method according to any one of technical solutions 74-86 of this aspect, wherein the plasmid acts as a donor plasmid in the gene editing knock-in.
88. The method according to technical solutions 87 of this aspect, wherein the donor plasmid comprises a nucleic acid molecule or fragments thereof of the exogenous gene to be knocked in.
89. The method according to technical solutions 88 of this aspect, wherein the exogenous gene comprises a nucleic acid molecule encoding at least one protein selected from the group consisting of: antibodies or antigen-binding fragments, chimeric antigen receptors (CAR), cytokines, and chemokines.
90. The method according to technical solutions 89 of this aspect, wherein the antibodies or antigen-binding fragments comprise bispecific antibodies or antigen-binding fragments.
91. The method according to any one of technical solutions 89-90 of this aspect, wherein the antibodies or antigen-binding fragments comprise bispecific T-cell engagers (BiTEs).
92. The method according to any one of technical solutions 87-91 of this aspect, wherein the donor plasmid comprises homology arms homologous to the ends of the nucleic acid molecule or fragments thereof of the exogenous gene to be knocked in.
93. The method according to technical solutions 92 of this aspect, wherein the homology arms are 100-1000 bp in size.
94. The method according to any one of technical solutions 53, 56-93 of this aspect, wherein the cell viability comprises indicators selected from the group consisting of: cell survival rate, cell proliferation capacity, cell killing capacity, cell secretion capacity, cell preservation capacity, and cell recovery capacity.
95. According to any one of the methods described in the technical solutions 50-94 of this aspect, the method comprises the following step: measuring the content of nucleic acid molecules or fragments thereof originating from the host (e.g., microorganism) genome.
96. According to any one of the methods described in the technical solutions 50-95 of this aspect, the method comprises the following step: measuring the total content of nucleic acid molecules in the transfection mixture.
97. The cells and/or cell lines obtained by the methods according to any one of technical solutions 50-96 of this aspect.
98. The transfection mixture obtained by the methods according to any one of technical solutions 50-97 of this aspect.

In another aspect, this application provides the following technical solutions:
1. A method for transfecting exogenous genes into cells using plasmids, wherein the exogenous genes comprise nucleic acid molecules encoding chimeric antigen receptors (CAR), and the exogenous genes are integrated into the genome of the transfected cells.
2. The method according to technical solution 1 of this aspect, wherein the size of the exogenous gene is at least about 1000 bp.
3. The method according to any one of technical solutions 1-2 of this aspect, wherein the size of the exogenous gene is at least about 3500 bp.
4. The method according to any one of technical solutions 1-3 of this aspect, wherein the size of the exogenous gene is up to about 10 kb.
5. The method according to any one of technical solutions 1-4 of this aspect, wherein the exogenous gene comprises nucleic acid molecules encoding chimeric antigen receptor (CAR) and/or a second protein that is different from the chimeric antigen receptor.
6. The method according to technical solution 5 of this aspect, wherein the chimeric antigen receptor comprises at least one antigen-binding domain targeting the antigen.
7. The method according to any one of technical solutions 5-6 of this aspect, wherein the second protein is selected from at least one of the following group: cytokines, chemokines, and antibodies or their antigen-binding fragments.
8. The method according to any one of technical solutions 5-7 of this aspect, wherein the second protein comprises a bispecific antibody.
9. The method according to any one of technical solutions 5-8 of this aspect, wherein the second protein comprises a bispecific T cell engager (BiTE).
10. The method according to any one of technical solutions 1-9 of this aspect, wherein the transfection comprises stable transfection.
11. The method according to any one of technical solutions 1-10 of this aspect, wherein the transfection comprises electroporation.
12. The method according to any one of technical solutions 1-11 of this aspect, wherein the transfection comprises gene editing knock-in using gene editing means.
13. The method according to technical solution 12 of this aspect, wherein the gene editing method is selected from one or more of the following group: CRISPR/Cas system, RNA editing system ADAR, RNA-guided nucleases, zinc finger nucleases, Mega-TAL nucleases, TALENs, and Meganucleases.
14. The method according to technical solutions 12-13 of this aspect, wherein the plasmid serves as a donor plasmid in the gene editing knock-in.
15. The method according to any one of technical solutions 1-14 of this aspect, wherein the plasmid comprises a multiple cloning site.
16. The method according to any one of technical solutions 1-15 of this aspect, wherein the plasmid comprises an exogenous promoter.
17. The method according to any one of technical solutions 1-16 of this aspect, wherein the plasmid comprises a homology arm homologous to the terminus of the exogenous gene or fragments thereof.
18. The method according to technical solution 17 of this aspect, wherein the homology arm is in the size range of 100-1000 bp.
19. The method according to any one of technical solutions 1-18 of this aspect, wherein the plasmid comprises a circular plasmid.
20. The method according to any one of technical solutions 1-19 of this aspect, wherein the plasmid is a DNA plasmid.
21. The method according to any one of technical solutions 1-20 of this aspect, wherein the cell to be transfected is a eukaryotic cell.
22. The method according to any one of technical solutions 1-21 of this aspect, wherein the cell to be transfected comprises immune cell, stem cell, immune cell, fibroblast, and/or muscle cell.
23. A method according to technical solutions 22 of this aspect, wherein the immune cell is selected from the group consisting of: unactivated or activated T lymphocytes, B lymphocytes, NK cells, macrophages, dendritic cells, monocytes, granulocytes, and mast cells.
24. A method according to any one of technical solutions 22-23 of this aspect, wherein the stem cell comprises pluripotent stem cells.
25. A method according to any one of technical solutions 22-24 of this aspect, wherein the stem cell comprises hematopoietic stem cells and/or mesenchymal stem cells.
26. A method according to any one of technical solutions 1-25 of this aspect, wherein in the transfection mixture comprising the plasmid, the content of nucleic acid molecules or fragments thereof with a size of at least about 48kb accounts for about 10% or less of the total nucleic acid content of the transfection mixture.
27. A method according to technical solution 26 of this aspect, wherein the content of nucleic acid molecules or fragments thereof with a size of at least about 48kb accounts for about 2% or less of the total nucleic acid content of the transfection mixture.
28. A method according to any one of technical solutions 26-27 of this aspect, wherein the content of nucleic acid molecules or fragments thereof with a size of at least about 48kb accounts for about 5‰ or less of the total nucleic acid content of the transfection mixture.
29. A method according to any one of technical solutions 26-28 of this aspect, wherein the content of nucleic acid molecules or fragments thereof with a size of at least about 48kb accounts for about 1‰ or less of the total nucleic acid content of the transfection mixture.
30. A method according to any one of technical solutions 26-29 of this aspect, wherein the size of the nucleic acid molecules or fragments thereof with a size of at least about 48kb is at least about 1Mb.
31. A method according to any one of technical solutions 26-30 of this aspect, wherein the size of the nucleic acid molecules or fragments thereof with a size of at least about 48kbis at least about 10Mb.
32. A method according to any one of technical solutions 26-31 of this aspect, wherein the nucleic acid molecules or fragments thereof with a size of at least about 48kb are derived from a microorganism.
33. A method according to any one of technical solutions 1-32 of this aspect, wherein in the transfection mixture comprising the plasmid, the content of nucleic acid molecules or fragments thereof derived from the host genome (e.g., microorganisms) accounts for about 10% or less of the total nucleic acid content of the transfection mixture.
34. A method according to any one of technical solutions 1-33 of this aspect, wherein in the transfection mixture comprising the plasmid, the content of nucleic acid molecules or fragments thereof derived from the host genome (e.g., microorganisms) accounts for about 2% or less of the total nucleic acid content of the transfection mixture.
35. A method according to any one of technical solutions 1-34 of this aspect, wherein in the transfection mixture comprising the plasmid, the content of nucleic acid molecules or fragments thereof derived from the host genome (e.g., microorganisms) accounts for about 5‰ or less of the total nucleic acid content of the transfection mixture.
36. A method according to any one of technical solutions 1-35 of this aspect, wherein in the transfection mixture comprising the plasmid, the content of nucleic acid molecules or fragments thereof derived from the host genome (e.g., microorganisms) accounts for about 1‰ or less of the total nucleic acid content of the transfection mixture.
37. A method according to technical solution 36 of this aspect, wherein the size of the nucleic acid molecules or fragments thereof derived from the host genome (e.g., microorganisms) is at least about 48kb.
38. A method according to any one of technical solutions 36-37 of this aspect, wherein the size of the nucleic acid molecules or fragments thereof derived from the host genome (e.g., microorganisms) is at least about 1Mb.
39. A method according to any one of technical solutions 36-38 of this aspect, wherein the size of the nucleic acid molecules or fragments thereof derived from the host genome (e.g., microorganisms) is at least about 10Mb.
40. A method according to any one of technical solutions 36-39 of this aspect, wherein the microorganism is selected from one or more of the group consisting of: bacteria, fungi, actinomycetes, mycoplasmas, chlamydiae, rickettsiae, and spirochetes.
41. A method according to any one of technical solutions 36-40 of this aspect, wherein the microorganism comprises Gram-negative bacteria.
42. A method according to any one of technical solutions 36-41 of this aspect, wherein the microorganism comprises *Escherichia coli.*
43. A cell and/or cell line obtained by the method according to any one of technical solutions 1-42 of this aspect.
44. The cell and/or cell line according to technical solution 43 of this aspect, which simultaneously expresses CAR and BiTE.
45. The cell and/or cell line according to any one of technical solutions 43-44 of this aspect, which simultaneously expresses CAR and at least one cytokine.
46. Application of reducing the content of nucleic acid molecules or fragments thereof of at least about 48kb size as a proportion of the total nucleic acid molecule content of the transfection mixture comprising the plasmid, in enhancing the transfection efficiency of cells transfected with the plasmid containing the exogenous gene encoding the chimeric CAR.
47. Application of reducing the content of nucleic acid molecules or fragments thereof from the host (e.g., microorganism) genome as a proportion of the total nucleic acid molecule content of the transfection mixture comprising the plasmid, in enhancing the transfection efficiency of cells transfected with the plasmid containing the exogenous gene encoding the chimeric CAR.
48. The application according to any one of technical solutions 46-47 of this aspect, wherein the chimeric CAR is selected from the group consisting of: CAR-BiTE, CAR-cytokine, and CAR comprising at least two different antigen-binding domains targeting different antigens.

In another aspect, this application provides the following technical solutions:
1. A method for increasing plasmid transfection efficiency in cells, comprising the step of: transfecting cells with a plasmid containing an exogenous gene treated with deoxyribonuclease (DNase).
2. The method according to technical solution 1 of this aspect, wherein the transfection comprises transient transfection and/or stable transfection.
3. The method according to any one of technical solutions 1-2 of this aspect, wherein the transfection comprises electroporation.
4. The method according to any one of technical solutions 1-3 of this aspect, wherein the exogenous gene is transiently transfected into the cell.
5. The method according to any one of technical solutions 1-4 of this aspect, wherein after the transient transfection, the cell expresses the protein encoded by the exogenous gene.
6. The method according to any one of technical solutions 2-5 of this aspect, wherein the stable transfection comprises using a transposon system and/or a gene-editing method.
7. The method according to any one of technical solutions 2-6 of this aspect, wherein after the stable transfection, the exogenous gene is integrated into the genome of the cell.
8. The method according to any one of technical solutions 2-7 of this aspect, wherein the stable transfection comprises using the Sleeping beauty transposon system.
9. The method according to any one of technical solutions 2-8 of this aspect, wherein the stable transfection method is selected from one or more of the following group: CRISPR/Cas system, RNA editing system ADAR, RNA-guided endonucleases, zinc finger nucleases, Mega-TAL nucleases, TALENs, and Meganucleases.
10. The method according to any one of technical solutions 2-9 of this aspect, wherein the stable transfection comprises gene editing knock-in.
11. The method according to technical solution 10 of this aspect, wherein the plasmid serves as a donor plasmid in the gene editing knock-in.
12. The method according to any one of technical solutions 1-11 of this aspect, wherein the DNase is capable of cleaving single-stranded DNA and/or double-stranded DNA.
13. The method according to any one of technical solutions 1-12 of this aspect, wherein the DNase is capable of non-specifically cleaving linear DNA.
14. The method according to any one of technical solutions 1-13 of this aspect, wherein the DNase is capable of cleaving linear double-stranded DNA.
15. The method according to any one of technical solutions 1-14 of this aspect, wherein the treatment with DNase comprises the following step: contacting the DNase with the plasmid.
16. The method according to technical solution 15 of this aspect, wherein the contact comprises contacting in a buffered environment containing Mg²⁺ and Ca²⁺.
17. The method according to any one of technical solutions 1-16 of this aspect, wherein the treatment with DNase comprises the following step: purifying the plasmid after the contact.
18. The method according to any one of technical solutions 1-17 of this aspect, wherein the cell comprises a eukaryotic cell.
19. The method according to any one of technical solutions 1-18 of this aspect, wherein the cell to be transfected comprises stem cells, immune cells, fibroblasts, and/or muscle cells.
20. The method according to technical solution 19 of this aspect, wherein the stem cell comprises pluripotent stem cells.
21. The method according to any one of technical solutions 19-20 of this aspect, wherein the stem cell comprises hematopoietic stem cells and/or mesenchymal stem cells.
22. The method according to any one of technical solutions 19-21 of this aspect, wherein the immune cell is selected from the following group: unactivated or activated T lymphocytes, B lymphocytes, NK cells, macrophages, dendritic cells, monocytes, granulocytes, and mast cells.
23. The method according to any one of technical solutions 1-22 of this aspect, wherein the plasmid comprises a circular plasmid.
24. The method according to any one of technical solutions 1-23 of this aspect, wherein the plasmid is a DNA plasmid.
25. The method according to any one of technical solutions 1-24 of this aspect, wherein the plasmid contains a polylinker site.
26. The method according to any one of technical solutions 1-25 of this aspect, wherein the plasmid contains an exogenous promoter.
27. The method according to any one of technical solutions 1-26 of this aspect, wherein the plasmid contains homology arms that are homologous to the termini of the exogenous gene or fragments thereof.
28. The method according to technical solution 27 of this aspect, wherein the homology arms are 100-1000bp in size.
29. The method according to any one of technical solutions 1-28 of this aspect, wherein the exogenous gene comprises a nucleic acid molecule encoding a chimeric antigen receptor (CAR).
30. A method according to any one of technical solutions 1-29 in this aspect, wherein the exogenous gene comprises nucleic acid molecules encoding a chimeric antigen receptor (CAR) and/or a second protein, the second protein being different from the chimeric antigen receptor.
31. The method according to technical solution 30 in this aspect, wherein the second protein is selected from at least one of the following group: cytokines, chemokines, and antibodies, or antigen-binding fragments thereof.
32. The method according to any one of technical solutions 30-31 in this aspect, wherein the second protein comprises a bispecific antibody.
33. The method according to any one of technical solutions 30-32 in this aspect, wherein the second protein comprises a bispecific T cell engager (BiTE).
34. The method according to any one of technical solutions 1-33 in this aspect, wherein the size of the exogenous gene is at least about 1,000 bp.
35. The method according to any one of technical solutions 1-34 in this aspect, wherein the size of the exogenous gene is at least about 3,500 bp.
36. The method according to any one of technical solutions 1-35 in this aspect, wherein the size of the exogenous gene is at least about 5,000 bp.
37. The method according to any one of technical solutions 1-36 in this aspect, wherein the size of the exogenous gene is at most about 10 Kb.
38. The method according to any one of technical solutions 1-37 in this aspect, wherein after the DNase treatment, in the transfection mixture containing the plasmid, the content of nucleic acid molecules or fragments thereof with a size of at least about 48 kb accounts for about 10% or less of the total nucleic acid content of the transfection mixture.
39. The method according to technical solution 38 in this aspect, wherein the content of nucleic acid molecules or fragments thereof with a size of at least about 48 kb accounts for about 2% or less of the total nucleic acid content of the transfection mixture.
40. The method according to any one of technical solutions 38-39 in this aspect, wherein the content of nucleic acid molecules or fragments thereof with a size of at least about 48 kb accounts for about 5‰ or less of the total nucleic acid content of the transfection mixture.
41. The method according to any one of technical solutions 38-40 in this aspect, wherein the content of nucleic acid molecules or fragments thereof with a size of at least about 48 kb accounts for about 1‰ or less of the total nucleic acid content of the transfection mixture.
42. The method according to any one of technical solutions 38-41 in this aspect, wherein the size of nucleic acid molecules or fragments thereof of at least about 48 kb is at least about 1 Mb.
43. The method according to any one of technical solutions 38-42 in this aspect, wherein the size of nucleic acid molecules or fragments thereof of at least about 48 kb is at least about 10 Mb.
44. The method according to any one of technical solutions 38-43 in this aspect, wherein the nucleic acid molecules or fragments thereof with a size of at least about 48 kb are derived from a microorganism.
45. The method according to any one of technical solutions 1-44 in this aspect, wherein after the DNase treatment, in the transfection mixture containing the plasmid, the content of nucleic acid molecules or fragments thereof originating from the host (e.g., microorganism) genome accounts for about 10% or less of the total nucleic acid content of the transfection mixture.
46. The method according to technical solution 45 in this aspect, wherein the content of nucleic acid molecules or fragments thereof originating from the host (e.g., microorganism) genome accounts for about 2% or less of the total nucleic acid content of the transfection mixture.
47. The method according to any one of technical solutions 45-46 in this aspect, wherein the content of nucleic acid molecules or fragments thereof originating from the host (e.g., microorganism) genome accounts for about 5‰ or less of the total nucleic acid content of the transfection mixture.
48. The method according to any one of technical solutions 45-47 in this aspect, wherein the content of nucleic acid molecules or fragments thereof originating from the host (e.g., microorganism) genome accounts for about 1‰ or less of the total nucleic acid content of the transfection mixture.
49. A method according to any one of technical solutions 45-48 of this aspect, wherein the size of the nucleic acid molecule or fragment thereof derived from the host (e.g., microorganism) genome is at least about 48kb.
50. A method according to any one of technical solutions 45-49 of this aspect, wherein the size of the nucleic acid molecule or fragment thereof derived from the host (e.g., microorganism) genome is at least about 1Mb.
51. A method according to any one of technical solutions 45-50 of this aspect, wherein the size of the nucleic acid molecule or fragment thereof derived from the host (e.g., microorganism) genome is at least about 10Mb.
52. A method according to any one of technical solutions 45-51 of this aspect, wherein the microorganism is selected from one or more of the following group: bacteria, fungi, actinomycetes, mycoplasma, chlamydia, rickettsia, and spirochetes.
53. A method according to any one of technical solutions 45-52 of this aspect, wherein the microorganism comprises Gram-negative bacteria.
54. A method according to any one of technical solutions 45-53 of this aspect, wherein the microorganism comprises *Escherichia coli.*
55. Cell(s) and/or cell line(s) obtained by the method according to any one of technical solutions 1-54.
56. Application of deoxyribonuclease (DNase) in the treatment of plasmids to make the plasmids suitable for transient transfection of cells.
57. A reagent kit for improving plasmid transfection efficiency, comprising deoxyribonuclease (DNase) and reagents and/or instruments required for transient transfection.
58. The reagent kit according to technical solution 57 of this aspect, wherein the reagents required for transient transfection comprise reagents required for electroporation.
59. The reagent kit according to technical solution 58 of this aspect, wherein the reagents required for electroporation comprise ddH₂O and/or glycerol.
60. The reagent kit according to any one of technical solutions 57-59 of this aspect, wherein the DNase can cleave single-stranded DNA and/or double-stranded DNA.
61. The reagent kit according to any one of technical solutions 57-60 of this aspect, wherein the DNase can nonspecifically cleave linear DNA.
62. The reagent kit according to any one of technical solutions 57-61 of this aspect, wherein the DNase comprises a nucleic acid exonuclease.
63. The reagent kit according to any one of technical solutions 57-62 of this aspect, comprising a buffer solution containing Mg²⁺ and Ca²⁺.

Without being limited by any theory, the following examples are provided merely to illustrate various technical solutions of this application and should not be construed as limiting the scope of this invention.

### Examples

### Example 1: Detection of the content of host genome DNA in plasmids

Quantitative PCR (qPCR) was used to measure the nucleic acid molecules or fragment thereof derived from the host (e.g., microorganism) genome (mainly Escherichia coli genome DNA) (i.e., host genome DNA) in commercially available plasmids (referred to as plasmids from source A and source B according to different purchase sources).

In addition, various parameters (e.g., open-circular, supercoiled, multimeric, endotoxin content) of plasmids from source A and source B were analyzed, with the results of open-circular, supercoiled, and multimeric analyses obtained by HPLC detection provided by Sino Biological Inc., and endotoxin content obtained by detection provided by the same company.

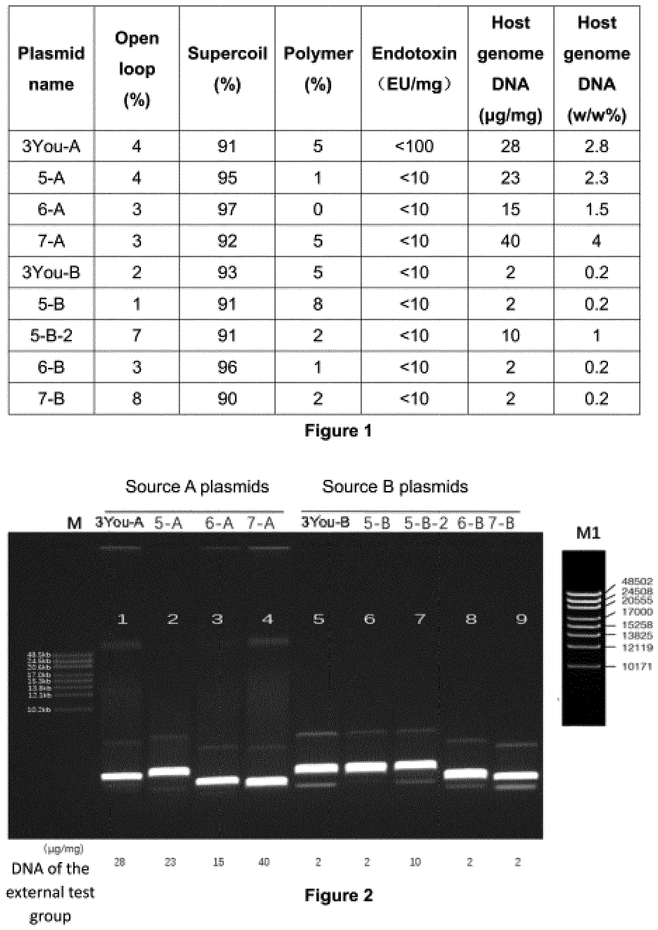

The results are shown in Figure 1. The results in Figure 1 show that there are no significant differences in the open-circular, supercoiled, multimeric, and endotoxin content of these plasmids, but there are significant differences in the content of host (e.g., microorganism) genome DNA in these plasmids.

Furthermore, gel electrophoresis analysis was performed on the DNA of plasmids from source A and source B. Plasmids 3 You-A, 5-A, 6-A, and 7A are from source A, while plasmids 3 You-B, 5-B, 5B-2, 6B, and 7B are from source B.

The results are shown in Figure 2, in which M represents the electrophoresis of the marker; 1-9 represent the electrophoresis results of different plasmids (each lane contains 100 ng of plasmid DNA). M1 displays the specific sizes of the fragments in the marker.

The results of Figure 2 indicate that the plasmids from source B comprise less host genomic DNA and have fewer bands larger than approximately 48.5 kb.

The description of the plasmids used in the present application is shown in the following table.

| **Plasmid name** | **Plasmid backbone** | **Encoded exogenous protein** | **5'homology arms** | **3'homology arms** |
|---|---|---|---|---|
| 3 You-A | pUC57-mini | CD19 CAR(SEQ ID NO: 9) | SEQ ID NO: 4 (TRAC) | SEQ ID NO: 5 (TRAC) |
| 5-A | pUC57-mini | GFP(SEQ ID NO: 1) | SEQ ID NO: 2 (TRAC) | SEQ ID NO: 3 (TRAC) |
| 6-A | pUC57-mini | GFP(SEQ ID NO: 1) | SEQ ID NO: 4 (TRAC) | SEQ ID NO: 5 (TRAC) |
| 7-A | pUC57-mini | GFP(SEQ ID NO: 1) | SEQ ID NO: 6 (TRAC) | SEQ ID NO: 7 (TRAC) |
| 3 You-B | pUC57-mini | CD19 CAR(SEQ ID NO: 9) | SEQ ID NO: 4 (TRAC) | SEQ ID NO: 5 (TRAC) |
| 5-B | pUC57-mini | GFP(SEQ ID NO: 1) | SEQ ID NO: 2 (TRAC) | SEQ ID NO: 3 (TRAC) |
| 5-B-2 | pUC57-mini | GFP(SEQ ID NO: 1) | SEQ ID NO: 2 (TRAC) | SEQ ID NO: 3 (TRAC) |
| 6-B | pUC57-mini | GFP(SEQ ID NO: 1) | SEQ ID NO: 4 (TRAC) | SEQ ID NO: 5 (TRAC) |
| 7-B | pUC57-mini | GFP(SEQ ID NO: 1) | SEQ ID NO: 6 (TRAC) | SEQ ID NO: 7 (TRAC) |
| 12-B | pUC57-mini | GPC3 CAR(SEQ ID NO: 11) | SEQ ID NO: 4 (TRAC) | SEQ ID NO: 5 (TRAC) |
| 19-B | pUC57-mini | CD19 CAR-2A-IL15(SEQ ID NO: 13) | SEQ ID NO: 4 (TRAC) | SEQ ID NO: 5 (TRAC) |
| 33-B | pUC57-mini | GPC3 CAR-2A-IL15(SEQ ID NO: 16) | SEQ ID NO: 4 (TRAC) | SEQ ID NO: 5 (TRAC) |
| 32-B | pUC57-mini | GPC3 CAR-2A-IL7-2A-CCL19(SEQ ID NO: 17) | SEQ ID NO: 4 (TRAC) | SEQ ID NO: 5 (TRAC) |
| 17-B | pUC57-mini | CD19 CAR-2A-BiTE(SEQ ID NO: 15) | SEQ ID NO: 4 (TRAC) | SEQ ID NO: 5 (TRAC) |
| 9-B | pUC57-mini | BiTE(SEQ ID NO: 14) | SEQ ID NO: 19 (AAVS-I) | SEQ ID NO: 20 (AAVS-I) |
| 11-A | pUC57-mini | GFP(SEQ ID NO: 1) | N.A. | N.A. |

In the CD19 CAR, the amino acid sequence of the scFV targeting CD19 is shown in SEQ ID NO: 10.

In the GPC3 CAR, the amino acid sequence of the scFV targeting GPC3 is shown in SEQ ID NO: 12.

### Example 2 Plasmids with less host genomic DNA content have higher cell transfection efficiency

### 2.1 Expression of GFP

Plasmids 5-A (from source A), 5-B (from source B, industrial grade), and 5-B-2 (laboratory grade) were taken. A chemically synthesized sgRNA targeting the human TRAC gene was designed (5'TCAGGGTTCTGGATATCTGT (SEQ ID NO: 8, with 3 sulfur and oxygen methylation modifications at both the 5' and 3' ends of the sgRNA, purchased from GenScript Biotech, Nanjing)). The Cas9 protein was purchased from Sino Biological Inc. (Catalog number: 40572-A08B). The sgRNA and Cas9 protein were prepared as ribonucleoprotein RNP for subsequent cell modification.

By electroporation, 5-A, 5-B, and 5-B-2 were separately introduced into human T cells activated for two days with Dynabead (from ThermoFisher, comprising anti-CD3 and anti-CD28 antibodies), and the prepared RNP was also introduced into the cells. The nucleic acid molecule encoding GFP was integrated into the human T cell TRAC gene and could be expressed through the endogenous promoter of the TRAC gene.

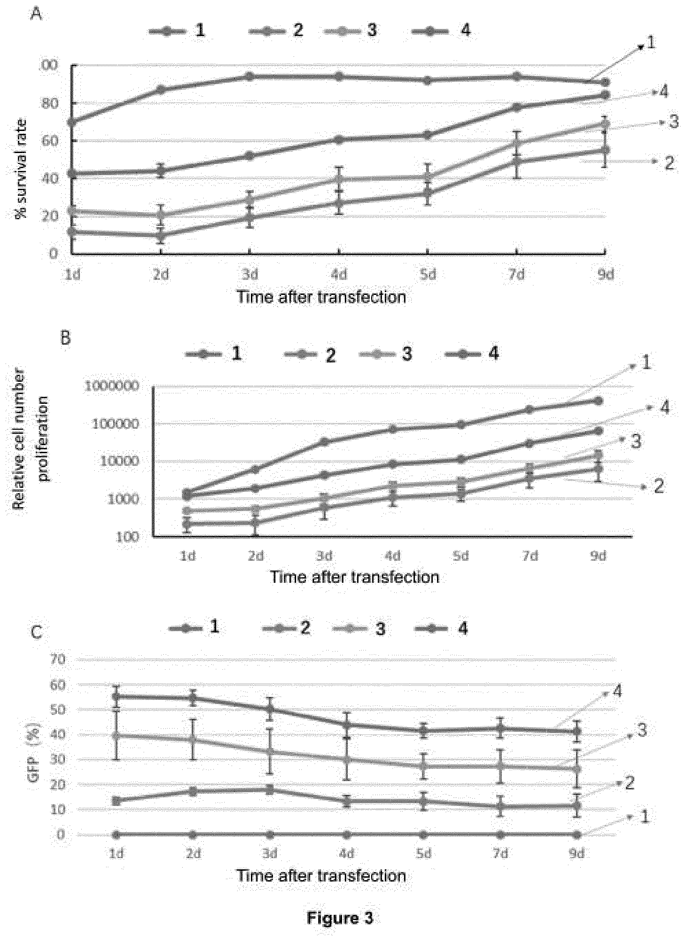

The survival rate and cell proliferation of T cells transfected with plasmids 5-A, 5-B, or 5-B-2 were detected by FACS, and the expression level of GFP in the transfected T cells was also detected. The results are shown in Figures 3A-3C. The cell proliferation was determined by counting the number of cells detected by FACS for 20 seconds; cell survival data were obtained from the PI-negative FSC-SSC gated cell population verified by propidium iodide (PI) staining. The gating of GFP-positive cells was obtained with a control group live cell positivity rate of ≤1%.

In Figure 3, 1-4 show the conditions of the control group (1), 5-A (2), 5-B-2 (3), and 5-B (4), respectively. The results of Figure 3A indicate that, compared to plasmid 5-A containing more nucleic acid molecules or fragments thereof from the host (e.g., microorganisms), the survival rate of T cells transfected with plasmids 5-B or 5-B-2 is significantly increased, with lower host genomic DNA content corresponding to higher survival rates of transfected T cells. The results of Figure 3B indicate that, compared to plasmid 5-A containing more host genomic DNA, the proliferation capacity of T cells transfected with plasmids 5-B or 5-B-2 is significantly increased, with lower host genomic DNA content corresponding to stronger proliferation capacity of transfected T cells. The results of Figure 3C indicate that, compared to plasmid 5-A containing more host genomic DNA, the expression level of GFP in T cells transfected with plasmids 5-B or 5-B-2 is significantly increased, with lower host genomic DNA content corresponding to higher GFP expression levels in transfected T cells.

### 2.2 Expression of GFP or CAR

According to the method described in Example 2.1, plasmids 3 You-A, 5-A, 6-A, 7-A, 3 You-B, 5-B, 5-B-2, 6-B, or 7-B were electroporated into activated T cells, obtaining a total of 25 types of T cells transfected and integrated with exogenous genes.

Calculate the relative overall expression level of the knocked-in protein in these T cells, where the relative overall expression level = post-transfection T cell survival rate (%) * post-transfection T cell expression rate of knocked-in protein (%). The post-transfection T cell survival rate is the cell survival rate on day 1 after transfection; the post-transfection T cell expression rate of the knocked-in protein is the expression rate on day 4 after transfection. The post-transfection T cell expression rate of GFP is obtained by FACS detection; the post-transfection T cell expression rate of CD19 CAR is obtained by CD19 antibody detection (Cat#102, BioSwan Lab, Shanghai).

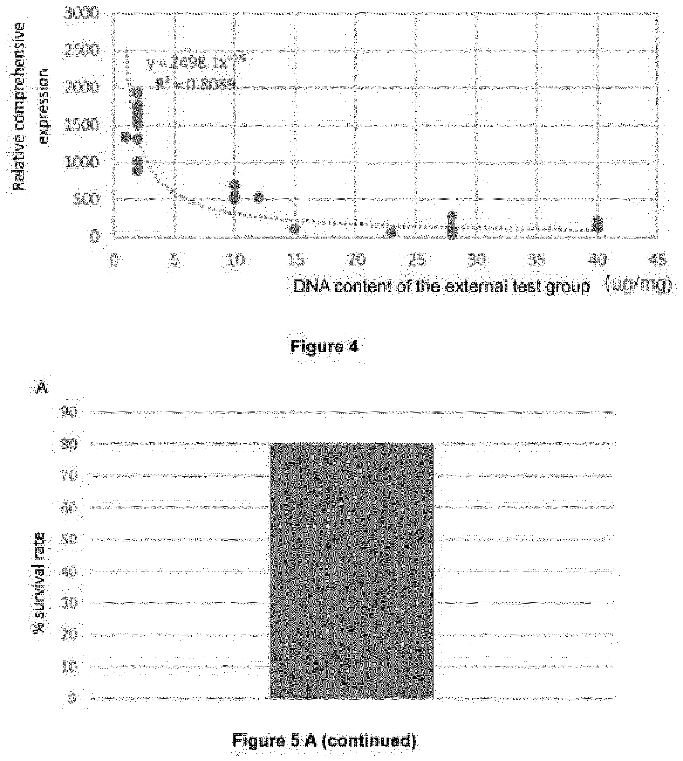

Results are shown in Figure 4, where the curves represent the fitting curves of the relative overall expression level data relative to the host genome DNA content. The results show that the lower the content of host genome DNA in plasmid, the higher the cell survival rate and/or expression rate of the knocked-in protein in T cells after transfection.

### 2.3 CAR expression

A chemically synthesized sgRNA targeting the human TRAC gene (SEQ ID NO: 8, purchased from GenScript Biotech, Nanjing) is designed, with three phosphorothioate and methoxy modifications at both the 5' and 3' ends. The purchased Cas9 protein is sourced from Sino Biological Inc. (Product No. 40572-A08B).

The prepared plasmid 3 You-B, sgRNA, and Cas9 (prepared as RNP system, Cas9:sgRNA at a weight ratio of approximately 3:1) are mixed with human T cells activated by Dynabead for 2-3 days by electroporation. The nucleic acid molecule encoding the CD19 CAR is integrated into the human TRAC gene and may be expressed through the endogenous promoter of the TRAC gene.

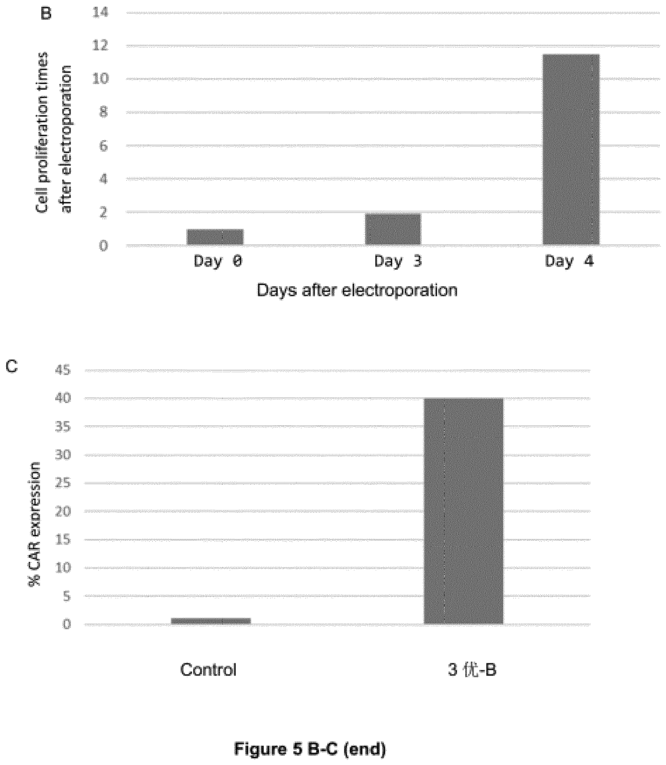

Calculate the cell survival rate and cell proliferation of T cells after transfection according to the method described in Example 2.1, and detect the expression level of CD19 CAR in T cells after transfection. Results are shown in Figures 5A-5C. The cell proliferation is obtained by counting the number of cells detected by FACS for 20 seconds; the data for cell survival rate is obtained by propidium iodide (PI) validation for PI-negative FSC-SSC gated cell population. The results are verified by the cell counter NC-200. GFP-positive cell gating is obtained by a positive rate of control group live cells ≤1%. The control in Figure 5C is T cells without any plasmid transfection.

Figure 5A shows the situation on day 5 after electroporation, and the results of Figure 5A indicate that the T cell viability of cells transfected with plasmids containing less host genome DNA (e.g., plasmid 3 You-B) is excellent, exceeding about 80%. Figure 5B shows the situation on days 0-5 after electroporation, and the results of Figure 5B indicate that T cells transfected with plasmids containing less host genome DNA have excellent proliferation capacity. Figure 5C shows the situation on day 2 after electroporation, and the results of Figure 5C indicate that T cells transfected with plasmids containing less host genome DNA have excellent expression levels of exogenous proteins (i.e., high positive rate of exogenous protein expression). It is indicated that the lower the content of host genome DNA in the plasmid, the further promotion of cell survival rate, cell proliferation capacity, and the expression level of exogenous proteins integrated into the T cell genome after transfection.

### Example 3 Tumor cells can be specifically killed by immune effector cells after transfection with plasmids containing less host genome DNA

### 3.1 In vitro specific killing of tumor cells

A chemically synthesized sgRNA targeting the human TRAC gene (SEQ ID NO: 8, purchased from GenScript Biotech, Nanjing) is designed, with three phosphorothioate and methoxy modifications at both the 5' and 3' ends. The purchased Cas9 protein is sourced from Sino Biological Inc. (Product No. 40572-A08B).

The aforementioned plasmid 3You-B, sgRNA, and Cas9 (prepared as RNP system, Cas9:sgRNA at a weight ratio of approximately 3:1) are mixed with human T cells activated by Dynabead for 2-3 days by electroporation to obtain 3You-B CART cells.

The 3-optimized-B CART cells were co-cultured with Calcein-AM labeled target cells Nalm6 (Clone G5, CRL3273, purchased from ATCC) (human B-cell acute lymphoblastic leukemia cells). After co-cultivation, the mortality of the target cells killed by the CART cells was calculated. Mortality = N_{D} / N_{L} * 100%. Wherein N_{L} is the number of target cells collected during 20s of FACS detection, and N_{D} is the number of dead target cells among them.

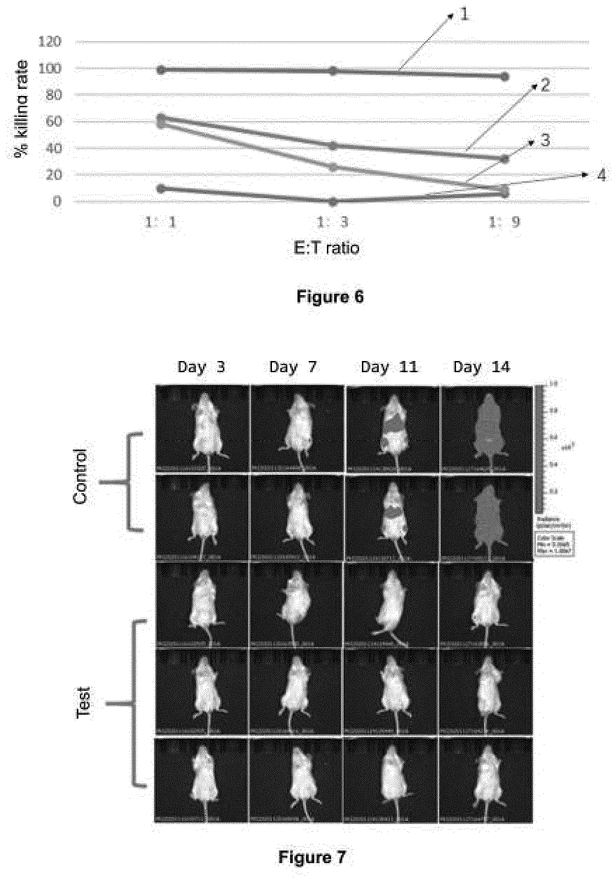

The mortality results are shown in Figure 6. Figure 6 shows the mortality results of target cells Nalm6, with the horizontal axis E:T representing the ratio of effector cells (CART cells) to target cells (tumor cells). 1-4 represent 34 hours of 3 You-B CART cell killing, 10 hours of 3 You-B CART cell killing, 34 hours of control T cell killing, and 10 hours of control T cell killing, respectively. The control is T cells that have not undergone any plasmid transfection.

The results of Figure 6 show that, under the condition of low host genomic DNA content, the T cells transfected with this plasmid have good tumor cell killing ability.

### 3.2 In vivo specific killing of tumor cells

Nalm-6-luc cells (purchased from PharmaLegacy, Shanghai) were injected intravenously into NCG mice (purchased from Gempharmatech Co., Ltd) at a concentration of 1×10⁶/ml to establish a mouse model of B-cell acute lymphoblastic leukemia.

Four days later, 3 mice in the mouse model were injected with 100µl of the 3 You-B CART cells and PBMC cells from the same cell donor described in Example 3.1 at a concentration of 1×10⁸/mL via the tail vein, and these mice were referred to as the experimental group. The other 2 mice in the mouse model were only injected with 100µl of the PBMC cells at a concentration of 1×10⁸/mL via the tail vein without receiving any other treatment and served as the control group.

On the 3rd, 7th, 11th, or 14th day after intravenous injection of the CART cells, the in vivo tumor conditions of the experimental group mice and control group mice were detected by fluorescence (Lumina XRMS, PerkinElmer). The results are shown in Figure 7.

The results of Figure 7 show that the tumors in the control group grow rapidly, while the tumors in the experimental group almost disappear (no detectable fluorescence value). It shows that under the condition of low host genomic DNA content, the CART cells after plasmid transfection have a significant killing effect on tumors in mice.

### Example 4 T cells transfected with plasmids containing less host genomic DNA can specifically kill tumor cells

A chemically synthesized sgRNA targeting the human TRAC gene (SEQ ID NO: 8, purchased from GenScript Biotech, Nanjing) was designed, with 3 thiols and 3 O-methyl modifications at both the 5' and 3' ends. The purchased Cas9 protein is sourced from Sino Biological (Product Code: 40572-A08B).

The plasmid 19-B was mixed with sgRNA and Cas9 (prepared as an RNP system, with a Cas9:sgRNA weight ratio of about 3:1), and human T cells activated by Dynabead for 2-3 days were electroporated. The nucleic acid molecule encoding CD19 CAR-2A-IL15 was knocked into the human TRAC gene and can be expressed through the endogenous promoter of the TRAC gene. The obtained cells are 19-B T cells.

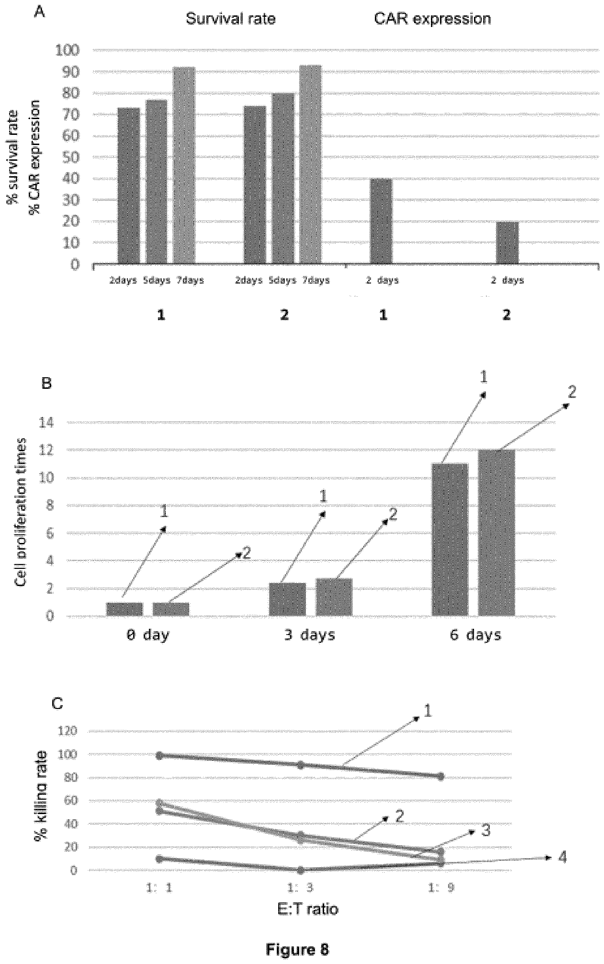

According to the method in Example 2.1, the cell survival rate, CAR expression level, and cell proliferation of T cells after electroporation with plasmid 19-B are calculated, while the death rate of Nalm6 target cells transfected with plasmid 19-B is calculated according to the method of Example 3. The results are shown in Figures 8A-8C. The cell proliferation is obtained by counting the cell number detected by FACS for 20 seconds; the cell survival rate data is obtained by counting the cell population in the FSC-SSC gate that is propidium iodide (PI) negative. The gate for CAR-positive cells is obtained by setting the positive rate of live cells in the control group at ≤1%.

Figure 8A shows the situation on day 2, day 5, and day 7 after electroporation, wherein group 1 T cells are transfected with plasmid 3You-B and group 2 T cells are transfected with plasmid 19-B.

The results of Figure 8A indicate that the survival rate of T cells after transfection with plasmid 19-B is similar to that of plasmid 3You-B, both of which are excellent and exceed about 90% on day 7 after electroporation. At the same time, plasmid 19-B can effectively express CD19 CAR after transfection, but the expression level is slightly lower than that of plasmid 3You-B.

Figure 8B shows the situation on day 0, day 3, and day 6 after electroporation. The results of Figure 8B indicate that after transfection with plasmids containing less host genomic DNA, T cells have excellent proliferation ability. Whether it is plasmid 19-B or plasmid 3You-B, the proliferation multiple of T cells after transfection can reach more than 10 times.

Figure 8C shows the death rate of Nalm6 target cells on day 2 after electroporation. The horizontal axis E:T represents the ratio of effector cells (CART cells) to target cells (tumor cells). In Figure 8C, 1-4 represent the killing of 19-B T cells for 34 hours, 19-B T cells for 10 hours, control T cells for 34 hours, and control T cells for 10 hours, respectively. The control T cells are T cells that have not been transfected with plasmids. The results of Figure 8C show that CART cells obtained by transfection with the bispecific CAR molecules (e.g., CD19 CAR-IL-15) described in the present application have the ability to effectively kill tumor cells.

### Example 5 Tumor cell-specific killing by T cells transfected with plasmids containing less host genomic DNA

### (1) Detection of cell survival rate, cell proliferation ability, cell preservation ability, and cell recovery ability

A chemically synthesized sgRNA targeting the human TRAC gene (SEQ ID NO: 8, purchased from GeneScript Biotech, Nanjing) is designed, with 3 thioate and O-methyl modifications at both the 5' and 3' ends. The Cas9 protein is purchased from Sino Biological (catalog number: 40572-A08B).

The plasmid 17-B, sgRNA, and Cas9 (prepared as an RNP system, with a Cas9:sgRNA weight ratio of approximately 3:1) are mixed with human T cells activated by Dynabeads for 2-3 days, and the nucleic acid molecule encoding CD19 CAR-2A-BiTE is knocked into the human TRAC gene and can be expressed by the endogenous promoter of the TRAC gene. 17-B T cells are obtained.

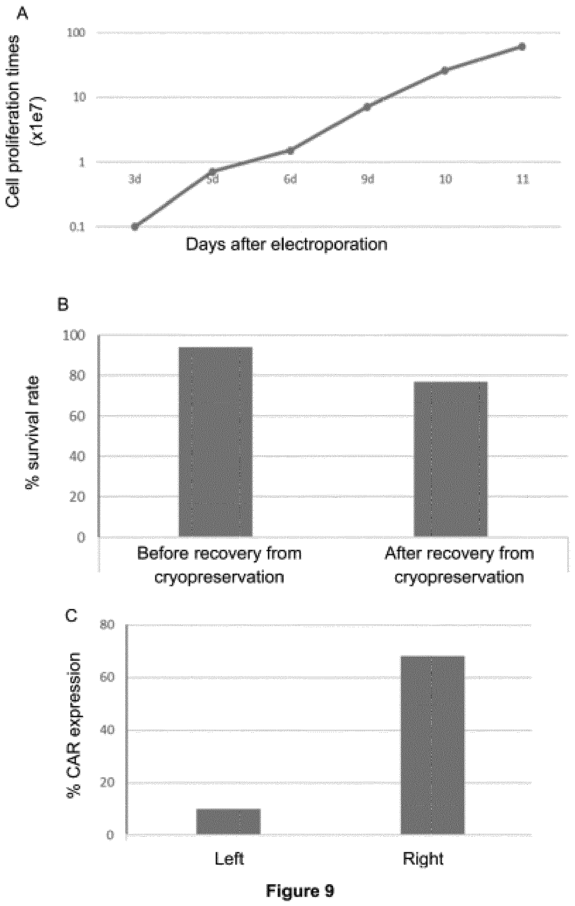

The results are shown in Figures 9A-9C. Figure 9A shows the situation on day 6 after electroporation, where 17-B T cells and Nalm6 cells are co-cultured in a culture medium containing X vivo-15, 5% AB serum, 100 U/ml IL-2, 10 ng/mL IL-7, and 5 ng/mL IL-15 under an effector-target cell ratio (17-B T cells:Nalm6 cells) of E:T = 2:1. The results of Figure 9A show that 17-B T cells can natural proliferate significantly within 6 days after electroporation and can further proliferate significantly when supplemented with target cell activation on the 6th day.

Figure 9B shows the cell viability of 17-B T cells before and after cryopreservation and recovery. The 17-B T cells were cryopreserved with CryoStor CS10 after 11 days post electroporation. Approximately 1 hour after cryopreservation recovery, the cell viability was measured using NC-200. The results show that 17-B T cells have high cell viability after cryopreservation recovery.

Figure 9C shows the expression level of CD19 CAR in 17-B T cells at different time points. The left and right columns of Figure 9C show the expression level of CD19 CAR in 17-B T cells on the 6th day after electroporation (before the activation of target cells Nalm6) and after cryopreservation and recovery post target cell activation, respectively. The results show that after the activation of target cells Nalm6, 17-B T cells can undergo extensive natural expansion after electroporation and then further expansion. The CAR positive rate also significantly increased after 6 days of further expansion caused by target cell activation.

### (2) Detection of cell killing ability

17-B T cells were co-cultured with Calcein-AM (purchased from Invitrogen, catalog number Cat# C3099) labeled target cells Nalm6 (clone G5, CRL3273, purchased from ATCC) (human B-cell acute lymphoblastic leukemia cells). After incubation, the mortality rate of target cells killed by T cells was calculated. Mortality rate = N_{D} / N_{L} * 100%. Here, N_{L} is the number of target cells collected in 20 seconds during FACS detection, and N_{D} is the number of dead target cells among them.

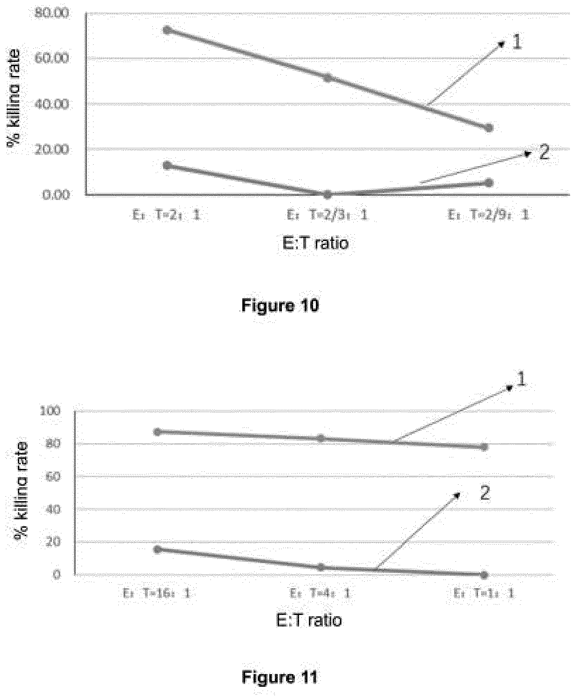

The mortality rate results are shown in Figure 10. In Figure 10, the horizontal axis E:T represents the ratio of effector cells (17-B T cells) to target cells (tumor cells). Bars 1-2 in Figure 10 show the killing conditions of 17-B T cells (1) and control cells (2), where the control cells are T cells without any plasmid transfection.

The results of Figure 10 show that under the condition of low host genomic DNA content, immune effector cells with good tumor killing ability can be obtained using the plasmid construct.

### (3) Detection of cell supernatant killing ability

17-B T cells were cultured in Xvivo-15 medium containing 5% AB serum, 100 U/ml IL-2, 10 ng/ml IN-7, and 5 ng/ml IL-15. The cell concentration was maintained at 5×10⁵-2×10⁶/ml. After 1 day of cell culture, the supernatant was collected by centrifugation. In the co-culture of control group cells and target cells Nalm6 at a fixed effector-to-target ratio, 50 µl of 17-B T cell supernatant was added or not added. One day later, the killing rate of target cells was measured.

The results are shown in Figure 11. Bars 1-2 represent the killing of target cells by the supernatant of 17-B T cells and the control target cells, respectively. The results of Figure 11 show that the supernatant of 17-B T cells has good tumor killing ability.

### (4) Detection of cell killing ability

Nalm-6-luc cells (purchased from PharmaLegacy, Shanghai) were injected intravenously into NCG mice (purchased from Gempharmatech Co., Ltd) at a concentration of 1×10⁶/ml to establish a mouse model of B-cell acute lymphoblastic leukemia.

Four days later, 100 µl of 17-B T cells and PBMC cells from the same cell donor were injected intravenously into the tail vein of 3 mice in the mouse model at a concentration of 1×10⁸/mL, and these mice were referred to as the experimental group. The other 2 mice in the mouse model were injected intravenously with 100 µl of PBMC cells at a concentration of 1x108/mL without receiving any other treatment and served as the control group.

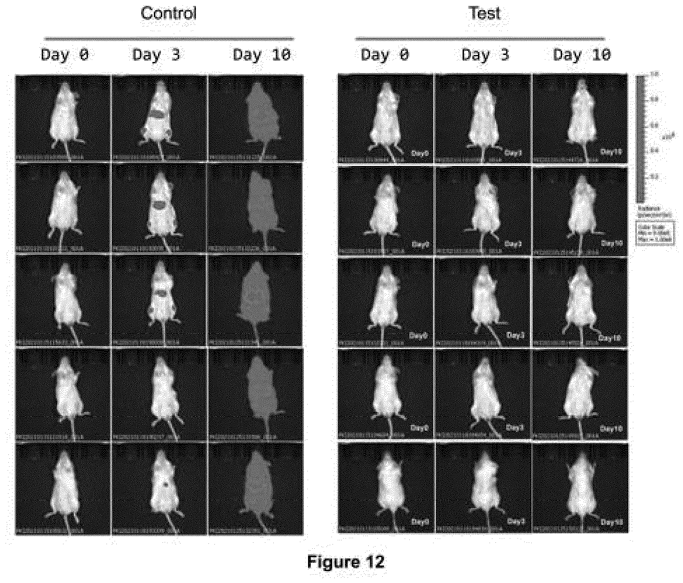

On days 0, 3, and 10 after intravenous injection of T cells, the in vivo tumor status of the experimental group and control group mice was detected by fluorescence (Lumina XRMS, PerkinElmer). The results are shown in Figure 12.

The results of Figure 12 show that the tumor in the control group grew rapidly, while the tumor in the experimental group almost disappeared (no fluorescence value detected). It can be seen that under the condition of low host genomic DNA content, T cells transfected with the plasmid have a significant killing effect on tumor cells in mice.

### Example 6: Reducing the content of host genomic DNA in plasmids can improve transfection efficiency

Apply DNAse (Plasmid-Safe ATP-dependent DNase, catalog number E3110K, purchased from Lucigen) to plasmid 3You-A. Use DNase to treat the DNA plasmid according to the procedure provided in the kit: 50µg of plasmid 3You-A is added to a system with a total volume of 500µl, comprising 50µl DNase, 20µl ATP, and 50µl reaction buffer. Digest for one hour. Then, recover the digested plasmid using the sodium acetate precipitation method for DNA. After washing with 70% ethanol, centrifuge and suspend the plasmid in water. After digestion, the content of host genome DNA in the plasmid is reduced by about 150 times (i.e., its content accounts for about 0.018 (w/w)% of the total nucleic acid in the transfection composition).

Then, transfect human T cells activated with Dynabead for 2-3 days with plasmid 3You-A without DNAse treatment (Group A), plasmid 3You-A treated with DNAse (Group B), and plasmid 3You-B as a positive control (Group C). The control group is Group D, which consists of T cells without any plasmid transfection.

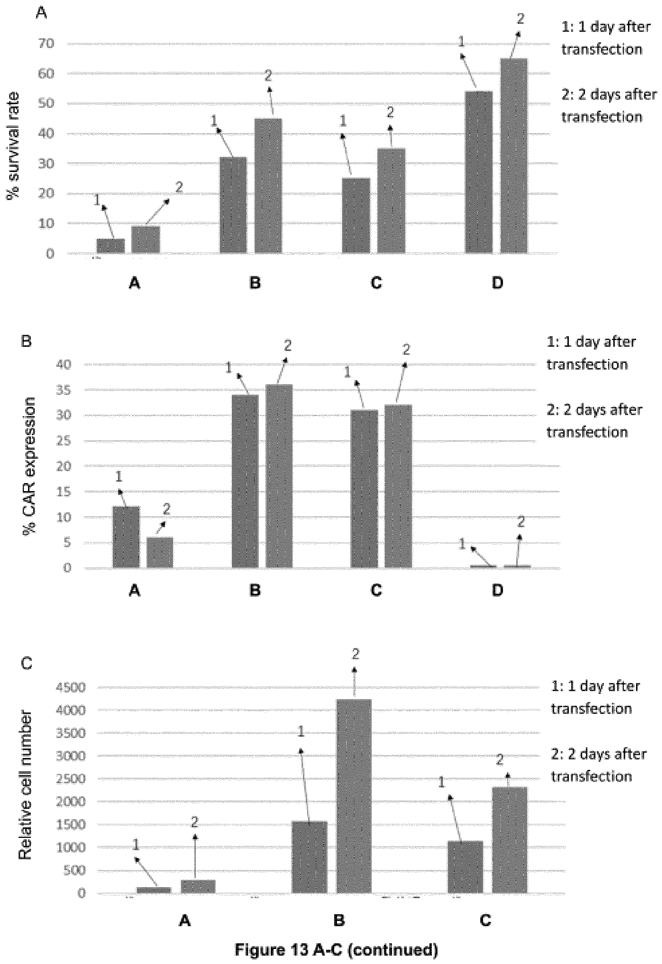

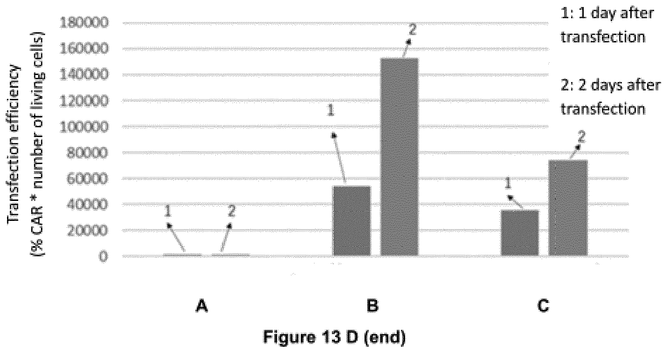

According to the method of Example 3, calculate the viability (Figure 13A), CAR expression level (Figure 13B), cell number (Figure 13C), and electroporation efficiency (Figure 13D) of the cells transfected with the above-mentioned plasmids on the first and second days after transfection.

The results of Figure 13 show that reducing the content of host genome DNA in the plasmid using DNAse can further improve the transfection efficiency of the plasmid in transfected cells.

### Example 7: Immune cells transfected with plasmids containing less host genome DNA can specifically kill tumor cells

A chemically synthesized sgRNA targeting human TRAC gene (SEQ ID NO: 8, purchased from GenScript, Nanjing) is designed, with 3 thio and O-methyl modifications at both the 5' and 3' ends. The purchased Cas9 protein is sourced from Sino Biological (catalog number: 40572-A08B).

Electroporate the human T cells activated with Dynabead for 2-3 days with plasmid 33-B, mixed with sgRNA and Cas9 (prepared as RNP system, Cas9: sgRNA at about 3:1 weight ratio). The nucleic acid molecule encoding GPC3 CAR-2A-IL15 is knocked into the human TRAC gene and can be expressed through its endogenous promoter. The obtained cells are 33-B T cells.

Inject 1×10⁷ HepG-2 cells (purchased from PharmaLegacy, Shanghai) subcutaneously into NCG mice (purchased from Gempharmatech Co., Ltd) to establish a subcutaneous tumor mouse model.

Approximately 14 days later, when the tumor grows to about 100 mm³ in volume, inject 100µl of 33-B T cells at a concentration of 1×10⁸/mL into the tail veins of 3 mice in the mouse model, and refer to these mice as the experimental group. The other 3 mice in the mouse model do not receive any treatment and serve as the control group.

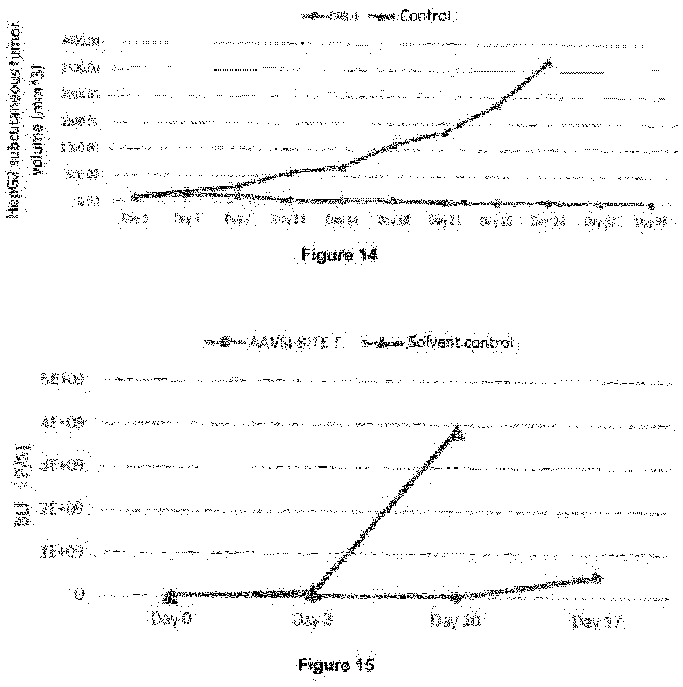

During the 35 days after intravenous injection of T cells, measure tumor volume weekly to detect the tumor growth in the experimental group mice (CAR-1) and control group mice. The results are shown in Figure 14.

The control group mice die at about 28 days. In the experimental group, the tumor volume tends to zero at 35 days. The results of Figure 14 show that the tumor in the control group grows rapidly, while the tumor in the experimental group almost disappears. It shows that when the content of host genome DNA in the plasmid is lower, the T cells transfected with the plasmid have a significant killing effect on tumor cells in the mice.

### Example 8: T cells transfected with plasmids containing less host genome DNA can specifically kill tumor cells

A chemically synthesized sgRNA targeting human AAVS-I gene (SEQ ID NO: 18, purchased from GenScript, Nanjing) is designed. The purchased Cas9 protein is sourced from Sino Biological (catalog number: 40572-A08B).

Plasmid 9-B is mixed with sgRNA and Cas9 (prepared as an RNP system, Cas9:sgRNA at a weight ratio of about 3:1) and electroporated into human T cells activated by Dynabead for 2-3 days, and the nucleic acid molecule encoding the BiTE is knocked into the human AAVS-I gene locus and expressed under the control of the exogenous promoter (hPGK promoter). The obtained cells are 9-B T cells.

Nalm-6-luc cells (purchased from PharmaLegacy, Shanghai) are intravenously injected into NCG mice (purchased from Gempharmatech Co., Ltd) at a concentration of 1×10⁶/ml to establish a mouse model of B-cell acute lymphoblastic leukemia.

Four days later, 100µl of 9-B T cells at a concentration of 1×10⁸/mL are injected into the tail vein of 3 mice in the mouse model, and these mice are referred to as the experimental group. The other 3 mice in the mouse model do not receive any treatment and serve as the control group.

On day 0, 3, 7, 10, 14, and 17 after intravenous injection of the T cells, the in vivo tumor conditions of the experimental group mice and control group mice are detected by fluorescence detection (Lumina XRMS, PerkinElmer). The results are shown in Figure 15.

The results of Figure 15 show that the tumors in the control group grow rapidly, while the experimental group has significant tumor suppression effects. It can be seen that under the condition of low host genome DNA content, the immune effector cells transfected with the plasmid and knocked into the exogenous gene at the AAVS-I locus have a significant killing effect on tumor cells in mice.

### Example 9 T cells transfected with plasmids containing less host genomic DNA can specifically kill tumor cells

A chemically synthesized sgRNA targeting the human TRAC gene (SEQ ID NO: 8, purchased from GenScript Biotech, Nanjing) is designed, with 3 thio and O-methyl modifications at both the 5' and 3' ends. The purchased Cas9 protein is sourced from Sino Biological Inc. (product number: 40572-A08B).

Plasmid 32-B is mixed with sgRNA and Cas9 (prepared as an RNP system, Cas9:sgRNA at a weight ratio of about 3:1) and electroporated into human T cells activated by Dynabead for 2-3 days, and the nucleic acid molecule encoding GPC3 CAR-2A-IL-7-2A-CCL19 is knocked into the human TRAC gene and can be expressed by the endogenous promoter of the TRAC gene. The obtained cells are 32-B T cells.

HepG-2 cells (purchased from PharmaLegacy, Shanghai) are subcutaneously injected into NCG mice (purchased from Gempharmatech Co., Ltd) at a concentration of 1×10⁷ to establish a subcutaneous tumor mouse model.

About 14 days later, when the tumor grows to a volume of about 100 mm3, 100µl of the 32-B T cells at a concentration of 1×10⁸/mL are injected into the tail vein of 3 mice in the mouse model, and these mice are referred to as the experimental group. The other 3 mice in the mouse model do not receive any treatment and serve as the control group.

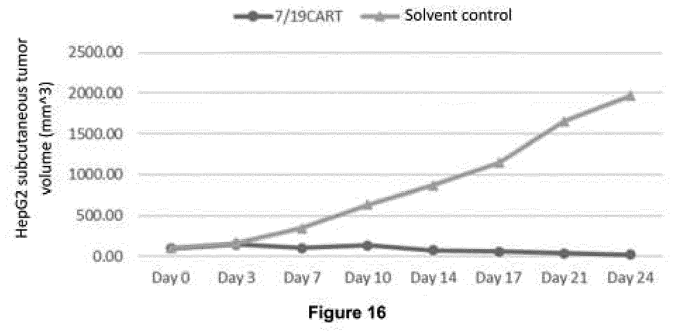

During the 24 days after the intravenous injection of the T cells, the tumor volume is measured weekly to detect the tumor growth of the experimental group mice (7/19CART) and the control group mice. The results are shown in Figure 16.

The results of Figure 16 show that the tumors in the control group grow rapidly, while the tumors in the experimental group almost disappear. It shows that under the condition of low host genome DNA content, T cells transfected with the plasmid and knocked into the human TRAC locus exogenous gene have a significant killing effect on tumor cells in mice.

### Example 10 T cells transfected with plasmids containing less host genomic DNA can increase the transfection efficiency of non-activated T cells or activated T cells

Treat plasmid 11-A with DNA enzyme (Plasmid-Safe ATP-dependent DNase, Cat No. E3110K, purchased from Lucigen). Process the DNA plasmid with DNase according to the protocol provided in the kit: add 50µg of plasmid 11-A to a final system of 500µl volume containing 50µl DNase, 20µl ATP, and 50µl reaction solution. Digest for one hour. Then, recover the digested plasmid using the sodium acetate precipitation method for DNA. Wash with 70% ethanol, centrifuge, and suspend the plasmid in water. After digestion, the content of host genomic DNA in the plasmid is reduced by about 10 times (i.e., after digestion, its content accounts for about 0.3 (w/w)% of the total nucleic acid content in the transfection composition).

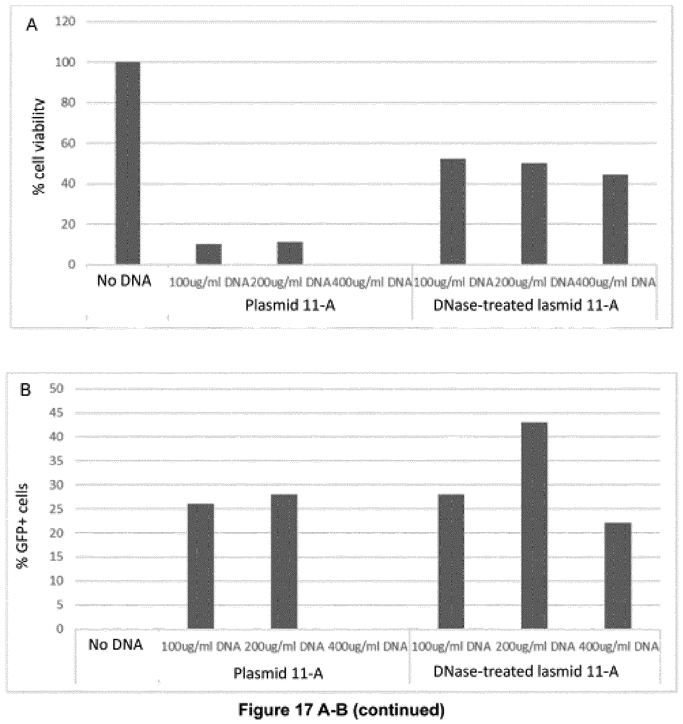

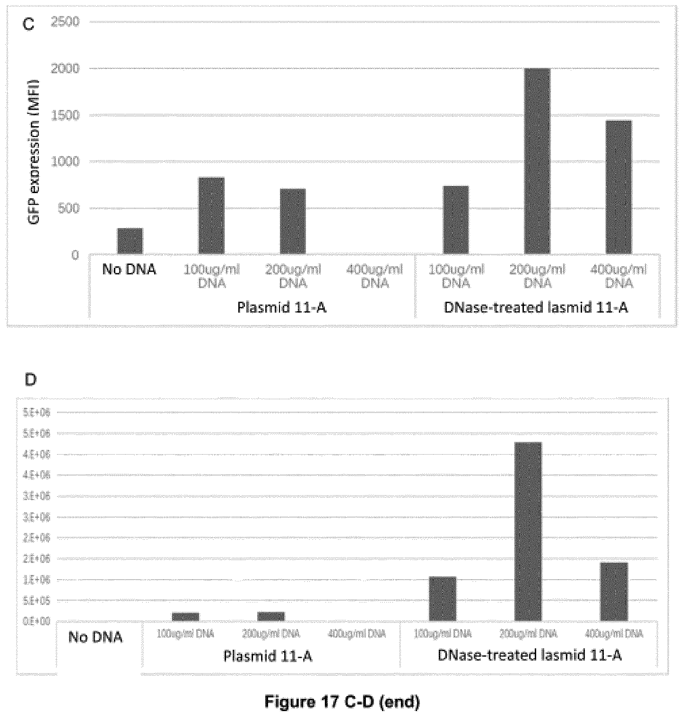

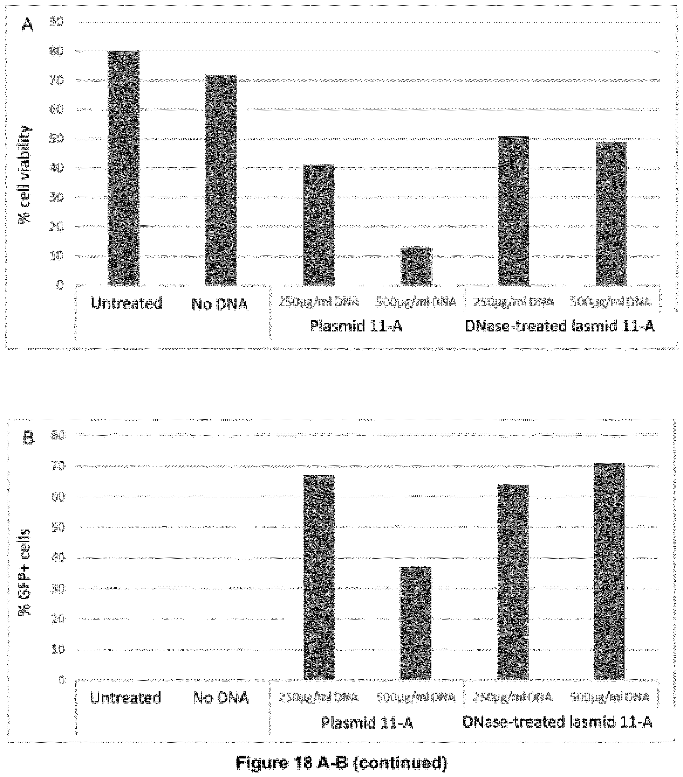

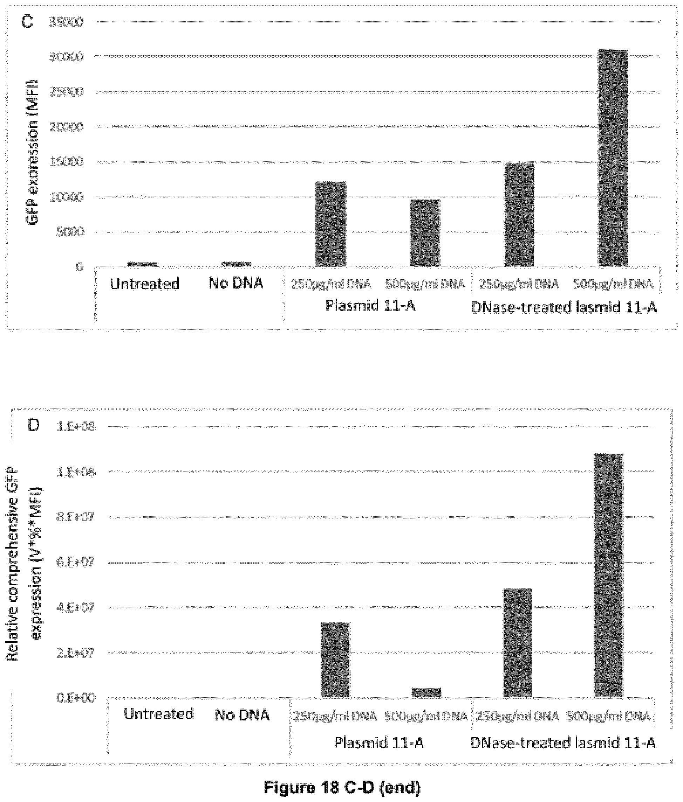

Next, use untreated plasmid 11-A (group A) and DNase-treated plasmid 11-A (group B) to transfect unactivated human T cells (Figures 17A-17D), or human T cells activated by Dynabeads for 2-3 days (Figures 18A-18D).

The results of Figures 17A-17D and Figures 18A-18D show that whether using non-activated T cells for transfection (Figures 17A-17D) or using activated T cells for transfection (Figures 18A-18D), when the content of host genomic DNA in the transfection composition is reduced, the transfection efficiency, cell viability (17A, 18A), GFP expression (17B, 18B, 17C, 18C), and GFP relative comprehensive expression (17D, 18D) can be significantly improved.

### Example 11: T cells transfected with plasmid with less host genomic DNA can specifically kill tumor cells

Knocking out PD-1 and CD95 can enhance the function of CART. For mainstream viral methods, it is challenging to design GMP-compliant methods that knock out endogenous genes in one step while infecting cells. For non-viral transfection methods, the transfection efficiency was low when using non-viral transfection methods (e.g., plasmid transfection methods) for cell transfection. If one hopes to knock out one or more endogenous genes while transfecting exogenous genes with plasmid, the transfection efficiency will be even lower.

Using the method described in the present application, the transfection efficiency, gene editing efficiency, and exogenous gene expression can be greatly improved, allowing for the simultaneous knock-in of exogenous genes and knockout of multiple endogenous genes in a single transfection.

Design chemically synthesized sgRNA targeting human TRAC gene (SEQ ID NO: 8, purchased from Genscript Biotech Corp, Nanjing). Design chemically synthesized sgRNA targeting human CD95 gene (SEQ ID NO: 28, purchased from Genscript Biotech Corp, Nanjing). Design chemically synthesized sgRNA targeting human PD-1 gene (SEQ ID NO: 25, purchased from Genscript Biotech Corp, Nanjing). The purchased Cas9 protein is sourced from Sino Biological Inc. (Cat No: 40572-A08B).

After mixing plasmid 12-B with the appropriate sgRNA and Cas9 (prepared as an RNP system, Cas9: sgRNA ratio of about 3:1 by weight), electroporate human T cells activated by Dynabeads for 2-3 days, the nucleic acid molecule encoding the GPC3 CAR is knocked into the human TRAC gene and can be expressed by the endogenous promoter of the TRAC gene, and the human PD-1 gene and/or human CD95 gene can be simultaneously knocked out (i.e., by adding the corresponding sgRNA). The obtained cells are 12-B T cells.

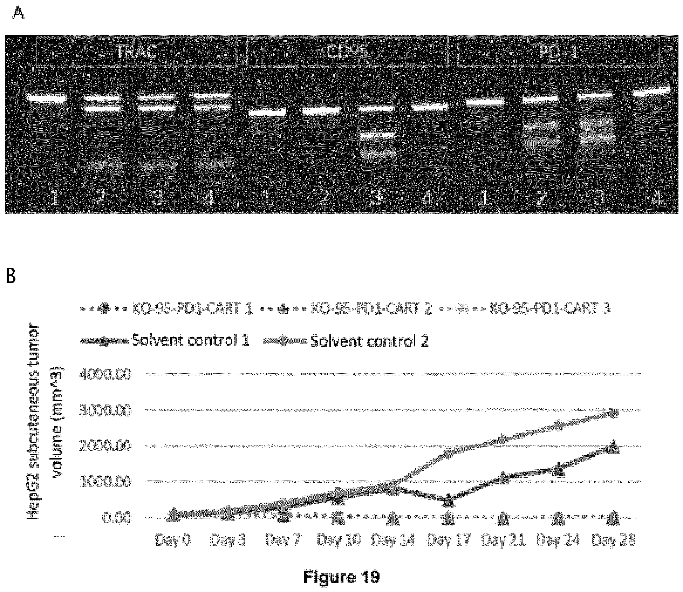

According to the manufacturer's instructions, the EnGen^{®} Mutation Detection Kit (NEB) is used to detect gene editing in cells and identify the cells requiring modification. Briefly, genomic DNA of the 12-B T cells is extracted and the target region for gene editing is amplified by PCR. The primer pair for amplifying the TRAC gene target region comprises the nucleic acid sequences shown in SEQ ID NO: 21 (forward primer) and SEQ ID NO: 22 (reverse primer). The primer pair for amplifying the PD-1 gene target region comprises the nucleic acid sequences shown in SEQ ID NO: 23 (forward primer) and SEQ ID NO: 24 (reverse primer). The primer pair for amplifying the CD95 gene target region comprises the nucleic acid sequences shown in SEQ ID NO: 26 (forward primer) and SEQ ID NO: 27 (reverse primer). T7 endonuclease is used to detect the presence of edited amplicons. The results are shown in Figure 19A.

In Figure 19A, the TRAC group shows gene editing at the TRAC locus, the CD95 group shows gene editing at the CD95 locus, and the PD-1 group shows gene editing at the PD-1 locus.

Lane 1 DNA is from control group cells (no transfection composition added); Lane 2 DNA is from cells transfected with plasmid 12-B (CAR expression=37%) and with PD-1 gene knocked out (i.e., the transfection composition also comprises sgRNA targeting PD-1); Lane 3 DNA is from cells transfected with plasmid 12-B (CAR expression=40%) and with knockout of PD-1 and CD95 genes (i.e., the transfection composition also comprises sgRNA targeting PD-1 and sgRNA targeting CD95); Lane 4 DNA is from cells transfected with plasmid 12-B (CAR expression=45%) but without knockout of PD-1 or CD95.

These results show that the method of the present application can achieve exogenous gene knock-in and endogenous gene knockout simultaneously by one-step transfection.

In addition, the *in vivo* tumor killing ability of 12-B T cells transfected with plasmid 12-B and simultaneously knocking out PD-1 and CD95 genes was also validated.

Subcutaneously injecting 1×10⁷ HepG-2 cells (purchased from PharmaLegacy, Shanghai) into NCG mice (purchased from Gempharmatech Co., Ltd) to establish a subcutaneous tumor mouse model.

About 14 days later, when the tumor grows to about 100 mm³ in volume, 100µl of the 12-B T cells at a concentration of 5×10⁵/mL is injected into the tail vein of 3 mice in the mouse model, and these mice are referred to as the experimental group. The other 2 mice in the mouse model do not receive any treatment and serve as controls.

During 28 days after intravenous injection of the T cells, tumor volume is measured twice a week to monitor tumor growth in the experimental group mice (KO-95-PD1-CART) and control group mice. The results are shown in Figure 19B.

The results of Figure 19B show that the tumor in the control group grows rapidly, while the tumor in the experimental group almost disappears. It shows that under conditions of low host genomic DNA content, immune effector cells with exogenous genes knocked in at the human TRAC locus and simultaneously knocking out PD-1 and CD95 through the plasmid transfection have a significant killing effect on tumor cells in mice.

The foregoing detailed description is provided for explanation and example purposes and is not intended to limit the scope of the appended claims. Various modifications of the embodiments listed in the present application are apparent to those skilled in the art and are reserved within the scope of the appended claims and their equivalents.

## Claims

1. A method for modifying a cell, which comprises: transfecting a cell to be modified with a transfection composition to cause the cell to comprise and/or express an exogenous gene, wherein the transfection composition comprises an exogenous nucleic acid molecule comprising the exogenous gene, wherein at least a portion of the exogenous nucleic acid molecules are derived from a host cell; and in the portion of the exogenous nucleic acid molecules derived from the host cell, the host cell genomic DNA has a content of about 10% (w/w) or less.

2. The method according to claim 1, wherein the cell to be modified is an eukaryotic cell.

3. The method according to any one of claims 1 to 2, wherein the cell to be modified is a mammalian cell.

4. The method according to any one of claims 1 to 3, wherein the cell to be modified is a human cell.

5. The method according to any one of claims 1 to 4, wherein the cell to be modified comprises stem cell, immune cell, fibroblast, fibrocyte, and/or muscle cell.

6. The method according to any one of claims 1 to 5, wherein the cell to be modified comprises multipotent stem cell, hematopoietic stem cell, and/or mesenchymal stem cell.

7. The method according to any one of claims 1 to 6, wherein the cell to be modified comprises immune effector cell.

8. The method according to any one of claims 1 to 7, wherein the cell to be modified comprises T lymphocyte, B lymphocyte, NK cell, macrophage, dendritic cell, monocyte, granulocyte, and/or mast cell.

9. The method according to any one of claims 1 to 8, wherein the cell to be modified comprises peripheral blood lymphocyte.

10. The method according to any one of claims 1 to 9, wherein the cell to be modified is a primary cell.

11. The method according to any one of claims 1 to 10, wherein the cell to be modified is an autologous cell derived from a subject.

12. The method according to any one of claims 1 to 11, wherein the transfected cell to be modified is an activated cell.

13. The method according to claim 12, wherein the activation comprises contacting the cell to be modified with an activation composition.

14. The method according to claim 13, wherein the cell to be modified comprises immune cell, and the activation composition comprises anti-CD3 and/or anti-CD28 antibody.

15. The method according to any one of claims 13 to 14, wherein the activation comprises contacting the cell to be modified with the activation composition for no more than about 4 days.

16. The method according to any one of claims 13 to 15, wherein the method comprises transfecting when the cell to be modified is contacted with the activation composition for a period of about 2 days or less.

17. The method according to any one of claims 1 to 16, wherein the transfection comprises electroporating the cell to be modified.

18. The method according to any one of claims 1 to 17, wherein the exogenous nucleic acid molecule derived from the host cell is a plasmid.

19. The method according to any one of claims 1 to 18, wherein the exogenous nucleic acid molecule comprises circular nucleic acid molecule, supercoiled nucleic acid molecule, and/or linear nucleic acid molecule.

20. The method according to any one of claims 1 to 19, wherein the exogenous nucleic acid molecule comprises DNA molecule and/or RNA molecule.

21. The method according to any one of claims 1 to 20, wherein the exogenous nucleic acid molecule comprises single-stranded nucleic acid molecule and/or double-stranded nucleic acid molecule.

22. The method according to any one of claims 1 to 21, wherein the concentration of the exogenous nucleic acid molecule in the transfection composition is about 5µg/mL to about 3000µg/mL.

23. The method according to any one of claims 1 to 22, wherein the concentration of the exogenous nucleic acid molecule in the transfection composition is about 200µg/mL to about 800µg/mL.

24. The method according to any one of claims 1 to 23, wherein the exogenous nucleic acid molecule is extracted from the host cell.

25. The method according to any one of claims 1 to 24, wherein the size of the exogenous nucleic acid molecule is at least about 3kb.

26. The method according to any one of claims 1 to 25, wherein the host is a microbial host.

27. The method according to any one of claims 1 to 26, wherein the host is selected from the group consisting of: bacteria, fungi, actinomycetes, mycoplasma, chlamydia, and archaea.

28. The method according to any one of claims 1 to 27, wherein the host comprises gram-negative bacterium.

29. A method according to any one of claims 1 to 28, wherein the host comprises *Escherichia coli.*

30. A method according to any one of claims 1 to 29, wherein the size of the genomic DNA of the host cell is at least about 10kb.

31. A method according to any one of claims 1 to 30, wherein the content of the genomic DNA of the host cell is less than about 1% (w/w).

32. A method according to any one of claims 1 to 31, wherein the content of the genomic DNA of the host cell is less than about 9‰ (w/w).

33. A method according to any one of claims 1 to 32, wherein the content of the genomic DNA of the host cell is determined by qPCR method.

34. A method according to any one of claims 1 to 33, wherein the genomic DNA of the host cell is not comprised in the portion of the exogenous nucleic acid molecules.

35. A method according to any one of claims 1 to 34, which comprising: treating the portion of the exogenous nucleic acid molecules derived from the host cell to reduce the content of the genomic DNA of the host cell therein; and/or measuring the content of the genomic DNA of the host cell in the portion of the exogenous nucleic acid molecules derived from the host cell, and determining whether the portion of the exogenous nucleic acid molecules part is to be treated to reduce the content of the genomic DNA of the host cell therein based on the content.

36. A method according to claim 35, wherein the content of the genomic DNA of the host cell in the portion of the exogenous nucleic acid molecule derived from the host cell is measured, and when the content of the genomic DNA of the host cell is about 10% (w/w) or more, the portion of the exogenous nucleic acid molecule is treated to reduce the content of the genomic DNA of the host cell therein.

37. A method according to any one of claims 35 to 36, wherein the treatment comprises contacting the portion of the exogenous nucleic acid molecule with one or more reagents selected from the group consisting of: deoxyribonuclease (DNase), SDS, TX-100, CTAB, and cesium chloride-ethidium bromide.

38. A method according to claim 37, wherein the DNase is capable of non-specifically cleaving linear DNA.

39. A method according to any one of claims 37 to 38, wherein the DNase is an exonuclease.

40. A method according to any one of claims 35 to 39, wherein the treatment comprises contacting the transfection composition with the reagent in the presence of Mg²⁺ and Ca²⁺.

41. A method according to any one of claims 1 to 40, wherein the exogenous gene encodes one or more exogenous proteins.

42. A method according to claim 41, wherein the exogenous protein comprises one or more proteins selected from the group consisting of: an antibody or antigen-binding fragment, a chimeric antigen receptor (CAR), a cytokine, and a chemokine.

43. A method according to claim 42, wherein the CAR comprises a target-binding domain targeting a tumor-associated antigen.

44. A method according to claim 43, wherein the tumor-associated antigen is selected from: GPC3, CD19, BCMA, Claudin18.2, and Mesothelin.

45. A method according to any one of claims 42 to 44, wherein the antibody or antigen-binding fragment comprises a multispecific antibody or an antigen-binding fragment thereof.

46. A method according to claim 45, wherein the multispecific antibods or the antigen-binding fragment thereof comprises a bispecific T-cell engager (BiTE).

47. A method according to claim 46, wherein the BiTE comprises a CD3-binding domain.

48. A method according to any one of claims 46 to 47, wherein the BiTE further comprises a tumor-associated antigen-binding domain.

49. A method according to any one of claims 42 to 48, wherein the cytokine comprises interleukin.

50. A method according to claim 49, wherein the interleukin comprises IL15, IL7 or functionally active fragments thereof.

51. A method according to any one of claims 42 to 50, wherein the chemokine comprises CCL19 or a functionally active fragment thereof.

52. A method according to any one of claims 1 to 51, wherein the exogenous protein comprises a multiprotein.

53. A method according to claim 52, wherein a cleavable part is present between two or more proteins within the multiprotein.

54. A method according to claim 53, wherein the cleavable part comprises a 2A peptide.

55. A method according to claim 54, wherein the 2A peptide comprises P2A, T2A, F2A, or E2A.

56. A method according to any one of claims 1 to 55, wherein the exogenous nucleic acid molecule further comprises one or more homologous regions.

57. The method of claim 56, wherein each of the homologous regions comprises at least 10 nucleotides.

58. A method according to any one of claims 56 to 57, wherein in the exogenous nucleic acid molecule, the homologous region is located at the 3' end and/or 5' end of the exogenous gene.

59. A method according to any one of claims 1 to 58, wherein the exogenous gene is integrated into the genome of the cell.

60. A method according to any one of claims 56 to 59, wherein the homologous regions are homologous to the target region of the cell genomic DNA.

61. The method of claim 60, wherein the target region is located in the TCR-α constant (TRAC) gene, or at the AAVS-I locus.

62. A method according to any one of claims 1 to 61, wherein the transfection composition does not comprise a viral vector.

63. A method according to any one of claims 1 to 62, wherein the transfection composition further comprises a gene editing system capable of integrating the exogenous gene into a targeted location in the cell genome.

64. The method of claim 63, wherein the gene editing system comprises a site-specific enzyme or a nucleic acid molecule encoding the enzyme.

65. The method of claim 64, wherein the site-specific enzyme is selected from the group consisting of: transcription activator-like effector nuclease (TALEN), zinc finger nuclease (ZFN), transposase, integrase, and Cas protein.

66. The method of claim 65, wherein the Cas protein is Cas9 protein.

67. The method of claim 65, wherein the transposase comprises PiggyBac (PB) transposase, and/or Sleeping Beauty (SB) transposase.

68. A method according to any one of claims 63 to 67, wherein the gene editing system further comprises one or more guide RNAs.

69. The method of claim 68, wherein the guide RNA is complementary to a nucleic acid sequence of the target region of the cell genome.

70. A method according to any one of claims 68 to 69, wherein the gene editing system comprises a ribonucleoprotein complex (RNP), and the RNP comprises the Cas protein and the guide RNA.

71. A method for enhancing efficiency of transfecting exogenous nucleic acid molecules derived from host cells into a cell, which comprises: reducing the content of the host cell genomic DNA in the portion of the exogenous nucleic acid molecules derived from the host cells.

72. The method of claim 71, wherein the cell to be transfected is an eukaryotic cell.

73. A method according to any one of claims 71 to 72, wherein the cell to be transfected is a mammalian cell.

74. A method according to any one of claims 71 to 73, wherein the cell to be transfected is a human cell.

75. A method according to any one of claims 71 to 74, wherein the cell to be transfected comprises stem cell, immune cell, fibroblast, fibrocyte, and/or muscle cell.

76. A method according to any one of claims 71 to 75, wherein the cell to be transfected comprises pluripotent stem cell, hematopoietic stem cell, and/or mesenchymal stem cell.

77. A method according to any one of claims 71 to 76, wherein the cell to be transfected comprises immune effector cell.

78. A method according to any one of claims 71 to 77, wherein the cell to be transfected comprises T lymphocyte, B lymphocyte, NK cell, macrophage, dendritic cell, monocyte, granulocyte, and/or mast cell.

79. A method according to any one of claims 71 to 78, wherein the cell to be transfected comprises peripheral blood lymphocytes.

80. A method according to any one of claims 71 to 79, wherein the cell to be transfected is primary cell.

81. A method according to any one of claims 71 to 80, wherein the cell to be transfected is autologous cell derived from a subject.

82. A method according to any one of claims 71 to 81, wherein the transfected cell is an activated cell.

83. The method of claim 82, wherein the activation comprises contacting the cell to be transfected with an activation composition.

84. The method of claim 83, wherein the cell to be transfected comprises immune cell, and the activation composition comprises anti-CD3 and/or anti-CD28 antibodies.

85. A method according to any one of claims 83 to 84, wherein the activation comprises contacting the cell to be transfected with the activating composition for no more than about 4 days.

86. A method according to any one of claims 83 to 85, wherein the method comprises transfecting when the cell to be transfected is in contact with the activation composition for a period of about 2 days or less.

87. A method according to any one of claims 71 to 86, wherein the transfection comprises electroporating the cell.

88. A method according to any one of claims 71 to 87, wherein the exogenous nucleic acid molecule is a plasmid.

89. A method according to any one of claims 71 to 88, wherein the exogenous nucleic acid molecule comprises circular nucleic acid molecule, supercoiled nucleic acid molecule, and/or linear nucleic acid molecule.

90. A method according to any one of claims 71 to 89, wherein the exogenous nucleic acid molecule comprises DNA molecule and/or RNA molecule.

91. A method according to any one of claims 71 to 90, wherein the exogenous nucleic acid molecule comprises single-stranded nucleic acid molecule and/or double-stranded nucleic acid molecule.

92. A method according to any one of claims 71 to 91, wherein the concentration of the exogenous nucleic acid molecule in the transfection composition is about 5µg/mL to about 3000µg/mL.

93. A method according to any one of claims 71 to 92, wherein the concentration of the exogenous nucleic acid molecule in the transfection composition is about 200µg/mL to about 800µg/mL.

94. A method according to any one of claims 71 to 93, wherein the exogenous nucleic acid molecule is obtained by extraction from the host cell.

95. A method according to any one of claims 71 to 94, wherein the size of the exogenous nucleic acid molecule is at least about 3kb.

96. A method according to any one of claims 71 to 95, wherein the host is a microbial host.

97. A method according to any one of claims 71 to 96, wherein the host is selected from the following group consisting of: bacteria, fungi, actinomycetes, mycoplasma, chlamydia, rickettsia, and spirochetes.

98. A method according to any one of claims 71 to 97, wherein the host comprises Gram-negative bacteria.

99. A method according to any one of claims 71 to 98, wherein the host comprises *Escherichia coli.*

100. A method according to any one of claims 71 to 99, wherein the size of the genomic DNA of the host cell is at least about 10kb.

101. A method according to any one of claims 71 to 100, wherein after the reduction, the content of the genomic DNA of the host cell is less than about 10% (w/w).

102. A method according to any one of claims 71 to 101, wherein after the reduction, the content of the genomic DNA of the host cell is less than about 1% (w/w).

103. A method according to any one of claims 71 to 102, wherein the content of the genomic DNA of the host cell is determined by qPCR.

104. A method according to any one of claims 71 to 103, wherein reducing the content of the genomic DNA of the host cell comprises contacting the exogenous nucleic acid molecule obtained from the host cell with one or more reagents selected from the group consisting of: deoxyribonuclease (DNase), SDS, TX-100, CTAB, and cesium chloride-ethidium bromide.

105. A method according to claim 104, wherein the DNase is capable of nonspecifically cleaving linear DNA.

106. A method according to any one of claims 104 to 105, wherein the DNase is an exonuclease.

107. A method according to any one of claims 104 to 106, wherein reducing the content of the genomic DNA of the host cell comprises contacting the exogenous nucleic acid molecule obtained from the host cell with the reagent in the presence of Mg²⁺ and Ca²⁺.

108. A method according to any one of claims 71 to 107, wherein the exogenous nucleic acid molecule comprises an exogenous gene as defined in any one of claims 1 to 70.

109. A method according to any one of claims 71 to 108, wherein the exogenous nucleic acid molecule further comprises one or more homologous regions as defined in any one of claims 56 to 70.

110. A method according to any one of claims 71 to 109, wherein the exogenous nucleic acid molecule does not comprise a viral vector.

111. A method according to any one of claims 108 to 110, wherein the transfection composition further comprises a gene editing system capable of integrating the exogenous gene into a targeted location of the cell genome.

112. The method according to claim 111, wherein the gene editing system is as defined in any one of claims 64 to 70.

113. A method for improving efficiency of transfecting exogenous nucleic acid molecules obtained from host cells into a cell, which comprises: treating the exogenous nucleic acid molecules with DNase.

114. The method according to claim 113, wherein the DNase is capable of non-specifically cleaving linear DNA.

115. The method according to any one of claims 113 to 114, wherein the DNase is a nucleic acid exonuclease.

116. The method according to any one of claims 113 to 115, wherein the treatment comprises contacting the exogenous nucleic acid molecules with the DNase in the presence of Mg²⁺ and Ca²⁺.

117. The method according to any one of claims 113 to 116, wherein the transfection comprises electroporation of the cell.

118. The method according to any one of claims 113 to 117, wherein the exogenous nucleic acid molecule is as defined in any one of claims 1 to 70.

119. The method according to any one of claims 113 to 118, wherein the cell to be transfected is as defined in any one of claims 1 to 70.

120. A method for optimizing a transfection composition, wherein the trasfection composition comprises exogenous nucleic acid molecules obtained from host cells, and the method comprises: reducing the content of host cell genomic DNA in the portion of the exogenous nucleic acid molecules obtained from the host cells.

121. The method according to claim 120, wherein the transfection composition is as defined in any one of claims 1 to 70.

122. The method according to any one of claims 120 to 121, wherein the host is as defined in any one of claims 1 to 70.

123. The method according to any one of claims 120 to 122, wherein, after the optimization, the content of host cell genomic DNA is less than about 10% (w/w).

124. The method according to any one of claims 120 to 123, wherein, after the optimization, the content of host cell genomic DNA is less than about 1% (w/w).

125. The method according to any one of claims 120 to 124, wherein the content of host cell genomic DNA is determined by qPCR.

126. The method according to any one of claims 120 to 125, wherein the optimization comprises contacting the part of exogenous nucleic acid molecules obtained from host cells with one or more reagents selected from the group consisting of: deoxyribonuclease (DNase), SDS, TX-100, CTAB, and cesium chloride-ethidium bromide.

127. The method according to claim 126, wherein the DNase is capable of non-specifically cleaving linear DNA.

128. The method according to any one of claims 126 to 127, wherein the DNase is a nucleic acid exonuclease.

129. The method according to any one of claims 120 to 128, wherein the optimization comprises contacting the portion of exogenous nucleic acid molecules obtained from host cells with the reagent in the presence of Mg²⁺ and Ca²⁺.

130. A method for optimizing a transfection composition, wherein the trasfection composition comprises a portion of exogenous nucleic acid molecules obtained from host cells, and the method comprises: treating the portion of exogenous nucleic acid molecules obtained from host cells with DNase.

131. The method according to claim 130, wherein the DNase is capable of non-specifically cleaving linear DNA.

132. The method according to any one of claims 130 to 131, wherein the DNase is a nucleic acid exonuclease.

133. The method according to any one of claims 130 to 132, wherein the treatment comprises contacting the portion of exogenous nucleic acid molecules obtained from host cells with the DNase in the presence of Mg²⁺ and Ca²⁺.

134. The method according to any one of claims 130 to 133, wherein the transfection composition is as defined in any one of claims 1 to 70.

135. A method for determining the quality of a transfection composition, wherein the trasfection composition comprises a portion of exogenous nucleic acid molecules obtained from host cells, and the method comprises: measuring a content of host cell genomic DNA in the part of exogenous nucleic acid molecules obtained from host cells and determining the quality of the transfection composition based on the content.

136. The method according to claim 135, wherein the transfection composition is as defined in any one of claims 1 to 70.

137. The method according to any one of claims 135 to 136, wherein the host is as defined in any one of claims 1 to 70.

138. The method according to any one of claims 135 to 137, wherein the quality of the transfection composition meets the standard when the content of the genomic DNA of the host cell is less than about 10% (w/w).

139. The method according to any one of claims 135 to 138, wherein the quality of the transfection composition meets the standard when the content of the genomic DNA of the host cell is less than about 1% (w/w).

140. The method according to any one of claims 135 to 139, wherein the content of the genomic DNA of the host cell is determined by qPCR method.

141. The use of reagent as defined in any one of claims 37 to 40 in optimizing the transfection composition.

142. The method according to any one of claims 1 to 141, which is an *in vitro* or ex *vivo* method.

143. A transfection reagent kit, which comprises:
1) the portion of exogenous nucleic acid molecule derived from the host cell as defined in any one of claims 1 to 70; and
2) a reagent capable of reducing or degrading the genomic DNA of the host cell.

144. The reagent kit according to claim 143, wherein the reagent described in 2) comprises one or more selected from the group consisting of: deoxyribonuclease (DNase), SDS, TX-100, CTAB, and cesium chloride-ethidium bromide.

145. The reagent kit according to claim 144, wherein the DNase is capable of non-specifically cleaving linear DNA.

146. The reagent kit according to any one of claims 144 to 145, wherein the DNase is a nucleic acid exonuclease.

147. The reagent kit according to any one of claims 143 to 146, which further comprises a reagent containing Mg²⁺ and Ca²⁺.

148. A reagent kit, which comprises:
1) a portion of exogenous nucleic acid molecules derived from the host cell as defined in any one of claims 1 to 70; and
2) an instruction, which records the process of the portion of exogenous nucleic acid molecule derived from the host cell as described in 1) according to any one of the methods of claims 113 to 134 and/or the determination of quality of the transfection composition comprising the exogenous nucleic acid molecule part derived from the host cell according to any one of the methods of claims 135 to 140.

149. A transfection composition comprising an exogenous nucleic acid molecule treated by any one of the methods of claims 113 to 134.

150. A cell transfected with the transfection composition according to claim 149.

151. The cell according to claim 150, wherein the transfection comprises electroporation of the cell.

152. A cell modified by any one of the methods according to claims 1 to 70.

153. A cell population, which comprises the cell and/or its progeny according to any one of claims 150 to 152.

154. A pharmaceutical composition, which comprises the transfection composition according to claim 149, the cell according to any one of claims 150 to 152, and/or the cell population according to claim 153.

155. The pharmaceutical composition according to claim 154, which further comprises a pharmaceutically acceptable adjuvant.

156. A use of the transfection composition according to claim 149, the cell according to any one of claims 150 to 152, the cell population according to claim 153, and/or the pharmaceutical composition according to any one of claims 154 to 155 for preparation of a medicament.

157. The use according to claim 156, wherein the medicament is used for the prevention, treatment, and/or alleviation of cancer.

158. The use according to any one of claims 156 to 157, wherein the medicament is for regulating the immune response.

159. A method for preventing, treating, and/or alleviating a disease or disorder in a subject, which comprises: administering to the subject an effective amount of the transfection composition according to claim 149, a cell according to any one of claims 150 to 152, a cell population according to claim 153, and/or a pharmaceutical composition according to any one of claims 154 to 155.

160. The method according to claim 159, wherein the disease or disorder is cancer.

161. The method according to any one of claims 159 to 160, wherein the disease or disorder is related to the immune response of the subject.
